# EUROPEAN PATENT APPLICATION

(11) **EP 1 602 642 A1**
(43) Date of publication of application: **07.12.2005**
(21) Application number: 04720259.3
(22) Date of filing: 12.03.2004
(51) Int. Cl.: C07C 251/54, C07D 317/14, A61K 31/357, A61P 3/04, A61P 3/06

(54) **IMINO ETHER DERIVATIVE COMPOUNDS AND DRUGS CONTAINING THE COMPOUNDS AS THE ACTIVE INGREDIENT**

(30) Priority: 13.03.2003 JP 2003068932
(71) Applicant: ONO PHARMACEUTICAL CO., LTD., Osaka-shi, Osaka 541-8526 (JP)
(72) Inventor: ISHIDA, A., Ono Pharmaceutical Co., Ltd., Mishima-gun, Osaka 618-8585 (JP); KUSUDA, S., Ono Pharmaceutical Co., Ltd., Mishima-gun, Osaka 618-8585 (JP); NAKAYAMA, Y., Ono Pharmaceutical Co., Ltd., Mishima-gun, Osaka 618-8585 (JP); TAJIMA, H., Ono Pharmaceutical Co., Ltd., Mishima-gun, Osaka 618-8585 (JP); KIDO, T., Ono Pharmaceutical Co., Ltd., Mishima-gun, Osaka 618-8585 (JP); KITAMINE, T., Ono Pharmaceutical Co., Ltd., Mishima-gun, Osaka 618-8585 (JP)
(74) Representative: Henkel, Feiler & Hänzel
(86) International application number: PCT/JP2004/003323
(87) International publication number: WO 2004/080947

(57) **Abstract**

The compound represented by formula (I) 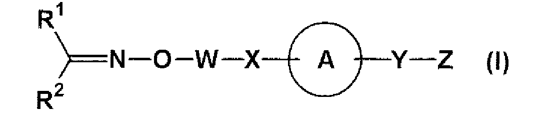 (wherein R¹ and R² are cyclic group which may have a substituent(s) and so on.; W is a spacer of which main chain has an atom number of 1-6.; X is -O- and so on.; ringA is cyclic group which may have a substituent(s).; Y is a spacer of which main chain has an atom number of 1-6 and so on.; Z is acidic group.) a salt thereof, a solvent thereof or an N-oxide thereof or a prodrug thereof Since the compounds in the present invention have a regulatory activity for peroxisome proliferator activated receptor, the compounds in the present invention are useful as a preventive and/or therapeutic agent for diseases associating metabolic disorders (*e.g.*, hypercholesterolemia, hyperlipoproteinemia, *etc*.), hyperlipidemia, atherosclerosis, hypertension, circulatory diseases, overeating, ischemic heart diseases, *etc*., an HDL cholesterol-elevating agent, an LDL cholesterol and/or a VLDL cholesterol-lowering agent and a drug for relief from risk factors of diabetes or metabolic syndrom.

Sequence Listing
<110> ONO Pharmaceutical Co.,Ltd.
<120> Iminoether derivative compounds and drugs containing these compounds as
the active ingredient
<130> ONF-4930PCT

<140> EP 04720259.3
<141> 2004-03-12

<150> PCT/JP2004/003323
<151> 2004-03-12

<150> JP 2003-68932
<151> 2003-03-13

<160> 3
<170> PatentIn Ver. 2.1

<210> 1
<211> 85
<212> DNA
<213> Artificial sequence
<220>
<223> Enhancer sequence including 4 times repeated Gal4 protein response
sequences
<400> 1
tcgacggagt actgtcctcc gcgacggagt actgtcctcc gcgacggagt actgtcctcc 60
gcgacggagt actgtcctcc gagct 85

<210> 2
<211> 9
<212> PRT
<213> Unknown
<220>
<223> Nuclear localozation signal derived from SV-40 T-antigen
<400> 2
Ala Pro Lys Lys Lys Arg Lys Val Gly
1               5

<210> 3
<211> 9
<212> PRT
<213> Influenza virus
<220>
<223> hemagglutinin epitope
<400> 3
Tyr Pro Tyr Asp Val Pro Asp Tyr Ala
1               5

## Description

### TECHNICAL FIELD

The present invention relates to imino ether derivative compounds.

For more detail, the present invention relates to
(1) a compound represented by formula (I) 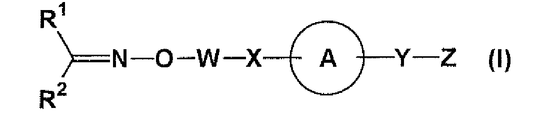 (wherein all the symbols have the same meanings as described below), a salt thereof, a solvent thereof or an N-oxide thereof, or a prodrug thereof,
(2) a process for the preparation thereof, and
(3) a pharmacological agent comprising of them as an active ingredient.

### BACKGROUND ART

Recently in the study of transcription factors associated with marker genes expression induction in adipocytes differentiation, peroxisome proliferator activated receptor (abbreviated as PPAR hereinafter), which is one of intranuclear receptors, has got attention. The cDNAs of PPAR were cloned from various kinds of animals, and plural isoform genes were found, particularly in mammals three types of isoforms (α, δ, γ) are known (see *J. Steroid Biochem. Molec. Biol*., 51, 157 (1994); *Gene Expression.,* 4, 281 (1995); *Biochem Biophys. Res. Commun*., 224, 431 (1996); *Mol. Endocrinology.,* 6, 1634 (1992)). Further, it is known that PPAR γ isoform predominantly expresses in adipose tissues, immune cells, adrenal gland, spleen, small intestine, PPAR α isoform mainly expresses in adipose tissue, liver, retina, and PPAR δ isoform universally expresses without specificity for tissue (see *Endocrinology*., 137, 354 (1996)).

By the way, thiazolidine derivatives such as pioglitazone hydrochloride, rosiglitazone maleate *etc*. are known as agents for the treatment of non-insulin dependent diabetes mellitus (NIDDM) and are hypoglycemic agents which are used for the improvement of hyperglycemia in the patients suffering from diabetes. They are also effective for the improvement of hyperinsulinemia, glucose tolerance and decrease of serum lipid and therefore they are thought to be considerably hopeful as agents for the improvement of insulin resistance.

In addition, one of the intracellular target proteins of these thiazolidine derivatives is exactly PPAR γ and it is resolved that they enhance the transcription activity of PPAR γ (see Endocrinology., 137, 4189 (1996); Cell., 83, 803 (1995); Cell., 83, 813 (1995); J. Biol. Chem., 270, 12953 (1995)). Therefore, a PPAR γ activator (agonist) which enhances its transcription activity is thought to be hopeful as a hypoglycemic agent and/or a hypolipidemic agent. Furthermore, since a PPAR γ agonist is known to promote the expression of PPAR γ protein itself (Genes & Development., 10, 974 (1996)), an agent which increases the expression of PPAR γ protein itself as well as PPAR γ activating agent is also thought to be clinically useful.

Intracellular receptor, PPAR γ is related to adipocytes differentiation (see J. Biol. Chem., 272, 5637 (1997) and Cell., 83, 803 (1995)). It is known that thiazolidine derivatives which activate this receptor promote adipocytes differentiation. Recently it was reported that thiazolidine derivatives increase body fat and cause man to gain weight and to become obese (see Lancet., 349, 952 (1997)). Therefore, it is also thought that antagonists which inhibit PPAR γ activity and agents that decrease the expression of PPAR γ protein itself are also clinically applicable. On the other hand, a compound that phosphorylates PPAR γ protein and decreases its activity is reported (Science., 274, 2100 (1996)). This implies that an agent which does not bind on PPAR γ protein as a ligand, but inhibits its activity is also clinically applicable.

From these, PPAR γ activators (agonists) and PPAR γ regulators for its expression that can increase the expression of the protein itself are expected to be useful as hypoglycemic agents, hypolipidemic agents, and agents for prevention and/or treatment of diseases associated with metabolic disorders such as diabetes, adiposity, metabolic sydrome, hypercholesterolemia and hyperlipoproteinemia *etc*., hyperlipidemia, atherosclerosis, hypertension, circulatory diseases and overeating *etc*.

On the other hand, antagonists that inhibit the transcription activity of PPAR γ or PPAR γ regulators that inhibit the expression of the protein itself are expected to be useful as hypoglycemic agents and agents for prevention and/or treatment of diseases associated with metabolic disorders such as diabetes, obesity and metabolic syndrome, *etc.,* hyperlipidemia, atherosclerosis, hypertension and overeating *etc.*

Additionally, the following fibrate compound (*e.g.*, chlofibrate) is known as a hypolipidemic agent. It is also resolved that one of the intracellular target proteins of fibrate compounds is PPAR α (see *Nature.,* 347, 645 (1990); *J. Steroid Biochem. Molec. Biol.,* 51, 157 (1994); *Biochemistry.,* 32, 5598 (1993)). From these facts, PPAR α, regulators which can be activated by fibrate compounds are thought to have a hypolipidemic effect, and so they are expected to be useful as agents for prevention and/or treatment of hyperlipidemia *etc.*

Besides, it has been recently reported that PPAR α possesses anti-obese activity in the specification of WO 9736579. In addition, it was reported that the elevation of high density lipoprotein (HDL) cholesterol level and the reduction of low density lipoprotein (LDL) cholesterol, very low density lipoprotein (VLDL) cholesterol and triglyceride levels were induced by activation of PPAR α *(J. Lipid Res.,* 39, 17 (1998)). It was also reported that composition of fatty acids in blood, hypertension and insulin resistance were improved by administration of bezafibrate which is one of fibrate compounds (Diabetes., 46, 348 (1997)). Therefore, agonists that activate PPAR α and PPAR α regulators that promote expression of PPAR α protein itself are useful as hypolipidemic agents and agents for treatment of hyperlipidemia, and are expected to have HDL cholesterol level-elevating effect, LDL cholesterol and/or VLDL cholesterol levels-lowering effect, inhibition on the progress of atherosclerosis and anti-obese effect. Therefore, they are thought to be hopeful agents for the treatment and/or prevention of diabetes as hypoglycemic agents, for the improvement of hypertension, for the relief from risk factor of metabolic syndrome and for the prevention of occurrence of ischemic coronary diseases.

On the other hand, few reports are found on ligands that activate PPAR δ significantly or on biological activities associated with PPAR δ.

PPAR δ is sometimes called PPAR β, or it is also called NUC1 in human. Until now, as for activity of PPAR δ, it is disclosed in the specification of WO 9601430 that hNUC1B (PPAR subtype whose structure is different from that of human NUC1 in one amino acid) inhibited the transcription activities of human PPAR α and thyroid hormone receptor. Recently in the specification of WO 9728149, it was reported that the compounds, which possessed high affinity to PPAR δ protein and which could activate PPAR δ significantly (i.e. agonists) were found out and that they had HDL (high density lipoprotein) cholesterol level-elevating activity. Therefore, agonists that can activate PPAR δ are expected to have HDL cholesterol level-elevating effect, and so they are expected to be useful for the inhibition on the progress of atherosclerosis and treatment thereof, as hypolipidemic agents and hypoglycemic agents, for the treatment of hyperlipidemia, as hypoglycemic agents, for the treatment of diabetes, for the relief from risk factor of metabolic syndrome, and for the prevention of occurrence of ischemic heart diseases.

In the specification of JP-A-9-323967, the compounds represented by formula (A) 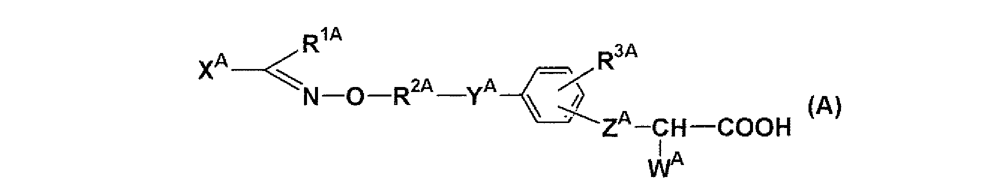 (wherein R^{1A} is alkyl *etc,;* R^{2A} is alkylene *etc.;* R^{3A} is hydrogen *etc*.; X^{A} is aryl *etc.;* Y^{A} is oxygen atom *etc.;* Z^{A} is alkylene *etc.;* W^{A} is alkyl *etc.)* are known to be useful for preventive·therapeutic agent for hyperlipidemia.

In addition, in the specification of WO 03/011807, the compounds represented bv formula (B) 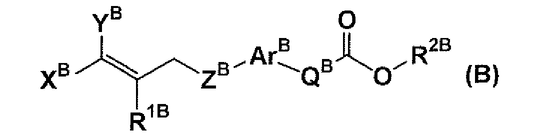 (wherein X^{B} is aryl *etc.;* Y^{B} is aryl, alkyl *etc.;* Z^{B} is O *etc.;* Q^{B} is -(CH₂)_{nB}- (nB is 0,1,2,3) *etc.;* R^{1B} is hydrogen atom, alkyl *etc.* R^{2B} is hydrogen atom *etc*.) are known to be useful for preventive therapeutic agent for PPAR mediated diseases.

Additionally, in the specification of WO 03/100812, the carboxylic acid derivatives represented by formula (C) 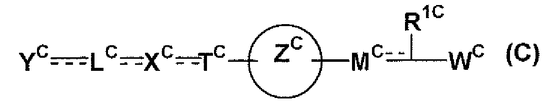 (wherein R^{1C} is carboxyl, alkyl *etc.;* L^{C}, T^{C} are single bond, alkylene *etc.;* M^{C} is alkylene *etc*.; W^{C} is carboxyl; X^{C} is hydrogen atom *etc.;* Y^{C}, Z^{C} are aromatic group etc.) have PPAR agonistic action and is known to be useful for improvement agent for insulin resistance.

In the specification of JP H6-87811, the compounds represented by formula (D) 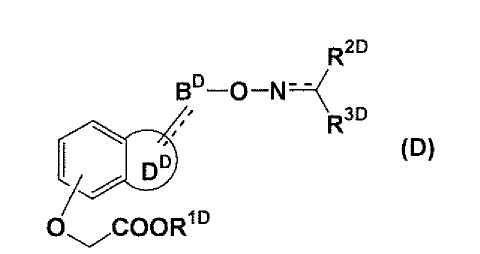 (wherein R^{1D} is hydrogen atom, C1-4 alkyl; B^{D} is hydrogen atom, C1-4 alkyl *etc.;* R^{2D} is hydrogen atom, C1-8 alkyl *etc.;* R^{3D} is C1-8 alkyl *etc.;* B^{D} is (CH₂)_{pD} (pD is an integer of 1 to 4.) etc.; ringD^{D} is carbocyclic ring.) have PGI2 agonistic action and is known to be useful for preventive and/or therapeutic agent for thrombosis, arteriosclerosis and so on.

In the specification of JP H6-56744, the compounds represented by formula (E) 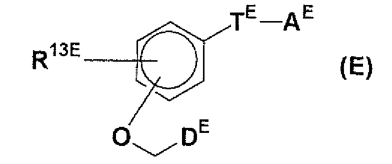 (wherein A^{E} is C(R¹)=NOR^{2E} *etc*. ; T^{E} is a single bond, C1-6 alkylene *etc;* D^{E} is CO₂R¹⁰, CONR¹¹R¹²; R^{13E} is hydrogen atom, C1-4 alkyl *etc*.) have PGI2 agonistic action and is known to be useful for preventive and/or therapeutic agent for thrombosis, arteriosclerosis and so on.

### DISCLOSURE OF THE INVENTION

It is longed that PPAR regulator which is useful for preventing treatment agent for hyperlipidemia *etc*., has superior oral absorption and is safe is developed.

As a result of the present inventors made further investigation to find out the compound which has PPAR regulatory action, they found out that the compound of the invention represented by formula (I) accomplished these purposes and the complete the present invention.

The present invention relates to
1. A compound represented by formula (I) 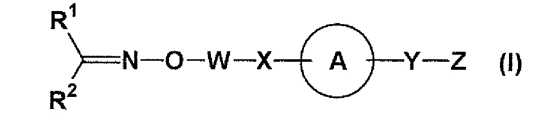 wherein R¹ and R² each independently represents (1) a hydrogen atom, (2) hydrocarbon which may have a substituent(s), (3) a cyclic group which may have a substituent(s), or (4) R¹ and R² are taken together to be a cyclic ring which may have a substituent(s),
   W represents a spacer of which main chain has an atom number of 1-6,
   X represents a single bond, -O-, -S-, -S(O)-, -SO₂- or -N(R³)-, in which R³ represents a hydrogen atom, alkyl which may have a substituent(s), acyl which may have a substituent(s), or alkoxycarbonyl which may have a substituent(s),
   ring A is a cyclic group which may further have a substituent(s),
   Y represents a single bond, or a spacer of which main chain has an atom number of 1-6,
   Z represents an acidic group,
   a salt thereof, a solvent thereof or an N-oxide thereof, or a prodrug thereof
2. The compound according to the above-described 1, wherein either R¹ or R² is alkyl which may have a substituent(s) or a cyclic ring which may have a substituent(s), a salt thereof, a solvent thereof or an N-oxide thereof, or a prodrug thereof.
3. The compound according to the above-described 1, wherein R¹ and R² are each a cyclic ring which may have a substituent(s), a salt thereof a solvent thereof or an N-oxide thereof, or a prodrug thereof.
4. The compound according to the above-described 1, wherein Y is unsubstituted -(CH₂)ₓ-, in which x represents an integer of 1 to 6, a salt thereof, a solvent thereof or an N-oxide thereof, or a prodrug thereof
5. The compound according to the above-described 1, wherein one of R¹ and R² is alkyl which may have a substituent(s), the other of R¹ and R² is a cyclic ring which may have a substituent(s), a salt thereof, a solvent thereof or an N-oxide thereof, or a prodrug thereof.
6. The compound according to the above-described 1, wherein ringA is a monocyclic ring which may have a substituent(s), a salt thereof, a solvent thereof or an N-oxide thereof, or a prodrug thereof.
7. The compound according to the above-described 1, wherein X is -O-, a salt thereof, a solvent thereof or an N-oxide thereof, or a prodrug thereof.
8. The compound according to the above-described 1, wherein W is C1-6 alkylene which may have a substituent(s), a salt thereof, a solvent thereof or an N-oxide thereof, or a prodrug thereof
9. The compound according to the above-described 1, wherein Z is carboxyl which may be esterified, a salt thereof, a solvent thereof or an N-oxide thereof, or a prodrug thereof.
10. The compound according to the above-described 1, which is selected from
   (1) (3-{2-[{(1E)-1-[4-(trifuloroemethyl)phenyl]propylidene}amino)oxy]ethoxy}phenyl)acetic acid,
   (2) {3-[2-({[(1E)-1-biphenyl-4-ylpropylidene]amino}oxy)ethoxy]phenyl}acetic acid,
   (3) (3-{2-[({(1E)-1-[4-(2-thienyl)phenyl]propylidene} amino)oxy]ethoxy}phenyl}acetic acid,
   (4) {3-[2-({[(1E)-1-(4-pyridin-2-ylphenyl)propylidene]amino}oxy)ethoxy]phenyl}acetic acid,
   (5) (3-{2-[({(1E)-1-[4-(1H-pyrazol-1-yl)phenyl]propylidene}amino)oxy]ethoxy}phenyl)acetic acid,
   (6) {3-[2-({[(1E)-1-biphenyl-4-ylpropylidene]amino}oxy)ethoxy]-2-methylphenyl}acetic acid,
   (7) (2-fuloro-3-{2-[({(1E)-1-[4-(1H-pyrazol-1-yl)phenyl]propylidene}amino)oxy]ethoxy}phenyl)acetic acid,
   (8) {2-fuloro-3-[2-({[{1E)-1-(4-pyridin-2-ylphenyl)propylidene]amino}oxy)ethoxy]phenyl}acetic acid,
   (9) (2-methyl-3-{2-[(1(1E)-[4-(1H-pyrazol-1-yl)phenyl]propylidene}amino)oxy]ethoxy}phenyl)acetic acid,
   (10) {2-methyl-3-[2-({[(1E)-1-(4-pyridin-2-ylphenyl)propylidene]amino}oxy)ethoxy]phenyl}acetic acid,
   (11) (2-methyl-3-{2-[({(1E)-1-[4-(trifuloromethyl)phenyl]propylidene}amino}oxy]ethoxy}phenyl)acetic acid,
   (12) {3-[2-({[(1E)-1-biphenyl-4-ylpropylidene]amino]oxy)ethoxy]-5-methoxyphenyl}acetic acid,
   (13) {3-[2-({[(1E)-1-biphenyl-4-ylbutylidene]amino}oxy}ethoxy]-2-methylphenyl}acetic acid,
   (14) {3-(2-({[(1E)-1-(3'-fulorobiphenyl-4yl)propylidene]amino}oxy}ethoxy]phenyl}acetic acid,
   (15) {3-[2-({[(1E)-1-biphenyl-4-yl-3-methylbutylidene]amino}oxy)ethoxy]-2-methylphenyl}acetic acid,
   (16) {2-methyl-3-[2-({[(1E)-6-phenyl-3,4-dihydronaphthalen-1(2H)-ylidene]amino}oxy)ethoxy]phenyl}acetic acid,
   (17) {3-[2-({[(1Z)-1-biphenyl-4-yl-2-(dimethylamino)ethylidene]amino}oxy)ethoxy]-2-methylphenyl}acetic acid,
   (18) (3-[2-({[(1Z)-1-biphenyl-4-yl-2-(1,3-thiazolidin-3-yl)ethylidene]amino}oxy)ethoxy]-2-methyphenyl}acetic acid,
   (19) {2-methyl-3-[2-({[(1E)-1-(4-pyridin-2-ylphenyl)butylidene]amino}oxy)ethoxy]phenyl}acetic acid,
   (20) (2-methyl-3-{2-[({(1E)-1-[4-(1H-pyrazol-1-yl)phenyl]butylidene}amino)oxy]ethoxy}phenyl)acetic acid,
   (21) {3-[2-({(1Z)-1-biphenyl-4-yl-2-(2,5-dihydro-1H-pyrol-1-yl)ethylidene]amino}oxy)ethoxy]-2-methylphenyl}acetic acid,
   (22) [3-(2-{[((1E)-1-biphenyl-4-yl-4,4,4-trifulorobutylidene)amino]oxy}ethoxy)-2-methylphenyl]acetic acid,
   (23) {2-methyl-3-[2-({[(1E)-1-(4-pyridin-2-ylphenyl)pentylidene]amino}oxy)ethoxy]phenyl}acetic acid,
   (24) (2-methyl-3-{2-[({(1E)-3-methyl-1-[4-(1H-pyrazol-1-yl)phenyl]butylidene}amino)oxy]ethoxy}phenyl)acetic acid,
   (25) [3-(2-{[((1Z)-1-biphenyl-4-yl-2-piperidin-1-ylethylidene)amino]oxy}ethoxy)-2-methylphenyl]acetic acid,
   (26) {3-[2-({[(1Z)-1-biphenyl-4-yl-2-(3,6-dihydropyridin-1(2H)-yl)ethylidene]amino}oxy)ethoxy]-2-methylphenyl}acetic acid,
   (27) [2-methyl-3-(2-{[((1E)-5-phenyl-2,3-dihydro-1H-inden-1-ylidene)amino]oxy}ethoxy)phenyl acetic acid,
   (28) [2-methyl-3-(2-{[((1E)-6-pyridin-2-yl-3,4-dihydronaphthalen-1(2H)-ylidene)amino]oxy}ethoxy)phenyl]acetic acid,
   (29) {2-methyl-3-[2-({[(1E)-3-methyl-1-(4-pyridin-2-ylphenyl)butylidene]amino}oxy)ethoxy]phenyl} acetic acid,
   (30) {2-methyl-3-[2-({[(1E)-6-(1H-pyrazol-1-yl)-3,4-dihydronaphthaten-1(2H)-ylidene]amino}oxy)ethoxy]phenyl}acetic acid,
   (31) (3-{2-[({(1Z)-2-(dimethylamino}-1-[4-(1H-pyrazol-1-yl)phenyl]ethylidene}amino)oxy]ethoxy}-2-methylphenyl)acetic acid,
   (32) {2-methyl-3-[2-{[(1Z)-1-[4-(1H-pyrazol-1-yl)phenyl]-2-(1,3-thiazolidin-3-yl)ethylidene]amino}oxy)ethoxy]phenyl}acetic acid,
   (33) {3-[2-({[(12)-2-(dimethylamino)-1-(4-pyridin-2-ylphenyl)ethyldene]amino}oxy)ethoxy]-2-methylphenyl}acetic acid, and
   (34) {2-methyl-3-[2-({[(1Z)-1-(4-pyridin-2-ylphenyl)-2-(1,3-thiazolidin-3-yl)ethylidene]amino}oxy)ethoxy]phenyl) acetic acid,
   a salt thereof, a solvent thereof or an N-oxide thereof, or a prodrug thereof
11. A pharmaceutical composition comprising the compound according to the above-described 1, a salt thereof, a solvent or an N-oxide or the prodrug thereof
12. The pharmaceutical composition according to the above-described 11, wherein the pharmaceutical composition is a preventive and/or therapeutic agent for a disease caused by PPARδ.
13. The pharmaceutical composition according to the above-described 12, wherein the disease caused by PPARδ is hyperlipidemia or adiposity.
14. A method for prevention and/or treatment for a disease caused by PPARδ in a mammal, which comprises administering to a mammal an effective amount of a compound represented by formula (I): 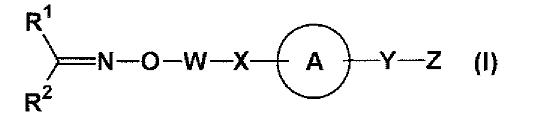 wherein all symbols have the same meanings as defined in the above-described 1, a salt thereof, a solvent thereof or an N-oxide thereof, or a prodrug thereof.
15. The method for prevention and/or treatment according to the above-described 14, wherein the disease caused by PPARδ is hyperlipidemia or adiposity.
16. Use of a compound represented by formula (I): 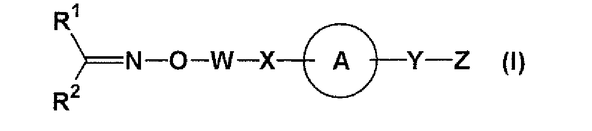 wherein all symbols have the same meanings as defined in the above-described 1, a salt thereof, a solvent thereof or an N-oxide thereof, or a prodrug thereof for preparing a prevention and/or treatment agent for a disease caused by PPARδ.
17. The use of the compound or a salt thereof according to the above-described 16, wherein the disease caused by PPARδ is hyperlipidemia or adiposity.
18. A medicament comprising the compound according to the above-described 1, a salt thereof, a solvent thereof or an N-oxide thereof, or a prodrug thereof and one kind or more kinds selected from an anti-adiposity drug, a therapeutic agent for diabetes and a lipid improvement drug.
19. The medicament according to the above-described 18, wherein the lipid improvement drug is an ACAT inhibitor, an MTP inhibitor, an HMG-CoA reductase inhibitor, a bile acid absorption inhibitor or a cholesterol absorption inhibitor.

In the specification, the definition of each group shows as follows

Cyclic group in cyclic ring which may have a substituent(s) represented by R¹ and R², cyclic group which may further have a substituent(s) represented by ringA, and cyclic ring which may have a substituent(s) represented by R¹ and R² taken together means, for example, carbocyclic ring and heterocyclic ring capable of existence in organic chemistry and so on.

Carbocyclic ring means, for example, C3-15 mono-, bi-, or tri-aromatic carbocyclic ring which may be partially or fully saturated and so on. C3-15 mono-, bi-, or tri-aromatic carbocyclic ring which may be partially or fully saturated means, for example, cyclopropane, cyclobutane, cyclopentane, cyclohexane, cycloheptane, cyclooctane, cyclononane, cyclodecane, cycloundecane, cyclododecane, cyclotridecane, cyclotetradecane, cyclopentacleeane, cyclopentene, cyclohexene, cycloheptene, cyclooctene, cyclopentadiene, cyclolzexadiene, cycloheptadiene, cyclooctadiene, benzene, pentalene, perhydropentalene, azulene, perhydroazulene, indene, perhydroindene, indane, naphthalene, dihydronaphthalene, tetrahydronaphthalene, perhydronaphthalene, heptalene, perhydroheptalene, biphenylene, as-indacene, s-indacene, acenaphthylene, acenaphtene, fluorene, phenalene, phenanthrene, anthracene and so on.

In addition, C3-15 mono-, bi-, or tri-aromatic carbocyclic ring which may be partially or fully saturated includes spire-linked bi-carbocyclic ring, and bridged bi-carbocyclic ring, for example, spiro[4.4]nonane, spiro[4.5]decane, spiro[5.5]undecane, bicyclo[2.2.1]heptane, bicyclo[2.2.1]hepta-2-ene, bicyclo[3.1.1]heptane, bicyclo[3.1.1]hepta-2-ene, bicyclo[2.2.2]octane, bicyclo[2.2.2]octa-2-ene, adamantane, noradamantane and so on.

Heterocyclic ring means, for example, 3-15 membered mono-, bi-, or tri-aromatic heterocyclic ring which may be partially or fully saturated containing 1 to 5 hetero atom(s) selected from oxygen, nitrogen and sulfur atom(s).

3-15 membered mono-, bi-, or tri-aromatic heterocyclic ring containing 1 to 5 hetero atom(s) selected from oxygen, nitrogen and sulfur atom(s) among 3-15 membered mono-, bi-, or tri-aromatic heterocyclic ring which may be partially or fully saturated containing 1 to 5 hetero atom(s) selected from oxygen, nitrogen and sulfur atom(s) means, for example, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, beta-carboline, acridine, phenazine, dibenzofuran, xanthene, dibenzothiophene, phenothiazine, phenoxazine, phenoxathiin, thianthrene, phenanthridine, phenanthroline, perimidine and so on.

3-15 membered mono-, bi-, or tri-aromatic heterocyclic ring partially or fully saturated containing 1 to 5 hetero atom(s) selected from oxygen, nitrogen and sulfur atom(s) among 3-15 membered mono-, bi-, or tri-aromatic heterocyclic ring which may be partially or fully saturated containing 1 to 5 hetero atom(s) selected from oxygen, nitrogen and sulfur atom(s) means, for example, aziridine, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxirane, oxetane, dihydrofuran, tetrahydrofuran, dihydropyran, tetrahydropyran, dihydrooxepine, tetrahydrooxepine, perhydrooxepine, thiirane, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindan, benzodioxane, chroman, benzodithiolane, benzodithiane and so on.

Substituent in cyclic ring which may have a substituent(s) represented by R¹ and R², cyclic group which may further have a substituent(s) represented by ringA, and cyclic ring which may have a substituent(s) represented by R¹ and R² taken together means, for example, (1) hydrocarbon which may have a substituent(s), (2) carbocyclic ring which may have a substituent(s), (3) heterocyclic ring which may have a substituent(s), (4) hydroxyl which may have a substituent(s), (5) mercapto which may have a substituent(s), (6) amino which may have a substituent(s), (7) carbamoyl which may have a substituent(s), (8) sulfamoyl which may have a substituent(s), (9) carboxyl which may be esterified, (10) sulfo, (11) sulfino, (12) phosphono, (13) nitro, (14) oxo, (15) thioxo, (16) cyano, (17) amidino, (18) imino, (19) dihydroxyborono, (20) halogen (*e.g.*, fluorine, chlorine, bromine, iodine), (21) alkylsulfinyl (*e.g.*, C1-4 alkylsulfinyl *etc*., such as methylsulfinyl, ethylsulfinyl and so on.) (22) arylsulfinyl (*e.g.,* C6-10 arylsulfinyl *etc.,* such as phenylsulfinyl and so on.), (23) alkylsulfonyl (*e.g.*, C1-4 alkylsulfonyl *etc*., such as methylsulfonyl, ethylsulfonyl and so on.), (24) arylsulfonyl (*e.g.*, C6-10 arylsulfonyl *etc*., such as phenylsulfonyl *etc*.), (25) acyl (C1-8 alkanoyl such as formyl, acetyl, propanoyl, butanoyl, pentanloyl, hexanoyl,heptanoyl, octanoyl, pivaloyl, C6-10 arylcarbony such as C6-10 arylcarbonyl and so on.) and so on. These optional substituents may be substituted at 1-5 replaceable positions.

Hydrocarbon in (1) hydrocarbon which may have a substituent(s) means, for example, alkyl, alkenyl, alkynyl or the like.

Alkyl means straight-chain or branched-chain C1-8 alkyl, for example, methyl, ethyl, n-propyl, isopropyl, n-butyl, isobutyl, sec-butyl, tert-butyl, pentyl, isopentyl, neopentyl, hexyl, heptiyl, octyl and so on.

Alkenyl means straight-chain or branched-chain C2-8 alkenyl, for example, ethenyl, propenyl, butenyl, pentenyl, hexenyl, heptenyl, octenyl and so on.

Alkynyl means straight-chain or branched-chain C2-8 alkynyl, for example, ethynyl, propynyl, butynyl, pentynyl, hexynyl, heptynyl, octynyl and so on.

Substituent in (1) hydrocarbon group which may have a substituent(s) means 1-5 group(s) selected from, for example, hydroxyl, C1-8 alkoxy (*e.g.*, methoxy, ethoxy, propoxy, isopropoxy, butoxy, isobutoxy, sec-butoxy, pentyloxy, isopentyloxy, neopentyloxy, hexyloxy, heptyloxy, octyloxy *etc*.), acyl (*e.g.,* C1-8 alkanoyl, such as formyl, acetyl, propanoyl, butanoyl, pentanoyl, hexanoyl, heptanoyl, octanoyl, pivaloyl and so on, C6-10 arylcarbonyl such as benzoyl and so on, *etc*.), amino, mono(C1-8 alkyl)amino (*e.g.*, methylamino, ethylamino, propylamino, butylamino, pentylamino, hexylamino, heptylamino, octylamino and the isomers thereof *etc.),* di(C1-8 alkyl)amino (*e.g*., dimethylamino, diethylamino, dipropylamino, dibutylamino, dipentylamino, dihexylamino, diheptylamino, dioctylamino, ethylmethylamino, methylpropylamino, ethylpropylamino and the isomers thereof *etc*.), C1-8 alkoxycarbonyl (*e.g.*, methoxycarbonyl, ethoxycarbonyl, propoxycarbonyl, butoxycarbonyl, pentyloxycarbonyl, hexyloxycarbonyl, pentyloxycarbonyl, octyloxycarbonyl, tert-butoxycarbonyl *etc*.), benzyloxycarbonyl, carboxyl, mercapto, C1-8 alkylthio (*e.g.*, methylthio, ethylthio, propylthio, butylthio, pentylthio, hexylthio, heptylthio, octylthio *etc*.), halogen (*e.g.*, fluorine, chlorine, bromine, iodine), (C1-8 alkyl)sulfonyl (*e.g.*, methylsulfonyl, ethylsufonyl, propylsulfonyl, butylsulfonyl, pentylsulfonyl, hexylsulfonyl, heptylsulfonyl, octylsulfonyl and the isomers thereof *etc*.), (C1-8 alkyl)sulfanylamino (*e*.*g*., methylsulfonylamino, ethylsulfonylamino, propylsulfonylamino, butylsulfanylamino, pentylsulfonylamino, hexylsulfonylamino, heptylsufonylamino, octylsufonylamino and the isomers thereof *etc*.), C2-8 acylamino (*e.g.*, acetylamino, propionylamino, butyrylamino, valerylamino, hexanoylamino, heptanoylamino, octanoylamino and the isomers thereof *etc.*), C1-8 acyloxy (*e.g.*, formyloxy, acetyloxy, propanoyloxy, butanoyloxy, pentanoyloxy, hexanoyloxy, octanoyloxy and the isomers thereof *etc*.), oxo, cyano, nitro, carbocyclic ring and heterocyclic ring (carbocyclic ring and heterocyclic ring have the same meaning as carbocyclic ring and heterocyclic ring of cyclic ring in cyclic ring which may have a substituent(s) represented by R¹ and R².).

Carbocyclic ring in (2) carbocyclic ring which may have a substituent(s) and (3) heterocyclic ring in heterocyclic ring which may have a substituent(s) have the same meanings as carbocyclic ring and heterocyclic ring of cyclic ring in cyclic ring which may have a substituent(s) represented by R¹ and R².

Substituent in (2) carbocyclic ring which may have a substituent(s) and (3) heterocyclic ring which may have a substituent(s) means, for example, C1-4 alkyl (*e.g*., methyl, ethyl, propyl, butyl *etc.),* C2-4 alkenyl (*e.g*., ethenyl, propenyl, butenyl *etc*.), C2-4 alkynyl (*e.g*., ethynyl, propynyl, butynyl *etc*.), hydroxyl, C1-4 alkoxy (*e.g*., methoxy, ethoxy, propoxy, butoxy *etc*.), mercapto, C1-4 alkylthio (*e.g.*, methylthio, ethylthio, propylthio, butylthio *etc*.), amino, mono- or di-C1-3 alkylamino (*e.g*., methylamino, ethylamino, propylamino, dimethylamino, diethylamino *etc.*), halogen atom (*e.g*., fluorine, chlorine, bromine, iodine), cyano, nitro, mono- or di-C1-4 alkylaminocarbonyl (*e.g*., methylaminocarbonyl, ethylaminocarbonyl, propylaminocarbonyl, dimethylaminocarbonyl, diethylaminocarbonyl *etc*.), and so on. These optional substituents may be substituted at 1-4 replaceable positions.

Substituent in (4) hydroxyl which may have a substituent(s), (5) mercapto which may have a substituent(s), (6) amino which may have a substituent(s), (7) carbamoyl which may have a substituent(s), (8) sulfamoyl which may have a substituent(s) means, for example, alkyl (*e.g.*, straight chain and branched chain C1-4 alkyl *etc*., such as methyl, ethyl, n-propyl, isopropyl, n-butyl, isobutyl, sec-butyl, tert-butyl and so on.), aryl (*e.g.*, phenyl, 4-methylphenyl *etc.*), aralkyl (*e.g.*, benzyl, phenetyl *etc.),* acyl (C1-6 alkanoyl *(e.g.,* formyl, acetyl, propanoyl, pivaloyl *etc*.), arylcarbonyl (*e.g.*, benzoyl *etc*.) and so on.), alkoxycarbonyl (*e.g.*, t-butoxycarbonyl *etc*.), aralkyloxycarbonyl (*e.g.*, benzyloxycarbonyl *etc.*) and so on. In addition, as amino which may have a substituent(s), two substituents may be taken together to form cyclic amino (*e.g.*, aziridine, azetidine, pyrrolidine, piperidine, azepane, morpholine, thiomorpholine, piperazine which may be substituted with lower alkyl (*e.g.*, methyl, ethyl, propyl, butyl *etc*.) *etc.*), (9) carboxyl which may be esterified means, for example, free carboxyl, alkoxycarbonyl (*e.g.*, C1-6 alkoxycarbonyl *etc.,* such as, methoxycaronyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl and so on.), aryloxycarbonyl (*e.g.,* C6-10 aryloxycarbonyl *etc.*, such as, phenoxycarbonyl, 2-naphthyloxycarbonyl and so on.), aralkyloxycarbonyl (*e*.*g*., C6-10 aryl-C1-4 alkoxycarbonyl *etc*., such as, benzyloxycarbonyl, phenethyloxycarbonyl and so on) and so on.

Hydrocarbon which may have a substituent(s) represented by R¹ and R² has the same meanings as the above-mentioned (1) hydrocarbon which may have a substituent(s).

Spacer that of which atom number of main chain represented by W is 1-6 means the distance that 1-6 atom(s) of main chain is(are) connected. Here, atom number of main chain is counted to be minimal. As spacer that of which atom number of main chain represented by W, for example, C1-6 alkylene which may have a substituent(s) (*e.g.*, -CH₂-, -(CH₂)₂-, -(CH₂)₃-, -(CH₂)₄-, -(CH₂)₅-, -(CH₂)₆- *etc*.), C2-6 alkenylene which may have a substituent(s) (*e.g.*, -CH=CH-, -CH₂-CH=CH-, -CH=CH-CH₂-, -(CH₂)₂-CH=CH-, -CH=CH-(CH₂)₂-, -CH₂-CH=CH-CH₂- *etc.*), C2-6 alkynylene which may have a substituent(s) (*e.g.*, -C≡C-, -CH₂-C≡C-, -C≡C-CH₂-, -(CH₂)₂-C≡C-, -C≡C-(CH₂)₂)-, -CH₂-C≡C-CH₂- *etc.*) and so on. Here, substituent of C1-6 alkylene, C2-6 alkenylnene and C2-6 alkynylene means, for example, halogen atom (*e.g.*, fluorine, chlorine, bromine, iodine), hydroxyl and so on. These optional substituents may be substituted at 1-3 replaceable positions.

Spacer of which main chain has an atom number of 1-6 represented by Y means the distance that 1-6 atom(s) of main chain is(are) connected. Here, the atom number of main chain is counted to be minimal. The spacer of which main chain has an atom number of 1-6 represented by Y includes C1-6 alkylene which may have a substituent(s) (*e.g.,* -CH₂-, -(CH₂)₂-, -(CH₂)₃-, -(CH₂)₄-, -(CH₂)₅-, -(CH₂)₆- *etc.*), C2-6 alkenylene which may have a substituent(s) (*e*.*g*., -CH=CH-, -CH₂-CH=CH-, -CH=CH-CH₂-, -(CH₂)₂-CH=CH-, -CH=CH-(CH₂)₂-, -CH₂-CH=CH-CH₂- *etc*.), C2-6 alkynylene which may have a substitutent(s) (*e*.*g*., -C≡C-, -CH₂-C≡C-, -C≡C-CH₂-, -(CH₂)₂-C≡C--C≡C-(CH₂)₂-, -CH₂-C≡C-CH₂- *etc*.), -(CH₂)ₘ-O-(CH₂)ₙ- which may have a substituent(s), -(CH₂)ₘ-S-(CH₂)ₙ- which may have a substituent(s), -(CH₂)ₘ-S(O)-(CH₂)ₙ- which may have a substituent(s), -(CH₂)ₘ-SO₂-(CH₂)ₙ- which may have a substituent(s), -(CH₂)ₘ-N(R⁴)-(CH₂)ₙ- which may have a substituent(s) (wherein R⁴ is hydrogen atom, or alkyl which may have a substituent(s).) and so on. m and n are 0 or an integer of 1-5, respectively, and the sum of m and n is 0-5. Additionally, the same carbon atom or the neighboring multiple carbon atoms may be taken together to form cyclic group (e.g., rings indicated by cyclic ring L formed by the same carbon 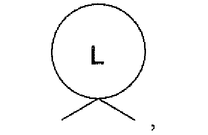 cyclic ring M formed by the neighboring two carbon atoms 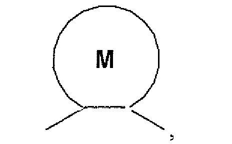 cyclic ring N formed by the neighboring three carbon atoms 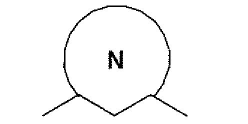 (the rings indicated by the above-mentioned L, M, N are rings capable of existence in organic chemistry.) *etc*.)

RingL means, for example, aromatic carbocyclic ring saturated partilally or fully (dihydronaphthalene, tetrahydronaphthalene, cycloalkane (cyclopropane, cyclobutan, cyclopentane, cyclohexane, cycloheptane, cyclooctane *etc*.), cycloalkene (cyclopentene, cyclohexene, cycloheptene *etc*.) *etc*.), aromatic heterocyclic ring saturated partially or fully (piperidine, pyrrolidine, morpholine, tetrahydrofuran, tetrahydrothiophen *etc*.) and so on.

RingM and ringN means, for example, aromatic carbocyclic ring which may by partially or fully saturated (cycloalkane (cyclopropane, cyclobutane, cyclopentane, cyclohexane, cycloheptane, cyclooctane *etc*.), cycloakene (cyclopentene, cyclohexene, cycloheptene *etc*.), dihydronaphthalene, tetrahydronaphthalene, benzene *etc*.), aromatic heterocyclic ring which may be partially or fully saturated (pyridine, furan, thiophene, quinoline, isoquinoline, benzofuran, benzothiophene, piperidine, morpholine, tetrahydrofuran, tetrahydrothiophene, pyrolidine *etc*.) *etc*.) and so on.

In addition, substituent of C1-6 alkylene, C2-6 alkenylene, C2-6 alkynylene, -(CH₂)ₘ-O-(CH₂)ₙ-, -(CH₂)ₘ-S-(CH₂)ₙ-, -(CH₂)ₘ-S(O)-(CH₂)ₙ-, -(CH₂)ₘ-SO₂-(CH₂)ₙ-, and -(CH₂)ₘ-NR⁴-(CH₂)ₙ- means, for example, halogen atom (*e.g*., fluorine, chlorine, bromine, iodine), hydroxyl, carboxyl which may be esterified (carboxyl which may be esterified has the same meanings as the above-mentioned (9) carboxyl which may be esterified.) and so on. These optional substituents may be substituted at 1-3 replaceable positions.

Acidic group represented by Z means, for example, carboxyl which may be esterified (carboxyl which may be esterified has the same meanings as the above-mentioned (9) carboxyl which may be esterified.), sulfo, -SO₂NHR⁵(R⁵ is hydrogen atom, or hydrocarbon which may have a substituent(s).), -NHSO₂R⁶-(R⁶ is hydrocarbon which may have a substituent(s).), phosphono (-PO(OH)₂), phenol (-C₆H₄OH) or various Broensted acid such as residue of nitrogen ring having deprotonatable hydrogen atom *etc.* Broensted acid indicates a substance which gives hydrogen atom to other substance. Residue of nitrogen ring having deprotonatable hydrogen atom means, for example, 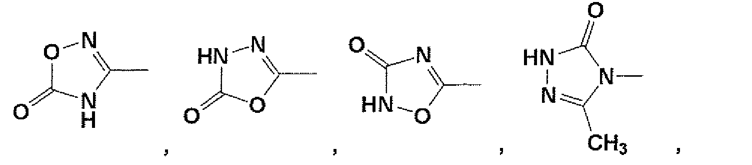 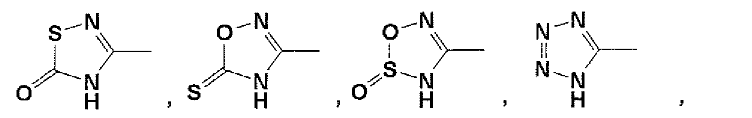 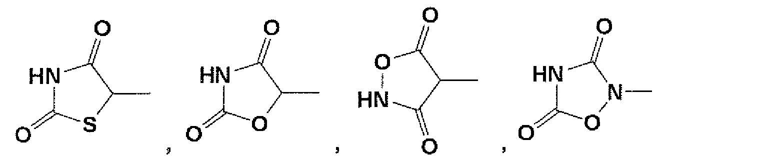 and so on.

Alkyl in alkyl which may have a substituent(s) represented by R³ and R⁴ has the same meanings as alkyl in the above-mentioned hydrocarbon which may have a substituent(s), and substiutuent has the same meanings as substituent in hydrocarbon which may have a substituent(s).

Acyl in acyl which may have a substituent(s) represented by R³ has the same meanings as (25) acyl of substituent in cyclic group which may have a substituent(s).

Alkoxycarbonyl in alkoxycarbonyl which may have a substituent(s) represented by R³ means, for example, C1-8 alkoxycarbonyl *etc*., such as methoxycarbonyl, ethoxycarbonyl, propoxycarbonyl, butoxycarbonyl, pentyloxycarbonyl, hexyloxycarbonyl, heptyloxycarbonyl, octyloxycarbonyl and so on. Substituent in acyl which may have a substituent(s) and alkoxycarbonyl which may have a substituent(s) represented by R³ has the same meaning as substituent in hydrocarbon which may have a substituent(s).

Hydrocarbon which may have a substituent(s) represented by R⁵ and R⁶ has the same meaning as the above-mentioned (1) hydrocarbon which may have a substituent(s).

As carbocyclic ring represented by R¹ and R², C5-10 mono-, or bi-aromatic carbocyclic ring which may have a substituent(s) and may be partially or fully saturated is preferred, respectively. C5-10 mono-aromatic carbocyclic ring which may have a substituent(s) is more preferred. Benzene which may have a substituent(s) is particularly preferred.

As heterocyclic ring represented by R¹ and R², 5-10 membered mono-, or bi-aromatic heterocyclic ring which may have a substituent(s) and may be partially or fully saturated containing 1 to 3 hetero atom(s) selected from oxygen, nitrogen and sulfur atom(s) is preferred. Pyridine which may have a substituent(s) is more preferred.

As hydrocarbon which may have a substituent(s) represented by R¹ and R², C1-6 alkyl which may have a substituent(s) is preferred. C1-3 alkyl which may have a substituent(s) is more preferred.

As cyclic ring represented by R¹ and R² taken together, aromatic carbocyclic ring which may be partially or fully saturated is preferred. C6-10 aromatic carbocyclic ring partially saturated is more preferred. 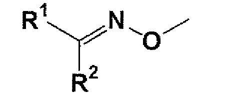 which is tetrahydronaphthalene ring represented by 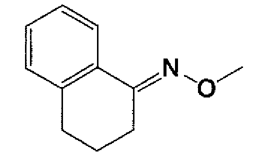 is particularly preferred.

As the combination of R¹ and R², it is preferred that one is cyclic ring which may have a substituent(s), the other hydrocarbon which may have a substituent(s).It is more preferred that one is carbocyclic ring which may have a substituent(s), the other C1-6 alkyl which may have a substituent(s).

As spacer represented by W that of which atom number of main chain is 1-6, C1-6 alkylene is preferred. C2-4 alkylene (-(CH₂)₂-, -(CH₂)₃-,-(CH₂)₄-) is more preferred.

As the group represented by X, single bond, -O-,-S- and -N(R³)- are preferred. -O-is more preferred.

As cyclic ring represented by ringA, C3-15 mono-, bi-, or tri-aromatic carbocyclic ring, or 3-15 membered mono-, bi-, or tri-aromatic heterocyclic ring containing 1 to 5 hetero atom(s) selected from oxygen, nitrogen and sulfur atom(s) is preferred. Mono-carbocyclic ring (C3-8 cycloalkyl *e.g.,* cyclopropyl, cyclobutyl, cyclopentyl, cyclohexyl, cyclohe0ptyl, cyclooctyl *etc*., benzene, cyclohexene, cyclohexadiene, cylcoheptene, cycloheptadiene, cyclooctene, cyclooctadiene, cyclooctatriene *etc*.) or bi-heterocyclic ring (benzothiophene, benzofuran, indole, indoline, benzoxazol, benzothiazol, quinoline, isoquinoline *etc*.) is more preferred. Benzene is particularly preferred.

As substituent in ringA which may have a further substituent(s), C1-4 alkyl, halogen, C1-4 alkoxy are preferred. Methyl, fluoro, chloro, methoxy are more preferred.

As spacer represented by Y that of which atom number of main chain is 1-6, (CH₂)ₓ (x is an integer of 1-6), i.e. methylene, ethylene, triethylene, tetramethylene, pentamethylene, hexamethylene are preferred (it is preferred that carbon atom in spacer forms ringL or ringN.). Straight chain C1-2 alkylene (methylene, ethylene) is more preferred. C1 alkylene (methylene) is particularly preferred.

As substituent in Y which may have a further substituent(s), carboxyl which may be esterified is preferred.

As acidic group represented by Z, carboxy which may be esterified or various Broensted acid *e.g.,* residue of nitrogen ring *etc.* is preferred.

As carboxyl which may be esterified in acidic group represented by Z, carboxyl, methoxycarbonyl and ethoxycarbonyl are preferred.

Any compound of the present invention is preferred. For example, the compound represented by formula (IA) 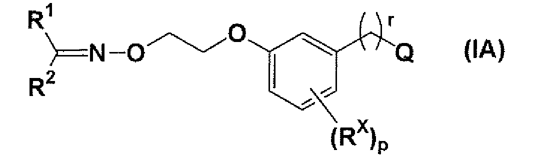 (wherein R^{x} has the same meaning as the substituent of ringA, p is 0 or an integer of 1-4, R^{z} is substituent of Y, Q is acidic group, r is an integer of 1 to 6, and the other symbols have the same meanings as that of the above-mentioned.), the compound represented by formula (IB) 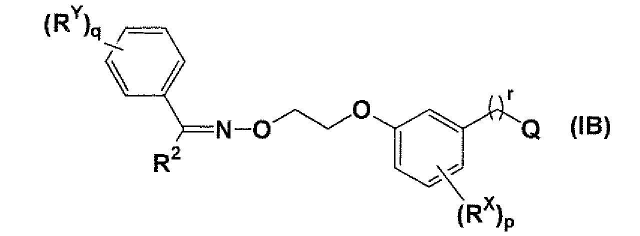 (wherein R^{Y} is substituent of R¹, q is 0 or an integer of 1 to 5, the other symbols have the same meanings as that of the above-mentioned.), the compound represented by formula (IC) 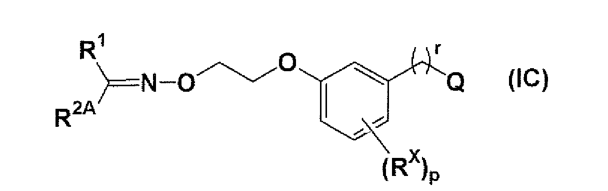 (wherein R^{2A} is C1-6 alkyl which may have a substituent(s), the other symbols have the same meanings as that of the above-mentioned.), the compound represented by formula (ID) 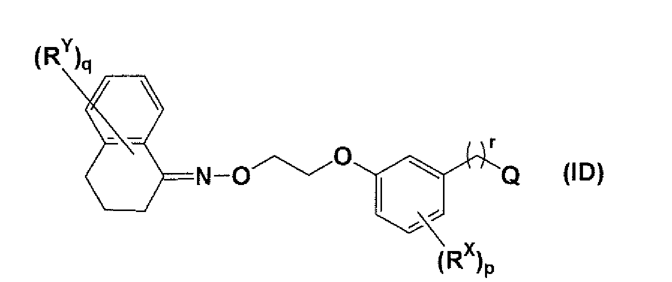 (wherein the other symbols have the same meanings as that of the above-mentioned.) is preferred.

The particularly preferred compounds include, concretely,
(1) (3-{2-[{(1E)-1-[4-(trifluoroemethyl)phenyl]propylidene}amino)oxy]ethoxy}phenyl)acetic acid,
(2) {3-[2-({[(1E)-1-biphenyl-4-ylpropylidene]amino}oxy)ethoxy]phenyl}acetic acid,
(3) (3-{2-[({(1E)-1-[4-(2-thienyl)phenyl]propylidene}amino)oxy]ethoxy}phenyl}acetic acid,
(4) {3-[2-({[(1E)-1-(4-pyridin-2-ylphenyl)propylidene]amino}oxy)ethoxy]phenyl}acetic acid,
(5) (3-{2-[({(1E)-1-[4-(1H-pyrazol-1-yl)phenyl]propylidene}amino)oxy]ethoxy}phenyl)acetic acid,
(6) {3-[2-({[(1E)-1-biphenyl-4-ylpropylidene]amino}oxy)ethoxy]-2-methylphenyl} acetic acid,
(7) (2-fuloro-3-{2-[({(1E)-1-[4-(1H-pyrazol-1-yl)phenyl]propylidene}amino)oxy]ethoxy}phenyl}acetic acid,
(8) {2-fuloro-3-[2-({[(1E)-1-(4-pyridin-2-ylphenyl)propylidene]amino}oxy)ethoxy]phenyl}acetic acid,
(9) (2-methyl-3-{2-[{(1E)-[4-(1H-pyrazol-1-yl)phenyl]propylidene}amino)oxy]ethoxy}phenyl)acetic acid,
(10) {2-methyl-3-[2-({[(1E)-1-(4-pyridin-2-ylphenyl)propylidene]amino}oxy)ethoxy]phenyl}acetic acid,
(11) (2-methyl-3-(2-[({(1E)-1-[4-(trifuloromethyl)phenyl]propylidene}amino)oxy]ethoxy}phenyl)acetic acid,
(12) {3-[2-({[(1E)-1-biphenyl-4-ylpropylidene]amino}oxy)ethoxy]-5-methoxyphenyl} acetic acid,
(13) {3-(2-({[(1E)-1-biphenyl-4-ylbutylidene]amino}oxy)ethoxy]-2-methylphenyl}acetic acid,
(14) {3-[2-({[(1E)-1-(3'-fulorobiphenyl-4yl)propylidene]amino}oxy)ethoxy]phenyl}acetic acid,
(15) {3-[2-({[(1E)-1-biphenyl-4-yl-3-methylbutylidene]amino}oxy)ethoxy]-2-methylphenyl}acetic acid,
(16) {2-methyl-3-[2-({[(1E)-6-phenyl-3,4-dihydronaphthalen-1(2H)-ylidene]amino}oxy)ethoxy]phenyl}acetic acid,
(17) {3-[2-({[(1Z)-1-biphenyl-4-yl-2-(dimethylamino)ethylidene]amino}oxy)ethoxy]-2-methylphenyl}acetic acid,
(18) {3-[2-({[(1Z)-1-biphenyl-4-yl-2-(1,3-thiazolidin-3-yl)ethylidene]amino}oxy)ethoxy]-2-methyphenyl}acetic acid,
(19) {2-methyl-3-[2-({[(1E)-1-(4-pyridin-2-ylphenyl)butylidene]amino}oxy)ethoxy]phenyl}acetic acid,
(20) (2-methyl-3-{2-[({(1E)-1-[4-(1H-pyrazol-1-yl)phenyl]butylidene}amino)oxy]ethoxy}phenyl)acetic acid,
(21) {3-[2-({[(1Z)-1-biphenyl-4-yl-2-(2,5-dihydro-1H-pyrol-1-yl)ethylidene]amino}oxy)ethoxy]-2-methylphenyl} acetic acid,
(22) [3-(2-{[((1E)-1-biphenyl-4-yl-4,4,4-trifulorobutylidene)amino]oxy}ethoxy)-2-methylphenyl]acetic acid,
(23) {2-methyl-3-[2-({[(1E)-1-(4-pyridin-2-ylphenyl)pentylidene]amino}oxy)ethoxy]phenyl}acetic acid,
(24) (2-methyl-3-{2-[({(1E)-3-methyl-1-[4-(1H-pyrazol-1-yl)phenyl]butylidene}amino)oxy]ethoxy}phenyl)acetic acid,
(25) [3-(2-{[((1Z)-1-biphenyl-4-yl-2-piperidin-1-ylethylidene)amino]oxy}ethoxy)-2-methylphenyl]acetic acid,
(26) {3-[2-({[(1Z)-1-biphenyl-4-yl-2-(3,6-dihydropyridin-1(2H)-yl)ethylidene]amino}oxy)ethoxy]-2-methylphenyl}acetic acid,
(27) [2-methyl-3-(2-{[((1E)-5-phenyl-2,3-dihydro-1H-inden-1-ylidene)amino]oxy}ethoxy)phenyl acetic acid,
(28) [2-methyl-3-(2-{[((1E)-6-pyridin-2-yl-3,4-dihydronaphthalen-1(2H)-ylidene)amino]oxy}ethoxy)phenyl]acetic acid,
(29) {2-methyl-3-[2-({[(1E)-3-methyl-1-(4-pyridin-2-ylphenyl)butylidene]amino} oxy)ethoxy]phenyl} acetic acid,
(30) {2-methyl-3-[2-({[(1E)-6-(1H-pyrazol-1-yl)-3,4-dihydronaphthalen-1(2H)-ylidene]amino}oxy)ethoxy]phenyl}acetic acid,
(31) (3-{2-[({(1Z)-2-(dimethylamino)-1-[4-(1H-pyrazol-1-yl)phenyl]ethylidene}amino)oxy]ethoxy}-2-methylphenyl)acetic acid,
(32) {2-methyl-3-[2-({[(1Z)-1-[4-(1H-pyrazol-yl)phenyl]-2-(1,3-thiazolidin-3-yl)ethylidene]amino}oxy)ethoxy]phenyl}acetic acid,
(33) {3-[2-({[(1Z)-2-(dimethylamino)-1-(4-pyridin-2-ylphenyl)ethylidene]amino}oxy)ethoxy]-2-methylphenyl}acetic acid, and
(34) {2-methyl-3-[2-({[(1Z)-1-(4-pyridin-2-ylphenyl)-2-(1,3-thiazolidin-3-yl)ethylidene]amino}oxy)ethoxy]phenyl}acetic acid.

Unless otherwise specified, all isomers are included in the present invention. For example, alkyl, alkenyl, alkynyl, alkoxy, alkylthio, alkylene alkenylene and alkynylene group means straight-chain or branched-chain ones. In addition, isomers on double bond, ring, fused ring (E-, Z-, cis-, trans-isomer), isomers generated from asymmetric carbon atom(s) (R-, S-isomer, α-, β-configuration, enantiomer, diastereomer), optically active isomers (D-, L-, d-, 1-isomer), polar compounds generated by chromatographic separation (more polar compound, less polar compound), equilibrium compounds, rotational isomers, mixtures thereof at voluntary ratios and racemic mixtures are also included in the present invention.

In the present invention, unless otherwise specified, the symbol 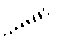 means that the α-configuration substituent, the symbol 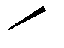 means that the β-configuration substituent, the symbol 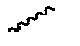 means α-configuration, β-configuration or a voluntary mixture of α-configuration and β-configuration, and the symbol 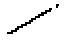 means that there is a voluntary mixture of α-configuration and β-configuration as would be clear to the person skilled in the art.

### [Salt, N-oxide and solvent]

The salts of the compounds represented by formula (I) include all of pharmaceutically acceptable ones. As pharmaceutically salts, non-toxic, water-soluble salts are preferred. The suitable salts include for example, salts of alkali metals (*e*.*g*., potassium, sodium, lithium, *etc*.), salts of alkaline earth metals (*e*.*g*., calcium, magnesium, *etc*.), ammonium salts (*e.g.*, tetramethylammonium salt, tetrabutylammonium salt, *etc*.), pharmaceutical acceptable salts of organic amine (*e.g*., triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc*.), acid addition salts (salts of inorganic acids (*e.g*., hydrochloride, hydrobromide, hydroiodide, sulfate, phosphate, nitrate *etc*.), and salts of organic acids (*e.g.,* acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate *etc*.). In addition, N-oxide means nitrogen atom of the compound represented by formula (I) is oxidized. The compound of the invention may be converted into N-oxide by voluntary methods. The salts and N-oxide of the compound of the invention include solvent, or the above-mentioned solvents of salts of alkali (earth) metals, ammonium salts, salts of organic amine and acid addition salts of the compound of the invention. The suitable solvates include for example, hydrates, solvates of the alcohols (*e.g.,* ethanol *etc*.), and so on. The compound of the invention is converted into pharmaceutically acceptable salt by known methods.

### Prodrug:

Additionally, the prodrug of the compounds represented by formula (I) means a compound is the compound represented by formula (I) by reaction with enzymes, gastric acids and so on within an organism. The prodrug of the compounds represented by formula (I) include, when the compounds represented by formula (I) have amino, the prodrug is the compounds the amino of which is acylated, alkylated, phosphorylated (*e*.*g*., the compounds are that the amino of the compounds represented by formula (I) is eicosanoated, alanylated, pentylaminocarbonylated, (5-methyl-2-oxo-1,3-dioxolan-4-yl)methoxycarbonylated, tetrahydrofuranated, pyrrolidylmethylated, pivaloyloxymethylated, acetoxymethylated, tert-butylated, *etc*.); when the compounds represented by formula (I) have hydroxyl, the prodrug is the compounds the hydroxyl of which are acylated, alkylated, phosphorylated, borated (*e.g.*, the compounds are that the hydroxyl of the compounds represented by formula (I) are acetylated, palmitoylated, propanoylated, pivaloylated, succinylated, fumarylated, alanylated, dimethylaminomethylcarbonylated *etc*.); when the compounds represented by formula (I) have carboxyl, the prodrug is the compound the carboxyl of which are esterified, amidated (*e*.*g*., the compounds are that the carboxyl of the compounds represented by formula (I) is ethylesterified, isopropylesterified, phenylesterified, carboxymethylesterified, dimethylaminomethylesterified, pivaloyloxymethylesterified, ethoxycarbonyloxyethylesterified, phthalidylesterified, (5-methyl-2-oxo-1,3-dioxolen-4-yl)methylesterified, cyclohexyloxycarbonylethylesterified, methylamidated *etc*.); and so on. In addition, the prodrug of the compound represented by formula (I) may be either hydrate or non-hydrate.

In the present invention, PPAR regulator includes all regulators of PPAR α regulator, PPAR γ regulator, PPAR δ regulator, PPAR α+γ regulator, PPAR α+δ regulator, PPAR γ+δ regulator, PPAR α+γ+δ regulator. In addition, as regulatory fashion of the invention, PPAR δ regulator, or PPAR δ+α regulator is preferred. Additionally, PPAR regulator of the invention includes PPAR agonist and PPAR antagonist. PPAR agonist is preferred. PPAR δ agonist or PPAR δ+α agonist is more preferred.

### Processes for the Preparation of the compound of the present invention:

The compound of the present invention represented by formula (I) can be prepared by combining the known processes, for example, the following processes, or the processes shown in Examples, which is the properly improved processes described in *"Comprehensive Organic Transformations : A Guide to Functional Group Preparations,* 2nd Edition "Richard C. Larock, Wiley & Sons Inc, 1999" and so on, Still, ingredients may be used as salts in the following each processes for the preparation. As these salts, the salts decribed as the pharmaceutically acceptable ones in the above-mentioned formula (I) are used.

### [1] The compound represented by formula (I) can be prepared by reacting a compound represented by formula (II)

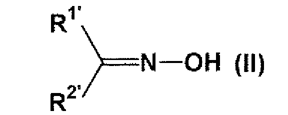

(wherein R^{1'} and R^{2'} have the same meaning as R¹ and R², respectively, but carboxyl, hydroxyl, amino and mercapto included the group represented by R^{1'} and R^{2'} are, if necessary, protected.) with a compound represented by formula (III) 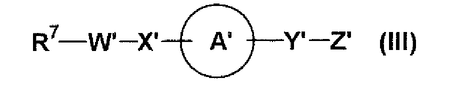 (wherein W', X', ringA', Y' and Z' have the same meanings as that of W, X, ringA, Y and Z, but carboxyl, hydroxyl, amino and mercapto included these group are, if necessary, protected. R⁷ is elimination group (halogen atom (*e*.*g*., fluorine, chlorine, bromine, iodine), mesyloxy, tosyloxy *etc*.)), if necessary, followed by subjecting to a deprotection reaction of the protective group.

The above-mentioned reaction is known. It is carried out, for example, in an organic solvent (*e*.*g*., tetrahydrofuran (THF), diethylether, methylene chloride, chloroform, carbon tetrachloride, pentane, hexane, benzene, toluene, N,N-dimethylformamide (DMF), dimethylsulfoxide (DMSO), hexamethylphosphoramide (HMPF), acetonitrile, *etc*.) in the presence of a base (*e.g.*, sodium hydroxide, potassium carbonate, potassium phosphate, potassium t-butoxide, triethylamine, pyridine, sodium iodide, cesium carbonate, *etc*.) at a temperature of 0 to 100°C.

The deprotection reaction of the protective group may be carried out by following method. The deprotectin reaction of a protective group for carboxyl, hydroxyl, amino, or mercapto is known, and it includes
(1) alkaline hydrosis,
(2) deprotection reaction under acidic conditions,
(3) deprotection reaction by hydrogenolysis,
(4) deprotection reaction of a silyl group,
(5) deprotection reaction using metals,
(6) deprotection reaction using metal complexes, and so on.

These methods are described concretely as follows.
(1) The deprotection reaction by alkaline hydrolysis is, for example, carried out in an organic solvent (*e.g*., methanol, tetrahydrofuran, or dioxane *etc*.) using a hydroxide of an alkali metal (*e*.*g*., sodium hydroxide, potassium hydroxide, or lithium hydroxide *etc*.), a hydroxide alkaline earth metal (*e*.*g*., barium hydroxide, or calcium hydroxide *etc*.), or a carbonate (*e*.*g*., sodium carbonate or potassium carbonate, *etc*.), or an aqueous solution thereof, or a mixture thereof at a temperature of 0 to 40°C.
(2) The deprotection reaction under acidic conditions is carried out, for example, in an organic solvent (*e.g*., dichloromethane, chloroform, dioxane, ethyl acetate, or anisole *etc*.) in an organic acid (*e.g*., acetic acid, trifuloroacetic acid, methansulfonic acid, or p-tosylate, *etc*.), or an inorganic acid (*e.g*., hydrochloric acid, or sulfuric acid, *etc.*) or a mixture thereof (*e.g*., hydrogen bormide/acetic acid, *etc*.) at a temperature of 0 to 100°C.
(3) The deprotection reaction by hydrogenolysis is carried out, for example, in a solvent (*e.g.,* ethers (*e.g*., tetrahydrofuran, dioxane, dimethoxyethane (DME), or diethylether, *etc*.), alcohols (*e.g*., methanol, or ethanol, *etc*.), benzenes (*e.g.,* benzene, or toluene *etc*.), ketones (*e.g*., acetone, or methylethylketone, *etc*.), nitriles (*e.g*., actetonitrile *etc.*), amides (*e.g.,* DMF *etc*.), water, ethyl acetate, acetic acid, or a mixed solvent of at least two of these *etc.*) in the presence of a catalyst (*e.g*., palladium-carbon, palladium black, palladium hydroxide-carbon, platinum oxide, or Raney nickel, *etc*.) under the hydrogen atomosphere at nomal pressure or under pressurization, or in the presence of ammonium formate at a temperature of 0 to 200°C.
(4) The deprotection reaction of a silyl group is carried out, for example, in a water-miscible organic solvent (*e.g.,* tetrahydrofuran, or acetonitrile, *etc*.) using tetrabutylammonium fluoride at a temperature of 0 to 40°C.
(5) The deprotection reaction using metals is carried out, for example, in an acidic solvent (*e.g.*, acetic acid, pH4.2-7.2 buffer solution, or a mixture of a solution thereof and an organic solvent of tetrahydrofran *etc*.) in the presence of zinc powder, if necessary sonicating, at the temperature of 0 to 40°C.
(6) The deprotection reaction using metal complexes is carried out, for example, in an organic solvent (*e.g.*, dichloromethane, DMF, THF, ethyl acetate, acetonitrile, dioxane, ethanol *etc*.), water, or a mixture thereof, in the presence of a trap reagent (*e.g.*, tributyltine hydride, triethylsilane, dimedone, morpholine, diethylamine, pyrrolidine, *etc*.), an organic acid (*e.g.*, acetic acid, formic acid, 2-ethyl hexanoic acid, *etc*.) and/or salts of organic acid (*e.g.,* sodium 2-ethylhexanoate, potassium 2-ethylhexanoate *etc*.), in the presence or absence of a phosphine reagent (*e.g.,* triphenylphosphine *etc*.), using metal complexes (*e.g.,* tetrakistriphenylphosphinepalladium(0), dichiorobis(triphenyiphosphine)palladium(II), palladium acetate(II), tris(triphenylphosphine)rhodium(I) chloride *etc*.) at the temperature of 0 to 40°C.

In addition, the deprotection reaction except the above-mentioned processes can be carried out, for example, by the process described in T.W. Greene, *Protective Groups in Organic Synthesis,* Wiley, New York, 1999. As is easily understood by those skilled in the art, the intended compounds of the invention may be readily prepared through selective use of these deprotecting reactions.

The protection group for carboxyl includes, for example, methyl, ethyl, allyl, t-butyl, trichloroethyl, benzyl (Bn), phenacyl, and so on.

The protection group for hydroxyl includes, for example, methyl, trytyl, methoxymethyl (MOM), 1-ethoxyethyl (EE), methoxyethoxymethyl (MEM), 2-tetrahydropyranyl (THP), trimethylsyryl (TMS), triethylsyryl (TES), t-butyldimethylsyryl (TBDMS), t-butyldiphenylsyryl (TBDPS), acetyl (Ac), pivaloyl, benzoyl, benzyl (Bn), p-methoxybenzyl, allyloxycarbonyl (Alloc), 2,2,2-trichloroethoxycarbonyl (Troc), and so on.

The protection group of amino includes benzyloxycarbonyl, t-butoxycarbonyl, allyloxycarbonyl (Alloc), 1-methyl-1-(4-biphenyl) ethoxycarbonyl (Bpoc), trifluoroacetyl, 9-fluorenylmethoxycarbonyl, benzyl (Bn), p-methoxybenzyl, benzyloxymethyl (BOM), 2-(trimethylsyryl) ethoxymethyl (SEM) and so on.

The protection group of mercapto includes, for example, benzyl (Bn), methoxybenzyl, methoxymethyl (MOM), 2-tetrahydropyranyl (THP), diphenylmethyl, acetyl (Ac) and so on.

The protective group for carboxyl, hydroxyl, amino or mercapto is not particularly limited to the above mentioned groups, so long as it can be easily and selectively left. For example, those described in T.W Greene, *Protective Groups in Organic Synthesis,* Wiley, New York, 1999 can be used.

### [2] The compound represented by formula (I) can be prepared by reacting a compound represented by formula (IV)

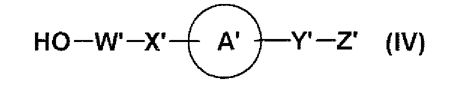

(wherein all symbols have the same meanings as those defined above) with the above-mentioned compound represented by formula (II), if necessary, followed by subjecting to a deprotection reaction of the protective group.

The above-mentioned reaction is known. It is carried out, for example, by reacting with a corresponding alcohol compound in an organic solvent (*e*.*g*., methylene chloride, diethylether, tetrahydrofuran, acetonitrile, benzene, toluene, *etc*.) in the presence of an azo-compound (*e*.*g*., diethyl azodicarboxylate, diisopropyl azodicarboxylate, 1,1'-(azodicarbonyl)dipiperidine, 1,1'-azobis(N,N-dimethylformamide), *etc*.) and a phosphine-compound (*e*.*g*., triphenylphosphine, tributylphosphine, trimethylphosphine, *etc*.) at a temperature of 0 to 60°C.

The deprotection reaction of the protective group may be carried out by the above-mentioned similar method.

### [3] The compound represented by formula (I) can be prepared by reacting a compound represented by formula (V)

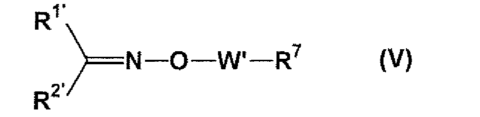

(wherein all the symbols have the same meanings as that of the above-mentioned) with a compound represented by formula (VI) 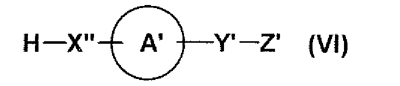 (wherein X" is -O-, -S- or -N(R^{3'})- (wherein R^{3'} has the same meanings as that of R³, but carboxyl, hydroxyl, amino and mercapto included these group are, if necessary, protected. The other symbols have the same meanings as that of the above-mentioned)), if necessary, followed by subjecting to a deprotection reaction of the protective group.

The above-mentioned reductive reaction is known. It is carried out in the same way as the above-mentioned [1].

The compounds represented by formula (II), (III), (IV), (V) and (VI) used in the present invention are known in themselves, or can be easily prepared by known method, or according to method described in Examples.

For example, among the compounds represented by formula (II), and formula (III) and formula (IV), the compound wherein X' is -O-, -S- or -N(R^{3'})-, can be prepared by the method indicated by reaction process 1 or reaction process 2.

In addition, the compounds represented by formula (V) can be prepared by the method indicated by reaction process 3.

In reaction process, R⁸ is halogen atom (*e.g.*, fluorine, chlorine, bromine, iodine), R¹⁰ is halogen atom (*e.g.*, fluorine, chlorine, bromine, iodine), and the other symbols have the same meanings as that of the above-mentioned.

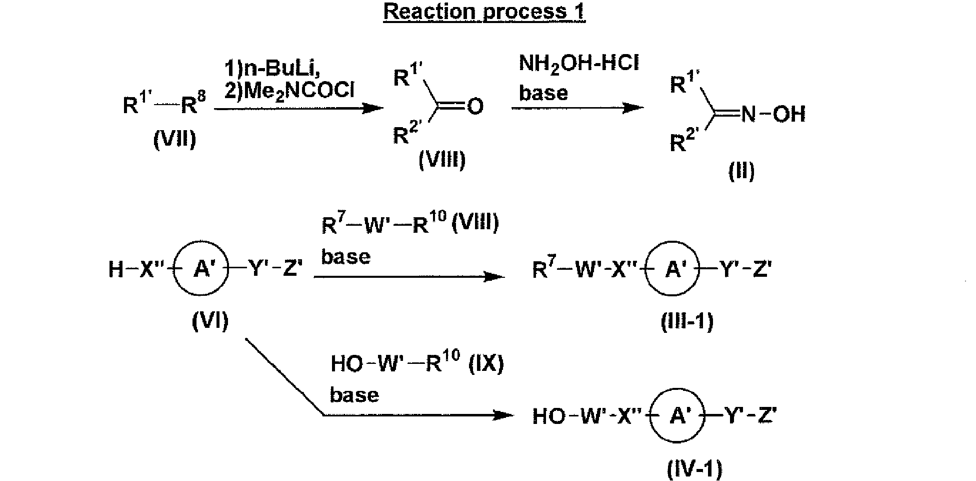

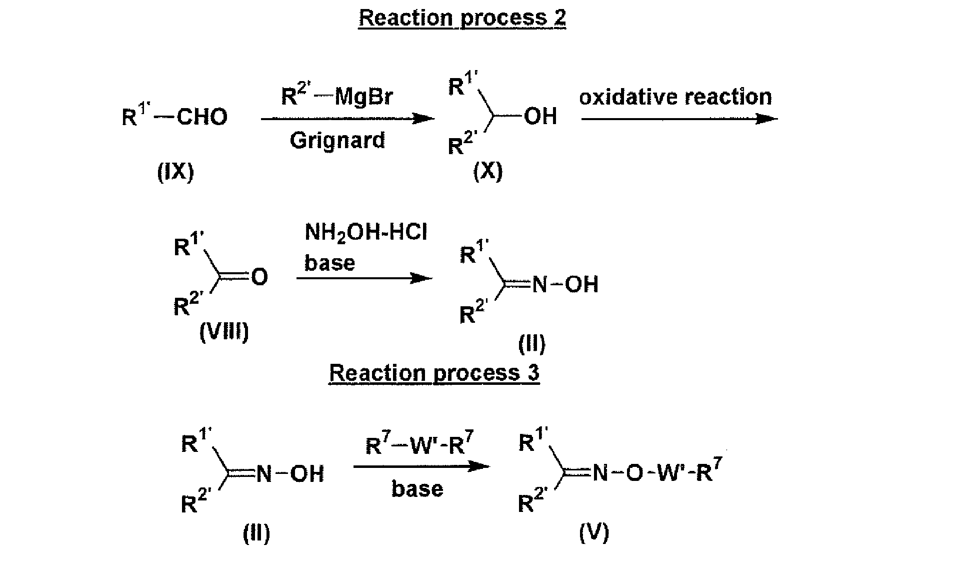

In reaction process, the compounds as starting materials are known in themselves, or can be easily prepared by known method.

In each reaction in the present specification, reaction products may be purified in an ordinary manner, for example, through normal-pressure or reduced-pressure distillation, or through high-performance liquid chromatography with silica gel or magnesium silicate, thin-layer chromatography, or column chromatography, or through washing or recrystallization and so on. The purification may be effected in each reaction stage or after some reaction stages.

### Pharmacological Activity:

As pharmacological test other than ones described in Example, particularly *in vivo* measurement using animals, for example, there are methods as follows. The hypoglycemic effect and hypolipidemic effect of the compound of the invention can be measured by methods as follows.

### Hypoglycemic and hypolipidemic effects:

Male, 8-weeks old KKAy/Ta Jcl mice (five mice per group) are pre-breaded individually in single cages for approximately one week and provided pellet diet and tap water from bottle of feed water *ad libitum.* Mice are acclimatized to switch over to milled diet for three days. On the first day of the experiment (Day 0), the body weight of mice are measured. Blood samples are collected from coccygeal vein using a microcapillary to measure plasma glucose concentration. Based on plasma glucose concentration, mice are divided into some groups (five mice per group) using a stratified randomization method. The body weight of mice are measured on the morning of the next day, and from the next day for six days they are given compounds by food mixture containing 0.03 % (w/w), 0.01% (w/w) or 0.003% (w/w) of the compound of the present invention or by milled diet only. On the morning of the fourth and the seventh day, body weights and food intakes of them are determined to calculate the mean administered dose. On the morning of the sixth day, blood samples were collected from coccygeal vein to measure glucose and triglyceride (TG) levels. On the seventh day after measuring body weight, blood samples are collected from abdominal vena cava under anesthetized condition by ether to determine plasma insulin, non-esterified fatty acid (NEFA), GOT and GPT levels using commercially available kits. And, the liver is removed and wet weight of the liver is measured. The total RNAs are prepared from left lobe of the liver and measured a gene expression level of bifunctional enzyme by Northern blot method. Actually, there is no significant difference in the food intake between control group (milled diet only) and compounds-treated group (milled diet containing 0.03 %, 0.01% or 0.003% of compounds). The calculated dose is approximately 40 mg/kg/day in the group given diet containing 0.03% of the compound.

It is suggested the possibility as an agent for preventing and/or treating of diabetes mellitus, hyperlipidemia, atherosclerosis *etc.,* from ameliorating effects of plasma glucose, plasma insulin, NEFA or TG levels in well-fed KKAy/Ta mice. This effect is likely to be mediated through PPAR γ activation *in vivo.* Additionally, it is likely that an increase in liver weight and in an expression amount of liver bifunctional enzyme reflects on PPAR α activation *in vivo.*

### Hypoglycemic and hypolipidemic effects:

Male, 8-weeks old Zucker fa/fa rats (Strain: Crj-[ZUC]-fa/fa} and healthy Zucker lean rats (Strain: Crj-[ZUC]-lean) to be purchased are pre-breaded individually in single cages for approximately two weeks and provided pellet diet and tap water from automatic water supplying equipment *ad libitum.* For five days before the treatment, rats are acclimatized to oral gavage administration. During this period, a general condition of them is observed, and healthy rats with 10-weeks of age are used for experiment. The body weight of each rats are measured on the morning of the first day of experiment (Day 0) and blood samples are collected from coccygeal vein using a microcapillary to measure plasma glucose, TG, NEFA concentrations and HbA1c. Based on the HbA1c and body weight, rats are assigned to groups comprised of five animals each using a stratified randomization method. Additionally, rats are interchanged optionally to prevent the deflection of other parameters' averages between groups. The body weight of each animal was measured every morning from the day after grouping. Volumes to be administered are calculated on the basis of body weight measured on the day of administration, and oral gavage administration of compound of the present invention or vehicle only (0.5% methylcellulose) is conducted once a day for 13 days. The healthy animals (lean rats) are given vehicle only.

Food consumption is measured on the morning of Day 1, 4, 7, 10 and 13 to calculate mean food intakes. On the seventh day, blood samples are corrected from coccygeal vein using microcapillary to measure plasma glucose, TG, NEFA concentrations and HbA1c. And on the 14th day, oral glucose tolerance test (OGTT) is performed to evaluate improving effect on glucose intolerance. Rats are fasted on the previous day (Day 13) to perform OGTT. After blood samples are collected on the next day (Day 14), 40% glucose solution is loaded at a volume of 2 g/5 ml/kg per oral administration. 60 and 120 minutes after loading, blood samples are collected from coccygeal vein using microcapillary to determine plasma glucose levels.

Animals are given food after the OGTT and administered compound of the present invention on Day 15. On the morning of the 16th day after measuring body weight, blood samples are collected from abdominal vena cava under anesthetized condition by ether to determine plasma glucose, plasma insulin, TG, NEFA, GOT and GPT levels. And, the liver is removed and weighed.

It is suggested the possibility as an agent for preventing and/or treating of diabetes mellitus, hyperlipidemia, atherosclerosis *etc.,* from ameliorating effects of plasma glucose, plasma insulin, TG, NEFA levels or HbAlc in well-fed Zucker fa/fa rats. Also, a decrease effect of fasting plasma glucose and improving effect of glucose intolerance during OGTT suggest the possibility as an agent for preventing and/or treating of diabetes mellitus. These effects are likely to be mediated through PPAR γ activation *in* vivo. Additionally, it is suggested that an increase in liver weight depends on PPAR α activation *in vivo.*

### Toxicity:

Toxicity of the compound represented by formula (I) is very low, and it is safe enough to use as a pharmaceutical agent.

### INDUSTRIAL AVAILABILITY

### Application to pharmaceutical preparations:

Since the compound represented by formula (I) of the present invention and nontoxic salt thereof have a PPAR modulating activity, it is expected to be applied as hypoglycemic agents, hypolipidemic agents, agents for preventing and/or treating of diseases associated with metabolic disorders such as diabetes, obesity, metabolic syndrome, hypercholesterolemia and hyperlipoproteinemia *etc*., hyperlipidemia, atherosclerosis, hypertension, circulatory diseases, overeating, ischemic heart diseases *etc*., HDL cholesterol-elevating agents, LDL cholesterol and/or VLDL cholesterol-lowering agents and agents for relieving risk factors of diabetes or metabolic syndrome.

Also, since the compound represented by formula (I) of the present invention, and the salts thereof, have particularly a PPAR δ agonist effect, it is expected to be useful as HDL cholesterol-elevating agent, inhibitory agent of progress of and therapeutic agent for atherosclerosis, hypolipidemic agent, hypoglycemic agent, therapeutic agent for hyperlipidemia, or therapeutic agent for diabetes. In addition, it is expected to be useful for relieving risk factors of metabolic syndrome or preventing onset of ischemic heart diseases.

The compound represented by formula (I) or the salts thereof may be administered in combination with other drugs for the purpose of
1) complement and/or enhancement of preventing and/or treating effect,
2) improvement of dynamics and absorption of the compound, and lowering of dose, and/or
3) alleviation of side effect of the compound.

The compound represented by formula (I) or the salts thereof and other pharmaceutical preparations may be administered in the form of formulation having these components incorporated in one preparation or may be administered in separate preparations. In the case where these pharmaceutical preparations are administered in separate preparations, they may be administered simultaneously or at different times. In the latter case, the compound represented by formula (I) or the salts thereof may be administered before the other pharmaceutical preparations. Alternatively, the other pharmaceutical preparations may be administered before the compound represented by formula (I) or the salts thereof The method for the administration of these pharmaceutical preparations may be the same or different.

The diseases on which the preventive and/or treatment effect of the above-mentioned combined preparations works are not specifically limited but may be those for which the preventive and/or treatment effect of the compound represented by formula (I) is compensated for and/or enhanced.

The compound represented by formula (I) or the salts thereof and other pharmaceutical preparations may be administered in the form of formulation having these components incorporated in one preparation or may be administered in separate preparations. In the case where these pharmaceutical preparations are administered in separate preparations, they may be administered simultaneously or at different times. In the latter case, the compound represented by formula (I) or the salts thereof may be administered before the other pharmaceutical preparations. Alternatively, the other pharmaceutical preparations may be administered before the compound represented by formula (I) or the salts thereof. The method for the administration of these pharmaceutical preparations may be the same or different.

The diseases on which the preventive and/or treatment effect of the above-mentioned combined preparations works are not specifically limited but may be those for which the preventive and/or treatment effect of the compound represented by formula (I) is compensated for and/or enhanced.

As other drugs to compensate and/or enhance for hypolipidemic effect of the compound represented by formula (I) or the salts thereof, i.e. lipid improvement agents, they include, for example, MTP (Microsomal Triglyceride Transfer Protein) inhibitor, HMG-CoA reductase inhibitor, squalene synthase inhibitor, fibrate (fibric acid derivative), ACAT (acyl CoA: Cholesterol O-acyltransferase) inhibitor, 5-lipoxygenase inhibitor, cholesterol absorption inhibitor, bile acid absorption inhibitor, ileal Na⁺/bile acid transporter (IBAT) inhibitor, LDL receptor activator/expression enhancer, lipase inhibitor, probucol formulation, nicotine acid formulation, other anti-hypercholesterolemia therapeutic agent and so on.

Examples of MTP inhibitor include BMS-201038, BMS-212122, BMS-200150, GW-328713, R-103757, and so on. Examples of HMG-CoA reductase inhibitor include atorvastatin, fulvastatin, lovastatin, pitavastatin, pravastatin, rosuvastatin, simvastatin, and so on. Examples of ACAT inhibitor include F-12511, F-1394, CI-1011, melinamide and so on. Examples of squalene synthase inhibitor include TAK-475 and so on. Examples of fibrate include gemfibrozil, clofibrate, bezafibrate, fenofibrate, and so on. Examples of ACAT inhibitor include C1-1101, FCE27677, RP73163, and so on. Examples of cholesterol absorption inhibitor include ezetimibe and so on. Examples of bile acid absorption inhibitor include cholestyramine, colesevelam, and so on. Examples of LDL receptor activator/expression enhancer include MD-700, LY295427, and so on. Examples of lipase inhibitor include orlistat and so on. It is known that there are sometimes associated with rhabdomyolysis in case of a combination of fibrate and HMG-CoA reductase inhibitor. In the above-mentioned combination, there is possibility to correct abnormal lipid metabolism without developing rhabdomyolysis.

As combination drugs, HMG-CoA reductase inhibitor, cholesterol absorption inhibitor, bile acid absorption inhibitor, pancreatic lipase inhibitor is preferred.

As other drugs to compensate and/or enhance for hypoglycemic effect of the compound represented by formula (I), and to enhance effect of the treatment of complication of diabetes, i.e. therapeutic agents for diabetes, they include, for example, sulfonylurea type hypoglycemic agent, biguanide preparation, alfa-glucosidase inhibitor, fast-acting insulin secretion accelerator, insulin preparation, dipeptidyl peptidase 4 inhibitor, beta-3 adrenaline receptor activator, PPAR agonist, other therapeutic agents for diabetes, therapeutic agents for complication of diabetes and so on.

Examples of sulfonylurea type hypoglycemic agents include acetohexamide, glibenclamide, gliclazide, glyclopyramide, chlorpropamide, tolazamide, tolbutamide and glimepiride, and so on. Examples of biguanide preparations include buformin hydrochloride and metformin hydrochloride, and so on. Examples of alfa-glucosidase inhibitors include acarbose and voglibose, and so on. Examples of fast-acting insulin secretion accelerators include nateglinide and repaglinide, and so on. Examples of dipeptidyl peptidase 4 inhibitor include NVP-DPP728A and so on. Examples of beta-3 adrenaline receptor activators include AJ-9677, BMS-210285, CP-331679, KUL-1248, LY 362884, L-750335 and CP331648, and so on. Examples of PPAR agonist include tesaglitazar (AZ-242), muraglitazar (BMS-298585), TAK-559, LY-510929, ONO-5129, netoglitazone (isaglitazone), GW-501516, LY-465608, GW-590735, RO-205-2349, GW-409544, pioglitazone hydrochloride, rosiglitazone maleate and so on.

Examples of therapeutic agents for complication of diabetes include aldose reductase inhibitor (epalrestat, fidarestat, zenarestat *etc*.) and so on.

As other drugs to compensate and/or enhance for anti-adiposity effect of the compound represented by formula (1), i.e. anti-adiposity agents, they include, for example, appetite suppressing agent, pancreatic lipase inhibitor, beta-3 adrenaline receptor activator, serotonin norepinephrine dopamine reuptake inhibitor and so on. Examples of appetite suppressing agent include leptin, mazindol, amphetamine, methamphetamine, and so on. Examples of pancreatic lipase inhibitor include orlistat and so on. Examples of beta-3 adrenaline receptor activator include AJ-9677, BMS-210285, CP-331679, KUL-1248, LY-362884, L-750335, CP-331648, and so on. Examples of serotonin norepinephrine dopamine reuptake inhibitor include sibutramine and so on.

The weight proportion of the compound represented by formula (I) or a salt thereof and the other drugs is not specifically limited. Arbitrary two or more of the other drugs may be administered in combination. Examples of the other pharmaceutical preparations for compensating for and/or enhancing the preventive and/or treatment effect of the compound represented by formula (I) or a salt thereof include not only those which have so far been found but also those which will be found on the basis of the above-mentioned mechanism.

In order to use the compound of the invention represented by formula (I) or a salt thereof, or the compound represented by formula (I) or a salt thereof in combination with the other pharmaceutical preparations, these compounds are normally administered to the entire of human body or topically orally or parenterally. The dose of these compounds depends on the age, weight and symptom of the patient, the remedial value, the administration method, the treatment time, *etc*. In practice, however, these compounds are administered orally once or several times per day each in an amount of from 1 mg to 1000 mg per adult, parenterally once or several times per day each in an amount of from I mg to 100 mg per adult or continuously administered into vein for 1 hour to 24 hours per day. It goes without saying that the dose of these compounds may be less than the above-mentioned value or may need to exceed the above-mentioned range because the dose varies under various conditions as mentioned above.

When the compounds of the invention represented by formula (I) or a salt thereof, or the compound represented by formula (I) or a salt thereof is administered in combination with the other pharmaceutical preparations, they are used in the form of solid or liquid agent for oral administration, injection, agent for external application, suppository, eye drops or inhalant for parenteral administration or the like.

Examples of the solid agent for oral administration include tablet, pill, capsule, powder, and pellet. Examples of the capsule include hard capsule, and soft capsule.

In such a solid agent for internal application, one or more active materials are used in the form of preparation produced by an ordinary method singly or in admixture with a vehicle (*e.g.*, lactose, mannitol, glucose, microcrystalline cellulose, starch *etc*.), binder (*e*.*g*., hydroxypropyl cellulose, polyvinyl pyrrolidone, magnesium metasilicoaluminate *etc*.), disintegrant (*e*.*g*., calcium fibrinoglycolate *etc*.), glidant (*e*.*g*., magnesium stearate *etc*.), stabilizer, dissolution aid (*e*.*g*., glutamic acid, aspartic acid *etc.*) or the like. The solid agent may be coated with a coating agent (*e*.*g*., white sugar, gelatin, hydroxypropyl cellulose, hydroxypropyl methyl cellulose phthalate *etc*.) or two or more layers. Alternatively, the solid agent may be capsulized by an absorbable material such as gelatin.

Examples of the liquid agent for oral administration include pharmaceutically acceptable aqueous solution, suspension, emulsion, syrup, and elixir. In such a liquid agent, one or more active agents are dissolved, suspended or emulsified in a commonly used diluent (*e.g.,* purified water, ethanol, mixture thereof *etc*.). Furthermore, such a liquid agent may comprise a wetting agent, a suspending agent, an emulsifier, a sweetening agent, a flavor, a preservative, a buffer, *etc.*

The agent for parenteral administration may be in the form of, *e.g.,* ointment, gel, cream, wet compress, paste, liniment, nebula, inhalant, spray, aerosol, eye drops, collunarium or the like. These agents each contain one or more active materials and are prepared by any known method or commonly used formulation.

The ointment is prepared by any known or commonly used formulation. For example, one or more active materials are triturated or dissolved in a base to prepare such an ointment. The ointment base is selected from known or commonly used materials. In some detail, higher aliphatic acid or higher aliphatic acid ester (*e.g.*, adipic acid, myristic acid, palmitic acid, stearic acid, oleic acid, adipic acid ester, myristic acid ester, palmitic acid ester, stearic acid ester, oleic acid ester *etc*.), wax (*e*.*g*., beeswax, whale wax, ceresin *etc*.), surface active agent (*e.g.*, polyoxyethylenealkylether phosphoric acid ester *etc*.), higher alcohol (*e.g.*, cetanol, stearyl alcohol, setostearyl alcohol *etc*.), silicon oil (*e*.*g*., dimethyl polysiloxane *etc*.), hydrocarbon (*e.g.*, hydrophilic petrolatum, white petrolatum, purified lanolin, liquid paraffin *etc*.), glycol (*e.g.*, ethylene glycol, diethylene glycol, propylene glycol, polyethylene glycol, macrogol *etc*.), vegetable oil (*e.g.,* castor oil, olive oil, sesame oil, turpentine oil), animal oil (mink oil, vitelline oil, squalane, squalene), water, absorption accelerator and rash preventive may be used singly or in admixture of two or more thereof. The base may further comprise a humectant, a preservative, a stabilizer, an antioxidant, a perfume, *etc*.

The gel is prepared by any known or commonly used formulation. For example, one or more active materials are dissolved in a base to prepare such a gel. The gel base is selected from known or commonly used materials. For example, lower alcohol (*e.g*., ethanol, isopropyl alcohol *etc*.), gelling agent (*e.g*., carboxymethyl cellulose, hydroxyethyl cellulose, hydroxypropyl cellulose, ethyl cellulose *etc*.), neutralizing agent (*e.g*., triethanolamine, diisopropanolamine *etc*.), surface active agent (*e.g.,* polyethylene glycol monostearate *etc*.), gums, water, absorption accelerator, and rash preventive are used singly or in admixture of two or more thereof. The gel base may further comprise a preservative, an antioxidant, a perfume, *etc*.

The cream is prepared by any known or commonly used formulation. For example, one or more active materials are dissolved in a base to prepare such a cream. The cream base is selected from known or commonly used materials. For example, higher aliphatic acid ester, lower alcohol, hydrocarbon, polyvalent alcohol (*e.g.,* propylene glycol, 1,3-butylene glycol *etc*.), higher alcohol (*e.g.,* 2-hexyl decanol, cetanol *etc*.), emulsifier (*e.g.*, polyoxyethylene alkyl ethers, aliphatic acid esters *etc.*), water, absorption accelerator, and rash preventive are used singly or in admixture of two or more thereof. The cream base may further comprise a preservative, an antioxidant, a perfume, *etc*.

The wet compress is prepared by any known or commonly used formulation. For example, one or more active materials are dissolved in a base to prepare a kneaded mixture which is then spread over a support to prepare such a wet compress. The wet compress base is selected from known or commonly used materials. For example, thickening agent (*e.g*., polyacrylic acid, polyvinyl pyrrolidone, gum arabic, starch, gelatin, methyl cellulose *etc*.), wetting agent (*e.g*., urea, glycerin, propylene glycol *etc*.), filler (*e.g.,* kaolin, zinc oxide, talc, calcium, magnesium *etc*.), water, dissolution aid, tackifier, and rash preventive may be used singly or in admixture of two or more thereof The wet compress base may further comprise a preservative, an antioxidant, a perfume, *etc*.

The pasting agent is prepared by any known or commonly used formulation. For example, one or more active materials are dissolved in a base to prepare a kneaded mixture which is then spread over a support to prepare such a pasting agent. The pasting agent base is selected from known or commonly used materials. For example, polymer base, fat and oil, higher aliphatic acid, tackifier and rash preventive may be used singly or in admixture of two or more thereof The pasting agent base may further comprise a preservative, an antioxidant, a perfume, *etc*.

The liniment is prepared by any known or commonly used formulation. For example, one or more active materials are dissolved, suspended or emulsified in water, alcohol (*e.g.*, ethanol, polyethylene glycol *etc*.), higher aliphatic acid, glycerin, soap, emulsifier, suspending agent, *etc*., singly or in combination of two or more thereof, to prepare such a liniment. The liniment may further comprise a preservative, an antioxidant, a perfume, *etc.*

The nebula, inhalant, spray and aerozol each may comprise a commonly used diluent, additionally, a stabilizer such as sodium hydrogen sulfite and a buffer capable of providing isotonicity such as isotonic agent (*e.g.*, sodium chloride, sodium citrate, or citric acid *etc*.). For the process for the preparation of spray, reference can be made to US Patents 2,868,691 and 3,095,355.

The injection for parenteral administration consists of solid injection used to be dissolved or suspended in the form of solution, suspension, emulsion and a solvent to be dissolved before use. The injection is prepared by dissolving, suspending or emulsifying one or more active materials in a solvent. As such a solvent there may be used distilled water for injection, physiological saline, vegetable oil, alcohol such as propylene glycol, polyethylene glycol and ethanol, *etc.,* singly or in combination thereof. The injection may further comprise a stabilizer, a dissolution aid (*e.g.*, glutamic acid, aspartic acid, Polysolvate 80 (trade name) *etc*.), a suspending agent, an emulsifier, a soothing agent, a buffer, a preservative, *etc*. The injection is sterilized at the final step or prepared by an aseptic process. Alternatively, an aseptic solid agent such as freeze-dried product which has previously been prepared may be rendered aseptic or dissolved in an aseptic distilled water for injection or other solvents before use.

The inhalant for parenteral administration may be in the form of aerosol, powder for inhalation or liquid for inhalation. The liquid for inhalation may be dissolved or suspended in water or other proper medium in use.

These inhalants are prepared by a known method.

For example, the liquid for inhalation is prepared from materials properly selected from preservatives (*e.g*., benzalconium chloride, Paraben *etc*.), colorants, buffering agents (*e.g.*, sodium phosphate, sodium acetate *etc*.), isotonic agents (*e.g.*, sodium chloride, concentrated glycerin *etc*.), thickening agents (*e.g*., carboxyvinyl polymer *etc*.), absorption accelerators, *etc*. as necessary.

The powder for inhalation is prepared from materials properly selected from glidants (*e.g.*, stearic acid and salt thereof *etc*.), binders (*e.g*., starch, dextrin *etc*.), vehicles (*e.g*., lactose, cellulose *etc*.), colorants, preservatives (*e.g*., benzalconium chloride, Paraben *etc*.), absorption accelerators, *etc*., if necessary.

In order to administer the liquid for inhalation, a sprayer (*e.g*., atomizer, nebulizer *etc*.) is normally used. In order to administer the powder for inhalation, a powder inhaler is normally used.

Other examples of the composition for parenteral administration include suppository for rectal administration and pessary for vaginal administration prepared by an ordinary formulation comprising one or more active materials.

### BEST MODE FOR CARRYING OUT THE INVENTION

The present invention is explained below in detail based on Examples and Comparative Examples, however, the present invention is not limited thereto.

The solvents in parentheses at chromatographic separations section and TLC section show the developing or eluting solvents and the ratios of the solvents used are indicated by volume. The solvents in parentheses indicated in NMR section show solvents used in determination.

All compounds described in the specification are named by using of ACD/Name (Trade mark, Advanced Chemistry Development Inc.) or ACD/Name (Trade mark, Advanced Chemistry Development Inc.) batch which is the computer program to name according to IUPAC rule, or according to IUPAC organic chemistry nomenclature

### Reference Example 1:

### bis(4-methylphenyl)methaneone oxime

A solution of bis(4-methylphenyl)methaneone (4.2g) in pyridine (40mL) was added by hydroxylamine- hydrochloride (2.1g) and stirred for an hour at 100°C. The mixture was poured into water and extracted by ethyl acetate. The extract was washed with 2N hydrochloric acid, water and saturated brine successively, dried over anhydrous magnesium sulfate and concentrated to give the title compound (4.5g) having the following physical data.
TLC: Rf 0.71 (hexane : ethyl acetate = 2 : 1).

### Reference Example 2:

### methyl (3-hydroxyphenyl)acetate

Methanol (200mL) was cooled down to -30°C, dropped by thionyl chloride (14mL), stirred for 15 minutes within -30 to -20°C, added by a solution of 3-hydroxyphenylacetic acid (25.8g) in methanol (80mL) and stirred for an hour at a room temperature. The mixture was concentrated, added by toluene and concentrated to give the title compound (30.2g) having the following physical data.
TLC: Rf 0.40 (hexane : ethyl acetate = 2 : 1).

### Reference Example 3:

### methyl [3-(2-bromoethoxy)phenyl]acetate

A solution of the compound prepared in Reference Example 2 (5.0g) in 2-butanone (100mL) was added by 1,2-dibromoethane (3.1mL) and potassium carbonate (6.2g) and refluxed for 16 hours. The mixture was moreover added by 1,2-dibromoethane (12.0mL) and refluxed overnight. The mixture was cooled down till room temperature and then solid was separated by filtration. The filtrate was concentrated and the residue was purified by column chromatography on silica gel (hexane : ethyl acetate = from 4 : 1 to 2 : 1) to give the title compound (2.1g) having the following physical data.
TLC: Rf 0.73 (hexane : ethyl acetate = 2 : 1).

### Example 1:

### methyl {3-[2-({[bis(4-methylphenyl)methylene]amino}oxy)ethoxy]phenyl}acetate

A solution of the compound prepared in Reference Example 1 (0.7g) in N,N-dimethylformamide (5mL; DMF) was added by a suspended solution of potassium t-butoxide (0.3g) in DMF (10mL), stirred for 30 minutes at a room temperature, added by a solution of the compound prepared in Reference Example 3 (0.6g) in DMF (2.5mL) and stirred for an hour. The mixture was poured into diluted hydrochloric acid and extracted by ethyl acetate. The extract was washed with saturated brine, dried over anhydrous magnesium sulfate and concentrated to give the title compound having the following physical data.
TLC: Rf 0.69 (hexane : ethyl acetate = 2 : 1).

### Example 2:

### {3-[2-({[bis(4-methylphenyl)methylene]amino}oxy)ethoxy]phenyl}acetic acid

A mixed solution of the compound prepared in Example 1 in tetrahydrofuran (5mL; THF) and methanol (5mL) was added by 2N sodium hydroxide (5mL) and stirred for an hour at a room temperature. The mixture was added by 2N hydrochloric acid (5mL), controlled in acidic condition and then extracted by ethyl acetate. The extract was washed with saturated brine, dried over anhydrous magnesium sulfate and then concentrated. The residue was purified by column chromatography on silica gel (from hexane : ethyl acetate = 2: 1 to ethyl acetate only). The obtained solid was recrystrallized by a mixed solution of hexane and ethyl acetate to give the title compound (0.4g) having the following physical data.
melting point (degree centigrade): 132.0 - 133.0;
TLC: Rf 0.33 (chloroform : methanol = 10 : 1);
NMR(CDCl₃): δ 7.35 (d, 2H), 7.26-7.17 (m, 5H), 7.12 (d, 2H), 6.89-6.82 (m, 3H), 4.48 (t, 2H), 4.26 (t, 2H), 3.60 (s, 2H), 2.38 (s, 3H), 2.35 (s, 3H)

### Example 3(1)-3(21)

By the same procedure as described in Example 1 and 2 using the corresponding derivative instead of the compound prepared in Reference Example 1, additionally if necessary, by converting into a corresponding salt by known method, the following compounds of the present invention were obtained. Also, NMR data of the following compounds of the present invention was described as the characteristic peak.

### Example 3(1):

[3-(2-{[(diphenylmethylene)amino]oxy}ethoxy)phenyl]acetic acid
TLC:Rf 0.52 (chloroform : methanol = 9 : 1);
NMR(CDCl₃): δ 4.50 (t, 2H), 3.59 (s, 2H).

### Example 3(2):

{3-[2-({[(1E)-phenyl(pyridin-3-yl)methylene]amino}oxy)ethoxy]phenyl}acetic acid
TLC:Rf 0.43 (chloroform : methanol =9 : 1);
NMR(CDCl₃): δ 4.52 (t, 2H), 3.61 (s, 2H).

### Example 3(3): {3-[2-({[bis(4-fulorophenyl)methylene]amino}oxy)ethoxy]phenyl}acetic acid

TLC:Rf 0.33 (chloroform : methanol = 10 : 1);
NMR(CDCl₃): δ 4.49 (t, 2H), 3.59 (s, 2H).

### Example 3(4):

(3-{2-[({bis[3-(trifuloromethyl)phenyl]amino)oxy]ethoxy}phenyl)acetic acid melting point (degree centigrade): 101.0 - 102.5;
TLC:Rf 0.51 (chloroform : methanol = 10 : 1);
NMR(CDCl₃): δ 4.54 (t, 2H), 3.60 (s, 2H).

### Example 3(5):

sodium {³-[3-({[bis(4-methylphenyl)methylene]amino}oxy)propoxy]phenyl}acetate
TLC:Rf 0.55 (chloroform ; methanol = 8 : 1);
NMR(DMSO-d₆): δ 3.17 (s, 2H), 2.04 (tt, 2H)

### Example 3(6):

sodium {3-[4-({[bis(4-methylphenyl)methylene]amino}oxy)butoxy]phenyl}acetate
TLC:Rf 0.60 (chloroform: methanol = 8 : 1);
NMR(DMSO-d₆): δ 3.13 (s, 2H), 1.86-1.60 (m, 4H).

### Example 3(7):

(3-{2-[({bis[4-(trifuloromethyl)phenyl]methylene}amino)oxy]ethoxy}phenyl)acetic acid
TLC:Rf 0.50 (hexane : ethyl acetate = 1 : 1);
NMR(CDCl₃): δ 4.54 (t, 2H), 3.61 (s, 2H).

### Example 3(8):

(3-{2-[({1-[4-(trifuloromethyl)phenyl]propylidene}amino)oxy]ethoxy}phenyl)acetic acid
TLC:Rf 0.50 (ethyl acetate);
NMR(CDCl₃): δ 3.61 (s, 2H), 1.26, 1.12 (t, 3H).

### Example 3(9):

(3-{2-[({phenyl[4-(trifuloromethyl)phenyl]methylene}amino)oxy]ethoxy}phenyl)acetic acid
TLC:Rf 0.53 (ethyl acetate);
NNM(CDCl₃): δ 3.60 (s, 2H), 4.51, 4.53 (t, 2H).

### Example 3(10):

(3-{2-[({[3-(trifuloromethyl)phenyl][4-(trifuloromethyl)phenyl]methylene}amino)oxy]ethoxy}phenyl)acetic acid
TLC:Rf 0.34 (ethyl acetate);
NMR(CDCl₃): δ 4.52-4.56 (m, 2H), 4.24-4.27 (m, 2H), 3.60 (s, 2H).

### Example 3(11):

{3-[2-({[1-(4-fulorobenzyl)-2-(4-fulorophenyl)ethylidene] amino}oxy)ethoxy]phenyl} acetic acid
TLC:Rf 0.55 (ethyl acetate);
NMR(CDCl₃): δ 3.62 (s, 2H), 3.52 (s, 2H), 3.35 (s, 2H).

### Example 3(12):

{3-[2-({[bis(4-chlorophenyl)methylene]amino}oxy)ethoxy]phenyl}acetic acid
TLC:Rf 0.64 (chloroform : methanol = 9 : 1);
NMR(CDCl₃); δ 4.50 (t, 2H), 3.61 (s, 2H).

### Example 3(13):

{3-[2-({[bis(4-methoxyphenyl)methylene]amino}oxy)ethoxy]phenyl}acetic acid
TLC:Rf 0.39 (chloroform : methanol = 9 : 1);
NMR(CDCl₃): δ 3.82 (s, 3H), 3.81 (s, 3H), 3.59 (s, 2H).

### Example 3(14):

(3-{2-[({bis[4-(dimethylamino)phenyl]methylene}amino)oxy]ethoxy}phenyl)acetic acid
TLC:Rf 0.47 (chloroform : methanol = 9 : 1);
NMR(CDCl₃): δ 3.59 (s, 2H), 2.97 (s, 6H), 2.96 (s, 6H).

### Example 3(15):

(3-{2-[({bis[3-(methylthio)phenyl]methylene}amino)oxy]ethoxy}phenyl)acetic acid
TLC:Rf 0.66 (ethyl acetate);
NMR(CDCl₃): δ 3.61 (s, 2H), 2.46 (s, 3H), 2.42 (s, 3H).

### Example 3(16):

{3-[2-({[bis(3-fulorophenyl)methylene]amino}oxy)ethoxy]phenyl}acetic acid
TLC:Rf 0.50 (ethyl acetate);
NMR(CDCl₃): δ 4.50-4.53 (m, 2H), 3.61 (s, 2H).

### Example 3 (17):

{3-[2-({[bis(4-phenoxyphenyl)methylene]amino}oxy)ethoxy]phenyl} acetic acid
TLC:Rf 0.59 (chloroform : methanol = 9 : 1);
NMR(CDCl₃): δ 4.50 (t, 2H), 3.57 (s, 2H).

### Example 3(18):

(3-{2-[({bis[4-(methylthio)phenyl]methylene}amino)oxy]ethoxy}phenyl)acetic acid
TLC:Rf 0.52 (chloroform : methanol = 9 : 1);
NMR(CDCl₃): δ 3.60 (s, 2H), 2.50 (s, 3H), 2.48 (s, 3H).

### Example 3(19):

(3-{2-[({bis[3-(dimethylamino)phenyl]methylene}amino)oxy]ethoxy}phenyl)acetic acid
TLC:Rf 0.42 (ethyl acetate);
NMR(CDCl₃): δ 3.60 (s, 2H), 2.91 (s, 6H), 2.89 (s, 6H).

### Example 3(20):

{3-[2-({[(1E)-(4-methylphenyl)methylene]amino}oxy)ethoxy]phenyl}acetic acid
TLC:Rf 0.50 (chloroform: methanol = 9 : 1);
NMR(CDCl₃): δ 3.60 (s, 2H), 2.36 (s, 3H).

### Example 3(21):

{3-[2-({[(1Z)-(4-methylphenyl)methylene]amino}oxy)ethoxy]phenyl}acctic acid
TLC:Rf 0.52 (chloroform : methanol = 9 : 1);
NMR(CDCl₃):δ 8.10 (s, 1H), 3.60 (s, 2H), 2.36 (s, 3H).

### Reference Example 4:

### bis[3-(1,3-dioxolan-2-yl)phenyl]methanone

Under atmosphere of argon, a solution of 2-(3-bromophenyl)-1,3-dioxolane (15.0g) in anhydrous THF (150mL) was dropped by a solution of n-butyllithium in hexane (41.2mL; 1.59M) at -50°C and stirred for an hour. The mixture was dropped by N,N-dimethylcarbamoyl chloride (2.9mL) and stirred for 3 hours with rising till -30°C. The mixture was diluted with saturated ammonium chloride aqueous solution and extracted by ethyl acetate. The extract washed with water and saturated brine successively, dried over and then concentrated to give the title compound (11.3g) having the following physical data.
TLC:Rf 0.25 (hexane : ethyl acetate = 2:1).

### Reference Example 5:

### bis[3-(1,3-dioxolan-2-yl)phenyl]methaneone oxime

Under atmosphere of argon, a solution of sodium methylate (13.0g) in anhydrous dioxane (20mL) was added by hydroxylamine·hydrochloride (2.3g) and stirred for an hour at 100°C. The mixture was cooled down till room temperature, diluted with saturated ammonium chloride aqueous solution and extracted by ethyl acetate. The extract was washed with water and saturated brine successively, dried over anhydrous magnesium sulfate and then concentrated. The residue was purified by column chromatography on silica gel (hexane : ethyl acetate = from 2 : 1 to 1 : 1) to give the title compound (4.8g) having the following physical data.
TLC:Rf 0.18 (hexane : ethyl acetate = 2 : 1).

### Example 4:

### methyl (3-{2-[({bis[3-(1,3-dioxolan-2-yl)phenyl]methylene}amino)oxy]ethoxy}phenyl)acetate

By the same procedure as described in Example 1 using the compound prepared in Reference Example 5 instead of the compound prepared in Reference Example 1, the following compounds of the present invention were obtained.
TLC:Rf 0.30 (hexane : ethyl acetate = 1 : 1).

### Example 5:

### methyl {3-[2-({bis(3-formylphenyl)methylene]amino}oxy)ethoxy]phenyl}acetate

A solution of the compound prepared in Example 4 in THF (11 mL) was added by 1N hydrochloric acid (2.2mL) at a room temperature and stirred for 6 hours. The mixture was diluted with saturated sodium hyrdo carbonate and extracted by ethyl acetate. The extract was washed with water and saturated brine successively, dried over anhydrous magnesium sulfate and then concentrated to give the title compound having the following physical data.
TLC:Rf 0.44 (hexane : ethyl acetate = 1 : 1).

### Example 6:

### {3-[2-({[bis(3-formylphenyl)methylene]amino}oxy)ethoxy]phenyl]acetic acid

By the same procedure as described in Example 2 using the compound prepared in Example 5 instead of the compound prepared in Example 1, the compounds of the present invention having the following physical data were obtained.
TLC:Rf 0.31 (chloroform : methanol = 9 : 1);
NMR(CDCl₃): δ 3.61 (s, 2H], 4.26-4.29 (m, 2H), 4.53-4.57 (m, 2H), 6.82-6.88 (m, 3H), 7.23 (t, 1H), 7.52 (t, 1H), 7.60-7.62 (m, 2H), 7.72 (ddd, 1H), 7.86-7.97 (m, 4H), 9.98 (s, 1H), 10.00 (s, 1H).

### Example 7:

### 3,3'-[({2-[3-(2-methoxy-2-oxoethyl}phenoxy]ethoxy}imino)methylene]dibenzoic acid

A suspended solution of the compound prepared in Example 5 in t-butanol/water (30mL; 2:1) was added by sodium dihydrogen phosphate (674mg) and 2-methyl-2-butene (992µL) at a room temperature, cooled down to 0°C, added by sodium chlorite (927mg) and stirred for 30 minutes. The mixture was diluted with 1N hydrochloric acid and extracted by ethyl acetate. The extract was washed with saturated sodium thiosulfate aqueous solution, water and saturated brine successively, dried over anhydrous magnesium sulfate and concentrated to give the compound in the present invention. The obtained compounds were directly used in next reaction.

### Example 8:

### methyl [3-(2-{[(bis{3-[(dimethylamino)carbonyl]phenyl}methylene)amino]oxy}ethoxy)phenyl]acetate

A solution of the compound prepared in Example 7 in DMF (20mL) was added by 1-ethyl-3-(3-dimethylaminopropyl)carbodiimide • hydrochloride (2.7g; EDC), 1-hydroxybenzotriazol (788mg; HOBt) and N-methylmorphorine (1.5mL) at a room temperature, stirred for 5 minutes, added by a solution of dimethylamine in THF (15.0mL; 2M) and stirred for 24 hours. The mixture was diluted with water and then extracted by ethyl acetate. The extract was washed with water and saturated brine successively, dried over anhydrous magnesium sulfate and then concentrated. The residue was purified by column chromatography on silica gel (from hexane : ethyl acetate = 2 : 3 to ethyl acetate only to ethyl acetate ; methanol = 10 : 1) to give the title compounds (400mg) having the following physical data.
TLC:Rf 0.62 (chloroform : methanol = 9 : 1).

### Example 9:

### [3-(2-{[(bis{3-[(dimethylamino)carbonyl]phenyl}methylene)amino]oxy}ethoxy)phenyl]acetic acid

By the same procedure as described in Example 2 using the compound prepared in Example 8 instead of the compound prepared in Example 1, the compounds in the present invention having the following physical data were obtained.
TLC:Rf 0.38 (chloroform : methanol = 9 : 1);
NMR(CDCl₃): δ 2.93 (s, 3H), 2.97 (s, 3H), 3.08 (s, 3H), 3.11 (s, 3H), 3.53 (s, 2H), 4.31-4.34 (m, 2H), 4.45-4.48 (m, 2H), 6.78-6.89 (m, 3H), 7.16-7.21 (m, 1H), 7.26-7.49 (m, 7H), 7.57-7.58 (m, 1H).

### Reference Example 6:

### bis[4-tetrahydro-2H-pyran-2-yloxy)phenyl]methaneone

A solution of bis(4-hydroxyphenyl)methaneone (2.0g) in methylene chloride (20mL) was added by dihyropyran (2.6mL) and p-toluenesulfonic acid • pyridine salt (469mg) at 0°C and under atmosphere of argon stirred for 10 minutes at 0°C and for 2 hours at a room temperature. The mixture was cooled down to 0°C, added by water and extracted by ethyl acetate. The extract was washed with saturated brine, dried over anhydrous magnesium sulfate and then concentrated. The obtained residue was washed with a mixed solution (hexane : ethyl acetate = 4 : 1) to give the title compounds (2.3g) having the following physical data.
TLC:Rf 0.61 (hexane : ethyl acetate = 1 : 1).

### Example 10:

### methyl (3-{2-[({bis[4-(tetrahydro-2H-pyran-2-yloxy)phenyl]methylene}amino)oxy]ethoxy}phenyl)acetate

By the same procedure as described in Reference Example 5 and Example 1 using the compound prepared in Reference Example 6 instead of the compound prepared in Reference Example 4, the compounds in the present invention having the following physical data were obtained.
TLC:Rf 0.54 (hexane : ethyl acetate = 2 : 1).

### Example 11:

### methyl {3-[2-({[bis(4-hydroxyphenyl)methylene]amino}oxy)ethoxyphenyl}acetate

A solution of the compound prepared in Example 10 (230mg) in THF (10mL) was added by 2N hydrochloric acid (2mL) and stirred for 3 hours at a room temperature. The mixture was added by 2N sodium hydroxide aqueous solution until pH of the solution came to 7.0 and then extracted by ethyl acetate. The extract was washed with saturated brine, dried over anhydrous magnesium sulfate and concentrated. The residue was purified by column chromatography on silica gel (hexane : ethyl acetate = 2 : 1) to give the title compounds (160mg) having the following physical data.
TLC:Rf 0.42 (hexane : ethyl acetate = 1 : 1);
NMR(CDCl₃). δ 3.60 (s, 2H), 3.70 (s, 3H), 4.26 (t, 2H), 4.48 (t, 2H), 6.88-6.77 (m, 6H), 7.26-7.19 (m, 4H), 7.38-7.34 (m, 2H).

### Example 12:

### {3-[2-({[bis(4-hydroxyphenyl)methylene]amino}oxy)ethoxy]phenyl} acetic acid

By the same procedure as described in Example 2 using the compound prepared in Example 11 instead of the compound prepared in Example 1, the compounds in the present invention having the following physical data were obtained.
TLC:Rf 0.15 (chloroform : methanol = 9 : 1);
NMR(DMSO-d₆): δ 3.51 (s, 2H), 4.21-4.17 (m, 2H), 4.36-4.32 (m, 2H), 6.85-6.71 (m, 6H), 7.12 (d, 2H), 7.22-7.18 (m, 2H), 9.73 (bs, 2H), 12.29 (bs, 1H).

### Example 13:

### (3-{2-[({bis[4-(acetyloxy)phenyl]methylene}amino)oxy]ethoxy}phenyl)acetic acid

Under atmosphere of argon, a solution of the compound prepared in Example 12 (210mg) in pyridine (3mL) was added by acetic anhydride (0.2mL) and 4-dimethylaminopyridine (4.0mg) and stirred for 30 minutes at a room temperature. The mixture was poured into iced water, neutralized by adding 1N hydrochloric acid and then extracted by ethyl acetate. The extract was washed with saturated brine, dried over anhydrous magnesium sulfate and then concentrated. The residue was purified by column chromatography on silica gel (hexane : ethyl acetate = 9 : 1) to give the title compounds (182g) having the following physical data.
TLC:Rf 0.55 (chloroform : methanol = 9 : 1);
NMR(CDCl₃): δ 2.30 (s, 3H), 2.32 (s, 3H), 3.60 (s, 2H), 4.26 (t, 2H), 4.50 (t, 2H), 6.88-6.83 (m, 3H), 7.07-7.04 (m, 2H), 7.14-7.12 (m, 2H),7.23 (t, 1H), 7.40-7.36 (m, 2H), 7.50-7.47(m,2H).

### Example 14:

### {3-[2-({[(1E)-1-(4-pyridin-2-ylphenyl)propylidene]amino}oxy)ethoxy]phenyl}acetic acid

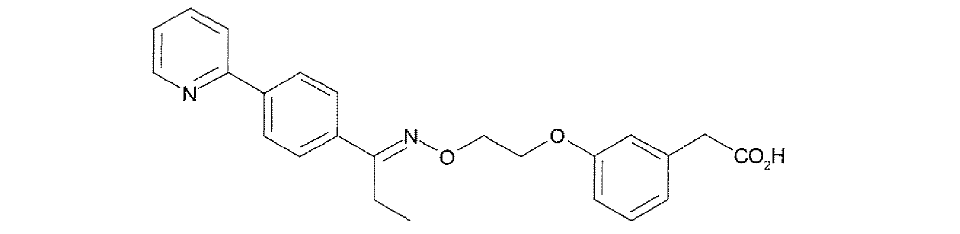

### Process 1:

Under atmosphere of argon, a solution of 4-(pyridyl-2-yl)benzaldehyde (1.8g) in anhydrous tetrahydrofuran (10mL) was dropped by ethylbromomagnesium (3M diethylether solution; 3.7mL) at -78°C and the mixture was stirred for 2 hours within -50 to -40°C. The mixture was added by water at 0°C and extracted by ethyl acetate. The organic layer was washed with saturated brine, dried over and then concentrated to give the rough purification compounds of 1-(pyridin-2-ylphenyl)propanol having the following physical data.
TLC:Rf 0.43 (n-hexane : ethyl acetate = 1 : 1).

### Process 2:

A solution of the rough purification compounds 1-(pyridin-2-ylpllenyl)propanol (2.0g) in dimethylsulfoxide (38mL)/dichloromethane (19mL) was added by triethylamine (8.1mL), added by sulfur trioxide-pyridine complex (4.6g) at 0°C and the mixture was stirred for 5 minutes at 0°C and for an hour at a room temperature. The mixture was added by water at 0°C and extracted by ethyl acetate. The organic layer was washed with water and saturated brine successively, dried over and then concentrated to give the rough purification compounds of I-(4--pyridin-2-ylphenyl)propan-1-one having the following physical data
TLC:Rf 0.63 (n-hexane : ethyl acetate = 1 : 1).

### Process 3:

By the same procedure as described in Reference Example 1 using the compound prepared in Process 2 instead of bis(4-methylphenyl)methaneone, 1-(4-pyridin-2-ylphenyl)propan-1-one oxime was obtained.

### Process 4:

By the same procedure as described in Example 1 and 2 using the compound prepared in Process 3 instead of the compound prepared in Reference Example 1, the compounds in the present invention having the following physical data were obtained.
melting point (degree centigrade): 162.0 - 165.0;
TLC.Rf 0.48 (methanol : methylene chloride = 1 : 10);
NMR(DMSO-d₆): δ 12.29 (bs, 1H), 8.68 (d, 1H), 8.12 (d, 2H), 7.98 (d, 1H), 7.88 (dt, 1H), 7.78 (d, 2H), 7.36 (dd, 1H), 7.20 (t, 1H), 6.91-6.79 (m, 3H), 4.47 (t, 2H), 4.25 (t, 2H), 3.52 (s, 2H), 2.75 (q, 2H), 1.06 (t, 3H).

### Example 14(1)-Example 14(184)

By the same procedure as described in Example 14 using 4-(pyridin-2-yl)benzaldehyde or the corresponding compounds instead of thereof, bromoethylmagnesium or the corresponding compounds instead of thereof and the compound prepared in Reference Example 3 or the corresponding compounds instead of thereof, additionally if necessary, by converting into a corresponding salt by known method, the following compounds of the present invention were obtained. Also, NMR data of the following compounds of the present invention was described as the characteristic peak.

### Example 14(1):

{3-[2-({[(4-fulorophenyl)methylene]amino}oxy)ethoxy]phenyl}acetic acid
TLC.Rf 0.44 (chloroform : methanol = 9 : 1);
NMR(CDCl₃): δ 3.59, 3.61 (s, 2H), 7.48, 8.10 (s, 1H).

### Example 14(2):

{3-[2-({[bis(4-fulorophenyl)methylene]amino}oxy)ethoxy]-4-methylphenyl}acetic acid
TLC:Rf 0.54 (chloroform : methanol = 9 : 1);
NMR(CDCl₃): δ 2.18 (s, 3H), 3.58 (s, 2H), 4.50-4.53 (m, 2H).

### Example 14(3):

(3-{2-[({(1E)-[4-(trifuloromethyl)phenyl]methylene}amino)oxy]ethoxy}phenyl)acetic acid
TLC:Rf 0.45 (chloroform : methanol = 9 : 1);
NMR(CDCl₃): δ 3.62 (s, 2H), 8.16 (s, 1H).

### Example 14(4):

{3-[2-({[(1E)-1-(4-fulorophenyl)ethylidene]amino }oxy)ethoxy]phenyl} acetic acid
TLC:Rf 0.35 (chloroform : methanol = 9 : 1);
NMR(CDCl₃): δ 2.22 (s, 3H), 3.62 (s, 2H), 4.50-4.53 (m, 2H).

### Example 14(5):

(3-{2-[({(1E)-1-[4-(trifuloromethyl)phenyl]ethylidene}amino)oxy]ethoxy}phenyl)acetic acid
TLC:Rf 0.35 (chloroform : methanol = 9 : 1);
NMR(CDCl₃): δ 2.25 (s, 3H), 3.62 (s, 2H), 4.53-4.57 (m, 2H)

### Example 14(6):

{3-[2-({[(1E)-1-(4-methylphenyl)ethylidene]amino}oxy)ethoxy]phenyl}acetic acid
TLC:Rf 0.31 (chloroform : methanol = 9 : 1);
NMR(CDCl₃): δ 2.22 (s, 3H), 2.36 (s, 3H), 3.62 (s, 2H).

### Example 14(7):

(3-{2-[({(1E)-1-[4-(trifuloromethyl)phenyl]propylidene}amino)oxy]ethoxy}phenyl)acetic acid
TLC:Rf 0.42 (chloroform : methanol = 9 : 1);
NMR(CDCl₃): δ 1.12 (t, 3H), 3.61 (s, 2H).

### Example 14(8):

{3-[2-({[(1E)-1-(4-methylphenyl)propylidene]amino}oxy)ethoxy]phenyl} acetic acid
TLC:Rf 0.35 (chloroform : methanol = 9 : 1);
NMR(CDCl₃): δ 1.11 (t, 3H), 2.35 (s, 3H), 3.50 (s, 2H).

### Example 14(9):

{3-[2-({[(1E)-1-(4-methoxyphenyl)propylidene]amino}oxy)ethoxy]phenyl}acetic acid
TLC:Rf 0.38 (chloroform : methanol =9 : 1);
NMR(CDCl₃): δ 1.11 (t, 3H), 3.61 (s, 2H), 3.82 (s, 3H).

### Example 14(10):

3-[2-({[(1E)-1-(4-chlorophenyl)propylidene]amino}oxy)ethoxy]phenyl}acetic acid
TLC:Rf 0.34 (chloroform : methanol = 9 : 1);
NMR(CDCl₃): δ 1.10 (t, 3H), 3.61 (s, 2H).

### Example 14(11):

{3-[2-({[(1E)-1-(4-methylphenyl)butylidene]amino}oxy)ethoxy]phenyl}acetic acid
TLC.Rf 0.36 (chloroform : methanol = 9 : 1);
NMR(CDCl₃): δ 0.93 (t, 3H), 2.35 (s, 3H), 3.61 (s, 2H).

### Example 14(12):

(3-{2-[({(1E)-1-[4-(trifuloromethyl)phenyl]butylidene} amino)oxy]ethoxy}phenyl)acetic acid
TLC:Rf 0.33 (chloroform , methanol = 9 : 1);
NMR(CDCl₃): δ 0.94 (t, 3H), 3.62 (s, 2H).

### Example 14(13):

(3-{2-[({2-methyl-1-[4-(trifuloromethyl)phenyl]propylidene}amino)oxy]ethoxy}phenyl)acetic acid.
TLC:Rf 0.29 (chloroform : methanol = 9 : 1);
NMR(CDCl₃); δ 1.10, 1.18 (d, 6H), 3.61, 3.62 (s, 2H).

### Example 14(14):

(3-{2-[({(1E)-1-[4-(trifuloromethyl)phenyl]pentylidene}amino)oxy]ethoxy}phenyl)acetic acid
TLC:Rf 0.36 (chloroform : methanol = 9 : 1);
NMR(CDCl₃): δ 0.88 (t, 3H), 3.61 (s, 2H)

### Example 14(15):

(3-{2-[({(1E)-3-methyl-1-[4-(trufuloromethyl)phenyl]butylidene}amino)oxy]ehtoxy}phenyl)acetic acid
TLC:Rf 0.33 (chloroform : methanol = 9 : 1);
NMR(CDCl₃): δ 0.89 (d, 6H), 3.61 (s, 2H).

### Example 14(16):

{3-[2-({[(1E)-1-pyridin-4-ylpropylidene]amino}oxy)ethoxy]phenyl}acetic acid
TLC:Rf 0.36 (chloroform : methanol = 9 : 1);
NMR(DMSO-d₆): 5 1.01 (t, 1H), 3.51 (s, 2H), 8.60 (d, 2H).

### Example 14(17):

{3-[2-({[(1E)-1-biphenyl-4-ylpropylidene]amino}oxy)ethoxy]phenyl}acetic acid
melting point (degree centigrade): 135.0 - 137.0;
TLC:Rf 0.46 (methanol : methylene chloride = 1 : 10);
NMR(DMSO-d₆): δ 4.46 (t, 2H), 3.52 (s, 2H), 1.05 (t, 3H).

### Example 14(18):

{3-[2-({[(1E)-1-pyridin-3-ylpropylidene]amino}oxy]ethoxy]phenyl}acetic acid
TLC:Rf 0.39 (methanol: methylene chloride = 1 : 10);
mm(DMSO-d₆); δ 4.46 (t, 2H), 3.51 (s, 2H), 1.02 (t, 3H).

### Example 14(19):

sodium (3-{3-[({(1E)-1-[4-(trifuloromethyl)phenyl]propylidene}amino)oxy]propoxy}phenyl)acetate
TLC:Rf 0.53 (methanol: methylene chloride = 1 : 10);
NMR(DMSO-d₆): δ 4.32 (t, 2H), 3.17 (s, 2H), 1.04 (t, 3H).

### Example 14(20);

{3-[2-({[(1E)-1-(4-ethylphenyl)propylidene]amino }oxy)ethoxy]phenyl} acetic acid
TLC:Rf 0.57 (methanol: methylene chloride = 1 : 10);
NNM(DMSO-d₆): δ 4.42 (t, 2H), 3.51 (s, 2H), 1.01 (t, 3H).

### Example 14(21):

{3-[2-({[(1E)-1-(4'-ethylbiphenyl-4-yl)propylidene]amino}oxy)ethoxy]phenyl}acetic acid
TLC:Rf 0.26 (chloroform : methanol = 9 : 1);
NMR(CDCl₃): δ 1.15 (t, 3H), 3.62 (s, 2H), 4.53 (t, 2H).

### Example 14(22):

sodium {3-[2-({[(1E)-1-(4-isopropylphenyl)propylidene]amino}oxy)ethoxy]phenyl }acetate
TLC:Rf 0.59 (methanol: methylene chloride = 1 : 10);
NMR(DMSO-d₆): δ 4.40 (t, 2H), 3.18 (s, 2H), 1.03 (t, 3H).

### Example 14(23):

sodium {3-[2-({[(1E)-1-(4-tert-butylphenyl)propylidene]amino}oxy)ethoxy]phenyl} acetate
TLC:Rf 0.56 (methanol : methylene chloride = 1 : 10);
NMR(DMSO-d₆) : δ 4.41 (t, 2H), 3.18 (s, 2H), 1.03 (t, 3H).

### Example 14(24):

(3-{2-[({(1E)-1-[3-(trifuloromethyl)phenyl]propylidene}amino)oxy]ethoxy}phenyl)acetic acid
TLC-RF 0.43 (chloroform : methanol = 9 : 1);
NMR(CDCl₃): δ 1.13 (t, 3H), 3.61 (s, 2H).

### Example 14(25):

{3-[2-({ [(1E)-1-(4'-chlorobiphenyl-4-yl)propylidene]amino}oxy)ethoxy]phenyl} acetic acid
TLC:Rf 0.54 (methanol: methylene chloride = 1 : 10);
NMR(DMSO-d₆): δ 4.58 (t, 2H), 3.52 (s, 2H), 1.05 (t, 3H).

### Example 14(26):

{3-[2-({[(1E)-1-(4'-methylbiphenyl-4-yl)propylidene]amino}oxy)ethoxy]phenyl}acetic acid
TLC:Rf 0.55 (methanol : methylene chloride = 1 : 10);
NMR(DMSO-d₆): δ 4.45 (t, 2H), 3.52 (s, 2H), 1.05 (t, 3H).

### Example 14(27):

{3-[2-({[(1E)-1-(4-phenoxyphenyl)propylidene]amino}oxy)ethoxy]phenyl}acetic acid
TLC:Rf 0.28 (chloroform: methanol = 9 : 1);
NMR(CDCl₃): δ 1.12 (t, 3H), 3.61 (s, 2H), 4.50 (t, 2H).

### Example 14(28):

{3-[2-({[(1E)-1-(4'-fulorobiphenyl-4-yl)propylidene]amino}oxy)ethoxy]phenyl}acetic acid
TLC:Rf 0.54 (methanol : methylene chloride = 1 : 10);
NMR(DMSO-d₆): δ 4.56 (t, 2H), 3.52 (s, 2H), 1.05 (t, 3H).

### Example 14(29):

(3-{2-[({(1E)-1-[4'-(trifuloromethyl)biphenyl-4-yl]propylidene}amino)oxy]ethoxy}phenyl)acetic acid
TLC:Rf 0.50 (chloroform : methanol = 9 : 1);
NMR(CDCl₃): δ 1.15 (t, 3H), 3.61 (s, 2H), 7.70 (s, 4H).

### Example 14(30):

{3-{2-[({(1E)-1-[4-(2-thienyl)phenyl]propylidene}amino)oxy]ethoxy}phenyl)acetic acid melting point (degree centigrade); 117.0 - 120.0;
TLC:Rf 0.35 (chloroform : methanol = 9 : 1);
NMR(CDCl₃): δ 1.13 (t, 3H), 3.60 (s, 2H), 4.52 (t, 2H).

### Example 14(31):

sodium {3-[2-({[(1E)-1-(2-phenyl-1,3-thiazol-4-yl)propylidene]amino}oxy)ethoxy]phenyl}acetate
TLC:Rf 0.58 (chloroform : methanol : acetic acid = 99 : 9 : 1);
NMR(CD₃OD): δ 1.17 (t, 2.28H), 4.50 (t, 2H).

### Example 14(32):

{3-[2-({[(1E)-1-(4'-isopropybiphenyl-4-yl)propylidene]amino} oxy)ethoxy]phenyl} acetic acid
TLC:Rf 0.45 (methanol : methylene chloride = 1 : 10);
NMR(DMSO-d₆): δ 4.45 (t, 2H), 3.52 (s, 2H), 1.05 (t, 3H).

### Example 14(33):

{3-[2-({[(1E)-1-(4-pyridin-3-ylphenyl)propylidene]amino}oxy)ethoxy]phenyl}acetic acid
TLC: Rf 0.48 (methanol : methylene chloride = 1 : 10);
NMR(DMSO-d₆): δ 4.46 (t, 2H), 3.52 (s, 2H), 1.05 (t, 3H).

### Example 14(34):

{3-[2-({[(1E)-1-(4-pyridin-4-ylphenyl)propylidene]amino}oxy)ethoxy]phenyl}acetic acid
TLC:Rf 0.35 (methanol : methylene chloride = 1 : 10);
NMR(DMSO-d₆): δ 4.47 (t, 2H), 3.52 (s, 2H), 1.05 (t, 3H).

### Example 14(35):

(3-{2-[({(1E)-1-[4-(1H-pyrazol-1-yl)phenyl]propylidene}amino)oxy]ethoxy}phenyl)acetic acid
melting point (degree centigrade): 143.5 - 147.0;
TLC:Rf 0.41 (chloroform : methanol = 9 : 1);
NMR(CD₃OD): δ 1.11 (t, 3H), 3.55 (s, 2H), 4.49 (t, 2H).

### Example 14(36):

(3-{2-[({(1E)-1-[4-(1H-pyrol-1-yl)phenyl]propylidene}amino)oxy]ethoxy}phenyl)acetic acid
TLC:Rf 0.42 (chloroform : methanol = 9 : 1);
NMR(CD₃OD): δ 1.10 (t, 3H), 3.53 (s, 2H), 4.47 (t, 2H).

### Example 14(37):

(3-{2-[({(1E)-1-[4'-(trifuloromethoxy)biphenyl-4-yl]propylidene}amino)oxy]ethoxy}phenyl)acetic acid
TLC:Rf 0.49 (methanol : methylene chloride = 1 : 10);
NMR(DMSO-d₆): δ 4.46 (t, 2H), 3.52 (s, 2H), 1.05 (t, 3H).

### Example 14(38):

{3-[2-({[(1E)-1-(4'-methoxybiphenyl-4-yl)propylidene]amino}oxy)ethoxy]phenyl}acetic acid
TLC:Rf 0.49 (methanol : methylene chloride = 1 : 10);
NMR(DMSO-d₆): δ 3.78 (s, 3H), 3.51 (s, 2H), 1.05 (t, 3H).

### Example 14(39):

(3-{2-[({(1E)-1-[4-(1H-1,2,4-triazol-1-yl)phenyl]propylidene}amino)oxy]ethoxy}phenyl)acetic acid
TLC:Rf 0.73 (ethyl acetate);
NMR(DMSO-d₆): δ 1.04 (t, 3H), 3.51 (s, 2H).

### Example 14(40):

sodium {3-[2-({[(1E)-1-(5-phenyl-2-furyl)propylidene]amino}oxy)ethoxy]phenyl}acetate
TLC:Rf 0.52 (n-hexane : ethyl acetate = 1 : 10);
NMR(DMSO-d₆): δ 1.10 (t, 2.4H), 3.15 (s, 2H), 4.44 (t, 2H).

### Example 14(41):

{3-[2-({[(1E}-1-(2,2-difuloro-1,3-benzodioxol-5-yl)propylidene]amino}oxy)ethoxy]phenyl} acetic acid
TLC:Rf 0.40 (chloroform : methanol = 9 : 1);
NMR(CDCl₃): δ 1.11 (t, 3H), 3.61 (s, 2H), 4.50 (t, 2H).

### Example 14(42):

{3-[2-{{[(1E)-1-(2,2'-bithien-5-yl)propylidene]amino}oxy}ethoxy]phenyl}acetic acid
TLC:Rf 0.26 (chloroform : methanol = 9 : 1);
NMR(CDCl₃): δ 1.17 (t, 3H), 3.60 (s, 2H), 4.48 (t, 2H).

### Example 14(43):

(3-{2-[({(1E)-1-[4-(3-thienyl)phenyl]propylidene}amino)oxy]ethoxy}phenyl)acetic acid
TLC:Rf 0.37 (chloroform : methanol = 9 : 1);
NMR(CDCl₃}: δ 1.14 (t, 3H), 3.60 (s, 2H), 4.52 (t, 2H).

### Example 14(44):

(3-{2-[({(1E)-1-[4-(3-furyl)phenyl]propylidene}amino)oxy]ethoxy}phenyl)acetic acid
TLC:Rf 0.50 (chloroform : methanol = 9 : 1);
NMR(CDCl₃): δ 1.13 (t, 3H), 3.61 (s, 2H), 4.52 (t, 2H).

### Example 14(45):

{3-[2-({[(1E)-1-(2-naphthyl)propylidene]amino}oxy)ethoxy]phenyl} acetic acid
TLC:RF 0.42 (chloroform : methanol = 9 : 1);
NMR(CDCl₃): δ 1.19 (t, 3H), 3.61 (s, 2H), 4.27-4.33 (m, 2H).

### Example 14(46):

(3-{2-[({(1E)-1-[4-(2-furyl)phenyl]propylidene}amino)oxy]ethoxy}phenyl)acetic acid
TLC:Rf 0.36 (chloroform : methanol = 9 : 1);
NMR(CDCl₃): δ 1.13 (t, 3H), 3.61 (s, 2H), 4.52 (t, 2H),

### Example 14(47):

{3-[2-({[(1E)-1-(4-pyrrolidin-1-ylphenyl)propylidene]amino}oxy)ethoxy]phenyl} acetic acid
TLC:Rf 0.33 (chloroform: methanol = 9 : 1);
NMR(CDCl₃) : δ 1.11 (t, 3H), 3.59 (s, 2H), 4.46 (t, 2H).

### Example 14(48):

{3-[2-({[(1E)-1-(4-piperidin-1-ylphenyl)propylidene]amino}oxy)ethoxy]phenyl}acetic acid
TLC:Rf 0.3 6 (chloroform : methanol = 9 : 1);
NMR(CDCl₃): δ 1.11 (t, 3H), 3.60 (s, 2H), 4.47 (t, 2H).

### Example 14(49):

sodium {3-[2-({[(1E)-1-dibenzo[b,d]furan-2-ylpropylidene]amino}oxy)ethoxy]phenyl}acetate
TLC:Rf 0.59 (methanol : methylene chloride = 1 : 9);
NMR(DMSO-d₆): δ 4.47 (t, 2H), 3.20 (s, 2H), 1.10 (t, 3H).

### Example 14(50):

{3-[2-({[(1E)-1-(6-phenylpyridin-3-yl)propylidene]amino}oxy)ethoxy]phenyl}acetic acid
TLC:Rf 0.47 (methanol: methylene chloride = 1 : 9);
NMR(DMSO-d₆): δ 4.48 (t, 2H), 3.52 (s, 2H), 1.06 (t, 3H).

### Example 14(51):

{3-[2-({[(1E)-1-(4-morpholin-4-ylphenyl)propylidene]amino}oxy)ethoxy]phenyl} acetic acid
TLC:Rf 0.38 (chloroform : methanol = 9 : 1);
NMR(CDCl₃): δ 1.11 (t, 3H), 3.60 (s, 2H), 4.48 (t, 2H).

### Example 14(52):

(3-{2-[({1E)-1-[4-(4-methylpiperazin-1-yl)phenyl]propylidene}amino)oxy]ethoxy}phenyl)acetic acid
TLC:Rf 0.21 (chloroform : methanol = 9 : 1);
NMR(CDCl₃): δ 1.08 (t, 3H), 2.43 (s, 3H), 3.55 (s, 2H).

### Example 14(53):

{3-[2-({[(1E)-1-biphneyl-4-ylpropylidene]amino}oxy)ethoxy]-2-fulorophenyl}acetic acid
TLC:Rf 0.30 (chloroform: methanol = 9 : 1);
NMW(CDCl₃): δ 1.15 (t, 3H), 3.71 (d, 2H).

### Example 14(54):

{3-[2-({[(1E)-1-biphenyl-4-ylpropylidene]amino} oxy)ethoxy]-4-fulorophenyl} acetic acid
TLC:Rf 0.39 (chloroform : methanol = 9 : 1);
NMR(CDCl₃): δ 1.15 (t, 3H), 3.57 (s, 2H), 4.54 (t, 2H).

### Example 14(55):

(3-{2-[({(1E)-1-[4-(1H-imidazol-1-yl)phenyl]propylidene}amino)oxy]ethoxy}phenyl)acetic acid
TLC:Rf 0.29 (chloroform : methanol = 9 : 1);
NMR(DMSO-d₆): δ 1.04 (t, 3H), 3.51 (s, 2H), 4.46 (t, 2H).

### Example 14(56):

(3-{2-[({(1E)-1-[4-(1H-pyrazol-1-yl)phenyl]ethylidene}amino)oxy]ethoxy}phenyl)acetic acid
TLC:Rf 0.32 (chloroform : methanol = 9 : 1);
NMR(CDCl₃): δ 2.25 (s, 3H), 3.62 (s, 2H).

### Example 14(57):

(3-{2-[({(1E)-1-[4-(1H-pyrazol-1-yl]phenyl]butylidene}amino)oxy]ethoxy}phenyl)acetic acid
TLC:Rf 0.32 (chloroform : methanol = 9 : 1);
NMR(CDCl₃) : δ 0.95 (t, 3H), 3.61 (s, 2H), 4.52 (t, 2H).

### Example 14(58):

[3-(2-{[((1E)-1-(4-[4-(trifuloromethyl)piperidin-1-yl]phenyl}propylidene)amino]oxy}ethoxy)phenyl]acetic acid
TLC:Rf 0.36 (chloroform : methanol = 9 : 1);
NMR(CDCl₃): δ 1.09 (q, 3H), 3.60 (s, 2H), 4.48 (t, 2H).

### Example 14(59):

(3-{2-[({(1E)-1-[4-(1,3-thiazol-2-yl)phenyl]propylidene}amino)oxy]ethoxy}phenyl)acetic acid
TLC:Rf 0.38 (chloroform : methanol = 9 : 1);
NMR(CDCl₃): δ 1.14 (t, 3H), 3.61 (s, 2H), 4.53 (t, 2H).

### Example 14(60):

{3-[2-({[(1E)-1-biphenyl-4-ylpropylidene]amino}oxy)ethoxy]-4-methylphenyl}acetic acid
TLC:Rf 0.42 (chloroform : methanol = 9 : 1);
NNM(CDCl₃): δ 1.16 (t, 3H), 2.22 (s, 3H), 3.58 (s, 2H).

### Example 14(61):

{3-[2-({[(1E)-1-(4-pyridin-2-ylphenyl)ethylidene]amino}oxy)ethoxy]phenyl}acetic acid
TLC:RF 0.57 (methanol : methylene chloride = 1 : 9);
NMR(DMSO-d₆): δ 4.48 (t, 2H), 3.52 (s, 2H), 2.22 (s, 3H).

### Example 14(62):

(3-{2-[({(1E)-1-[4-(1H-pyrazol-1-yl)phenyl]pentylidene}amino)oxy]ethoxy}phenyl)acetic acid
TLC:Rf 0.33 (chloroform : methanol = 9 : 1);
NMR(CDCl₃): δ 0.89 (t, 3H), 3.61 (s, 2H).

### Example 14(63):

sodium (3-{2-[({(1E)-3-methyl-1-[4-(1H-pyrazol-1-yl)phenyl]butylidene}amino)oxy]ethoxy}phenyl)acetate
TLC:Rf 0.37 (chloroform : methanol = 9 : 1),
NMR(DMSO-d₆): δ 0.85 (d, 6H), 3.13 (s, 2H).

### Example 14(64):

{3-[2-({[(1E)-1-biphenyl-4-ylpropylidene]amino} oxy)ethoxy]-2- methylphenyl} acetic acid
melting point (degree centigrade): 152.5 - 154.0;
TLC:Rf 0.53 (chloroform: methanol = 9 : 1);
NMR(CDCl₃): δ 1.16 (t, 3H), 2.22 (s, 3H), 3.67 (s, 2H).

### Example 14(65):

{5-[2-({ [(1E)-1-biphenyl-4-ylpropylidene]amino}oxy)ethoxy]-2-fluorophenyl}acetic acid
TLC:Rf 0.49 (chloroform : methanol = 9 : 1);
NMR(CDCl₃): δ 1.15 (t, 3H), 3.66 (s, 2H), 4.50 (t, 2H).

### Example 14(66):

{3-[2-({[(1E)-1-biphenyl-4- ylpropylidene]amino}oxy)ethoxy]-5-methylphenyl} acetic acid
TLC:Rf 0.50 (chloroform : methanol = 9 : 1);
NMR(CDCl₃): δ 1.15 (t, 3H), 2.30 (s, 3H), 3.56 (s, 2H).

### Example 14(67):

{3-[2-({[(1E)-1-(4-pyridin-2-ylphenyl)butylidene]amino}oxy)ethoxy]phenyl acetic acid
TLC:Rf 0.51 (methanol : methylene chloride = 1 : 9);
NMR(DMSO-d₆): δ 4.46 (t, 2H), 3.52 (s, 2H), 0.88 (t, 3H).

### Example 14(68):

sodium {3-[2-({[(1E)-3-methyl-1-(4-pyridin-2-ylphenyl)butylidene]amino}oxy)ethoxy]phenyl}acetate
TLC:Rf 0.51 (methanol: methylene chloride = 1 : 9);
NMR(DMSO-d₆): δ 4.44 (t, 2H), 3.19 (s, 2H), 0.85 (d, 6H).

### Example 14(69):

{-[2-({[(1E)-1-biphenyl-4-ylpropylidene]amino}oxy)ethoxy]-4-chlorophenyl}acetic acid
TLC:Rf 0.53 (chloroform : methanol = 9 : 1);
NMR(CDCl₃): δ 1.15 (t, 3H), 3.57 (s, 2H), 4.56 (t, 2H).

### Example 14(70):

{3-[2-({[(1E)-1-biphenyl-4-ylpropylidene]amino} oxy)ethoxy]-4-methoxyphenyl} acetic acid
TLC:Rf 0.50 (chloroform : methanol = 9 : 1);
NMR(CDCl₃): δ 1.16 (t, 3H), 3.54 (s, 2H), 3.84 (s, 3H).

### Example 14(71):

{2-[2-({[(1E)-1-biphenyl-4-ylpropylidene]amino}oxy)ethoxy]phenyl} acetic acid
TLC:Rf 0.44 (chloroform : methanol = 9 : 1);
NMR(CDCl₃): δ 1.14 (t, 3H), 3.70 (s, 2H), 4.53 (t, 2H).

### Example 14(72):

{4-[2-({[(1E)-1-biphenyl-4-ylpropylidene]amino}oxy)ethoxy]phenyl}acetic acid
TLC:Rf 0.47 (chloroform : methanol = 9 : 1);
NMR(CDCl₃): δ 1.15 (t, 3H), 3.58 (s, 2H), 4.52 (t, 2H).

### Example 14(73):

{3-[2-({[(1E)-1-(4-pyridin-2-ylphenyl)pentylidene]amino}oxy)ethoxy]phenyl}acetic acid
TLC:Rf 0.53 (methanol : methylene chloride = 1 : 9);
NMR(DMSO-d₆): δ 4.46 (t, 2H), 3.52 (s, 2H), 0.82 (t, 3H).

### Example 14(74):

sodium (3-{2-[({(1E)-1-[3-(1H-pyrazol-1-yl)phenyl]propylidene}amino)oxy]ethoxy}phenyl)acetate
TLC:Rf 0.36 (chloroform : methanol = 9 : 1);
NMR(DMSO-d₆): δ 1.06 (t, 3H), 3.16 (s, 2H).

### Example 14(75):

(2-fluoro-3-{2-[({(1E)-1-[4-(1H-pyrazol-1-yl)phenyl]propylidene}amino)oxy]ethoxy}phenyl)acetic acid
TLC:Rf 0.47 (chloroform : methanol = 9 : 1);
NMR(CDCl₃): δ 1.14 (t, 3H), 3.66 (s, 2H), 4.55 (t, 2H).

### Example 14(76):

sodium {3-[2-({[(1E)-1-(3-pyridin-2-ylphenyl)propylidene]amino}oxy)ethoxy]phenyl}acetate
TLC:Rf 0.36 (chloroform : methanol = 9 : 1);
NMR(DMSO-d₆): δ 1.07 (t, 3H), 3.16 (s, 2H).

### Example 14(77):

(2-fluoro-3-{2-[({(1E)-1-[4-2-thienyl)phenyl]propylidene}amino)oxy]ethoxy}phenyl)acetic acid
TLC:Rf 0.48 (chloroform : methanol = 9 : 1);
NMR(CDCl₃): δ 1.13 (t, 3H), 3.71 (s, 2H), 4.54 (t, 2H).

### Example 14(78):

{2-methyl-3-{2-[({(1E)-1-[4-(2-thienyl)phenyl]propylidene}amino)oxy]ethoxy}phenyl)acetic acid
TLC:Rf 0.49 (chloroform : methanol = 9 : 1);
NMR(CDCl₃): δ 1.14 (t, 3H), 2.22 (s, 3H), 3.69 (s, 2H).

### Example 14(79):

{3-[2-({[(1E)-1-biphenyl-4-ylpropylidene]amino}oxy)ethoxy]-2-methoxyphenyl}acetic acid
TLC:Rf 0.54 (chloroform : methanol = 9 : 1);
NMR(CDCl₃): δ 1.14 (t, 3H), 3.70 (s, 2H), 3.92 (s, 3H).

### Example 14(80):

{2-fluoro-3-[2-({[(1E)-1-(4-pyridin-2-ylphenyl)propylidene]amino}oxy)ethoxy]phenyl} acetic acid
TLC:RF 0.32 (chloroform : methanol = 9 : 1);
NMR(DMSO-d₆): δ 1.05 (t, 3H), 3.59 (s, 2H).

### Example 14(81):

(2-methoxy-3-{2-[({(1E)-1-[4-(2-thienyl)phenyl]propylidene}amino)oxy]ethoxy}phenyl)acetic acid
TLC:Rf 0.34 (chloroform : methanol = 9 : 1);
NMR(CDCl₃): δ 1.12 (t, 3H), 3.68 (s, 2H), 3.89 (s, 3H).

### Example 14(82):

(2-methoxy-3-{2-[({(1E)-1-[4-(1H-pyrazol-1-yl)phenyl]propylidene}amino)oxy]ethoxy}phenyl)acetic acid
TLC:Rf 0.33 (chloroform : methanol = 9 : 1);
NMR(CDCl₃): δ 1.12 (t, 3H), 3.68 (s, 2H), 3.89 (s, 3H).

### Example 14(83):

{2-methoxy-3-[2-({[(1E)-1-(4-pyridin-2-ylphenyl)propylidene]amino}oxy)ethoxy]phenyl} acetic acid
TLC:Rf 0.40 (chloroform : methanol = 9 : 1);
NMR(DMSO-d₆): δ 1.04 (t, 3H), 3.50 (s, 2H), 3.73 (s, 3H).

### Example 14(84):

(2-fluoro-3-{2-[({(1E)-1-[4-(3-thienyl)phenyl]propylidene}amino)oxy]ethoxy }phenyl)acetic acid
TLC:Rf 0.29 (chloroform : methanol = 9 : 1);
NMR(DMSO-d₆): δ 1.03 (t, 3H), 3.58 (d, 2H).

### Example 14(85):

(2-methoxy-3-{2-[({(1E)-1-[4-(3-thienyl)phenyl]propylidene}amino)oxy]ethoxy}phenyl)acetic acid
TLC:Rf 0.27 (chloroform : methanol = 9 : 1);
NMR(DMSO-d₆): δ 1.03 (t, 3H), 3.50 (s, 2H), 3.72 (s, 3H).

### Example 14(86):

(2-methyl-3-{2-[({(1E)-1-[4-(1H-pyrazol-1-yl)phenyl]propylidene}amino)oxy]ethoxy}phenyl)acetic acid
TLC:Rf 0.42 (chloroform : methanol = 9 : 1);
NMR(DMSO-d₆): δ 1.05 (t, 3H), 2.08 (s, 3H), 3.55 (s, 2H).

### Example 14(87):

{2-methyl-3-[2-({(1E)-1-(4-pyridin-2-ylphenyl)propylidene}anaino)oxy]ethoxy}phenyl)acetic acid
TLC:Rf 0.51 (chloroform: methanol = 9 : 1);
NMR(DMSO-d₆): δ 1.05 (t, 3H), 2.08 (s, 3H), 3.55 (s, 2H).

### Example 14(88):

(2-methyl-3-{2-[({(1E)-1-[4-(3-thienyl)phenyl]propylidene}amino)oxy]ethoxy}phenyl)acetic acid
TLC:Rf 0.34 (chloroform : methanol = 9 : 1);
NMR(DMSO-d₆): δ 1.05 (t, 3H), 2.07 (s, 3H), 3.55 (s, 2H).

### Example 14(89):

(2-fluoro-3-{2-[({(1E)-1-[4-(trifluoromethyl)phenyl]propylidene}amino)oxy]ethoxy}phenyl)acetic acid
TLC:Rf 0.56 (methanol : methylene chloride = 1 : 9);
NMR(DMSO-d₆): δ 3.58 (d, 2H), 1.02 (t, 3H).

### Example 14(90):

(2-methoxy-3-{2-[({(1E)-1-[4-(trifluoromethyl)phenyl]propylidene}amino)oxy]ethoxy}phenyl)acetic acid
TLC:Rf 0.55 (methanol : methylene chloride = 1 : 9);
NMR(DMSO-d₆): δ 3.71 (s, 3H), 3.50 (s, 2H), 1.01 (t, 3H).

### Example 14(91):

(2-methyl-3- {2-[({(1E)-1-[4-(trifluoromethyl)phenyl]propylidene}amino)oxy]ethoxy}phenyl)acetic acid
TLC:Rf 0.48 (methanol : methylene chloride = 1 : 9);
NMR(DMSO-d₆): δ 3.55 (s, 2H), 2.07 (s, 3H), 1.04 (t, 3H).

### Example 14(92):

{5-[2-({[(1E)-1-biphenyl-4-ylpropylidene]amino}oxy)ethoxy]-2-methoxyphenyl}acetic acid
TLC:Rf 0.63 (chloroform : methanol = 9 : 1);
NMR(CDCl₃): δ 1.16 (t, 2H), 3.63 (s, 2H), 3.78 (s, 3H).

### Example 14(93):

{3-[2-({[(1E)-1-biphenyl-4-ylpropylidene]amino}oxy)ethoxy]-5-methoxyphenyl}acetic acid
TLC-Rf 0.54 (chloroform : methanol = 9 : 1);
NMR(CDCl₃): δ 1.15 (t, 3H), 3.57 (s, 2H), 3.76 (s, 3H).

### Example 14(94):

3-{3-[({(1E)-1-[4-(1H-pyrazol-1-yl)phenyl]propylidene}amino)oxy]propoxy}benzoic acid
TLC:Rf 0.36 (chloroform : methanol = 9 : 1);
NMR(CDCl₃): δ 1.15 (t, 3H), 4.40 (t, 2H).

### Example 14(95):

1-{2-[({(1E)-1-[4-(1H-pyrazol-1-yl)phenyl]propylidene}amino)oxy]ethyl}-1H-indole-4-carboxylic acid
TLC:Rf 0.55 (chloroform : methanol = 9 : 1);
NMR(CDCl₃): δ 1.01 (t, 3H), 6.48 (dd, 1H).

### Example 14(96):

sodium [3-((methoxycarbonyl) (2-[({(1E)-1-[4-(1H-pyraxol-1-yl)phenyl]propylidene}amino)oxy]ethyl}amino)phenyl]acetate
TLC:Rf 0.59 (methanol : methylene chloride = 1 : 9);
NMR(DMSO-d₆): δ 3.54 (s, 3H), 3.19 (s, 2H), 1.00 (t, 3H).

### Example 14(97):

sodium 2-(3-{[({(1E)-1-[4-(1H-pyrazol-1-yl)phenyl]propylidene}amino)oxy]methyl}phenoxy)benzoate
TLC:Rf 0.33 (chloroform : methanol = 9 : 1);
NMR(DMSO-d₆): δ 1.01 (t, 3H), 5.13 (s, 2H).

### Example 14(98):

1-[2-({[(1E)-1-biphenyl-4-ylpropylidene]amino}oxy)ethyl]-1H-indole-4-carboxylic acid
TLC:Rf 0.47 (chloroform : methanol = 9 : 1);
NMR(DMSO-d₆): δ 0.86 (t, 3H), 4.59 (t, 2H).

### Example 14(99):

3-{3-[2-({[(1E)-1-biphenyl-4-ylpropylidene]amino}oxy)ethoxy]phenyl}propanoic acid
TLC:Rf 0.45 (chloroform : methanol = 9 : 1);
NMR(CDCl₃): δ 1.15 (t, 3H), 2.64 (t, 2H).

### Example 14(100):

{3-[2-({[(1E)-1-biphenyl-4-ylbutylidene]amino}oxy)ethoxy] phenyl}acetic acid
TLC:Rf 0.53 (chloroform: methanol = 9 : 1);
NMR(CDCl₃): δ 0.96 (t, 3H), 3.61 (s, 2H).

### Example 14(101):

{3-[2-({[(1E)-1-biphenyl-4-ylbutylidene]amino}oxy)ethoxy]-2-methylphenyl)acetic acid
melting point (degree centigrade): 150.0 - 152.0;
TLC:Rf 0.53 (chloroform : methanol = 9 : 1);
NMR(CDCl₃): δ 0.96 (t, 3H), 2.22 (s, 3H), 3.68 (s, 2H).

### Example 14(102):

3-[{3-[({[(1E)-1-biphenyl-4-ylpropylidene]amino}oxy)methyl]phenyl}(methyl)amino]propanoic acid
TLC:Rf 0.41 (chloroform: methanol = 9 : 1);
NMR(CDCl₃): δ 1.18 (t, 3H), 2.95 (s, 3H), 5.22 (s, 2H).

### Example 14(103):

{3-[2-({[(1Z)-1-biphenyl-4-yl-2-methoxyethylidene]amino}oxy)ethoxy]phenyl}acetic acid
TLC:Rf 0.41 (chloroform : methanol = 9 : 1);
NMR(CDCl₃): δ 3.32 (s, 3H), 3.61 (s, 2H), 4.65 (s, 2H).

### Example 14(104):

{3-[2-({[(1E)-1-biphenyl-4-yl-2-methoxyethylidene]amino}oxy)ethoxy]phenyl}acetic acid
TLC:Rf 0.34 (chloroform : methanol = 9 : 1);
NMR(CDCl₃): δ 3.37 (s, 3H), 3.60 (s, 2H), 4.35 (s, 2H).

### Example 14(105):

{1-[2-({[(1E)-1-biphenyl-4-ylpropylidene]amino}oxy)ethyl]-1H-indol-4-yl}acetic acid
TLC:Rf 0.42 (chloroform : methanol = 9 : 1);
NMR(CDCl₃): δ 1.05 (t, 3H), 3.92 (s, 2H), 4.49 (s, 4H).

### Example 14(106):

sodium {3-[[2-({[(1E)-1-biphenyl-4-ylpropylidene]amino}oxy)ethyl](methyl)amino]phenyl}acetate
TLC:Rf 0.61 (methanol : methylene chloride = 1 : 9);
NMR(DMSO-d₆): δ 3.15 (s, 2H), 2.91 (s, 3H), 1.03 (t, 3H).

### Example 14(107):

{1-[2-({[(1E)-1-biphenyl-4-ylpropylidene]amino}oxy)ethyl]-2,3-dihydro-1H-indol-4-yl}acetic acid
TLC:Rf 0.13 (chloroform : methanol = 9 : 1);
NMR(CDCl₃): δ 1.14 (t, 3H), 3.54 (s, 2H).

### Example 14(108):

{3-[2-({[(1E)-1-biphenyl-4-ylbutylidene]amino}oxy)ethoxy]-2-fluorophenyl}acetic acid
TLC:Rf 0.40 (chloroform : methanol = 9 : 1);
NMR(CDCl₃): δ 0.95 (t, 3H), 3.71 (s, 2H).

### Example 14(109):

{3 - [2-({[(1E)-1-biphenyl-4-ylbutylidene]amino}oxy)ethoxy]-5-methoxyphenyl}acetic acid
TLC:Rf 0.53 (chloroform : methanol = 9 : 1);
NMR(CDCl₃): δ 0.96 (t, 3H), 3.57 (s, 2H), 3.76 (s, 3H).

### Example 14(110):

{3-[2-({[(1E)-1-biphenyl-4-ylpropylidene]amino}oxy)ethyl]phenoxy}acetic acid
TLC:Rf 0.47 (chloroform : methanol = 9 : 1);
NMR(CDCl₃): δ 1.12 (t, 3H), 4.67 (s, 2H).

### Example 14(111):

3-{3-[({[(1E)-1-biphenyl-4-ylpropylidene]amino}oxy)methyl]phenoxy}propanoic acid
TLC:Rf 0.54 (methanol : methylene chloride = 1 : 9);
NMR(DMSO-d₆): δ 5.17 (s, 2H), 1.08 (t, 3H).

### Example 14(112):

{3-[2-({[(1E)-1-biphenyl-4-ylethylidene]amino}oxy)ethoxy]phenyl}acetic acid
TLC:Rf 0.57 (chloroform : methanol = 9 : 1);
NMR(DMSO-d₆): δ 2.21 (s, 3H), 3.51 (s, 2H).

### Example 14(113):

{3-[2-({[(1E)-1-(2'-fluorobiphenyl-4-yl)propylidene]amino}oxy)ethoxy]phenyl} acetic acid
TLC:Rf 0.52 (methanol: methylene chloride = 1 : 9);
NMR(DMSO-d₆): δ 3.52 (s, 2H), 1.06 (t, 3H).

### Example 14(114):

{3-[2-({[(1E)-1-(3'-fluorobiphenyl-4-yl)propylidene]amino }oxy)ethoxy]phenyl} acetic acid
TLC:Rf 0.51 (methanol : methylene chloride = 1 : 9);
NMR(DMSO-d₆): δ 3.52 (s, 2H), 1.05 (t, 3H).

### Example 14(115):

{3-[2-({[(1E)-1-(2'-methoxybiphenyl-4-yl)propylidene]amino}oxy)ethoxy]pheny}acetic acid
TLC:Rf 0.49 (methanol : methylene chloride = 1 : 9);
NMR(DMSO-d₆): δ 3.76 (s, 3H), 3.52 (s, 2H), 1.06 (t, 3R).

### Example 14(116):

sodium {3-(2-({[(1E)-1-(2'-chlorobiphenyl-4-yl)propylidene]amino}oxy)ethoxy]phenyl}acetate
TLC:Rf 0.50 (methanol : methylene chloride = 1 : 9);
NMR(DMSO-d₆): δ 3.19 (s, 2H), 1.07 (t, 3H).

### Example 14(117):

{3-[2-({[(1E)-1-(3'-chlorobiphenyl-4-yl)propylidene]amino}oxy)ethoxy]phenyl}acetic acid
TLC:Rf 0.49 (methanol : methylene chloride = 1 : 9);
NMR(DMSO-d₆): δ 3.52 (s, 2H), 1.05 (t, 3H).

### Example 14(118):

({3-[({[(1E)-1-biphenyl-4-ylpropylidene]amino}oxy)methyl]benzyl}oxy)acetic acid
TLC:Rf 0.45 (methanol : methylene chloride = 1 : 9);
NMR(DMSO-d₆) : δ 5.21 (s, 2H), 4.55 (s, 2H), 1.07 (t, 3H).

### Example 14(119):

{3-[2-({[(1E)-1-(2'-methylbiphenyl-4-yl)propylidene]amino}oxy}ethoxy]phenyl}acetic acid
TLC:Rf 0.53 (methanol : methylene chloride = 1 : 9);
NMR(DMSO-d₆): δ 3.52 (s, 2H), 2.23 (s, 3H), 1.07 (t, 3H).

### Example 14(120):

{3-[2-({[(1E)-1-(3'-methylbiphenyl-4-yl)propylidene]amino}oxy)ethoxy]phenyl}acetic acid
TLC:Rf 0.53 (methanol: methylene chloride = 1 : 9);
NMR(DMSO-d₆): δ 3.52 (s, 2H), 2.37 (s, 3H), 1.05 (t, 3H).

### Example 14(121):

{3-[2-({[(1E)-1-biphenyl-4-ylpentylidene]amino}oxy)ethoxy]phenyl}acetic acid
TLC:Rf 0.60 (chloroform : methanol = 9 : 1);
NMR(CDCl₃): δ 0.89 (t, 3H), 3.62 (s, 2H).

### Example 14(122):

{3-[2-({[(1E)-1-biphenyl-4-ylpentylidene]amino}oxy)ethoxy]-2-methylphenyl}acetic acid
TLC:Rf 0.61 (chloroform: methanol = 9 : 1);
NMR(CDCl₃): δ 0.80 (t, 3H), 2.14 (s, 3H), 3.59 (s, 2H).

### Example 14(123):

2-{3-[2-({[(1E)-1-biphenyl-4-ylpropylidene]amino}oxy)ethoxy]phenyl}-2-methylpropanoic acid
TLC:Rf 0.56 (chloroform : methanol = 9 : 1);
NMR(DMSO-d₆): δ 1.05 (t, 3H), 1.44 (s, 6H).

### Example 14(124):

(4-{[2-({[(1E)-1-biphenyl-4-ylpropylidene]amino}oxy)ethyl]thio}-2-methylphenoxy)acetic acid
TLC:Rf 0.3 (chloroform : methanol = 9 : 1);
NMR(CDCl₃): δ 1.15 (t, 3H), 2.25 (s, 3H), 4.65 (s, 2H).

### Example 14(125):

(3-{[2-({[(1E)-1-biphenyl-4-ylpropylidene]amino}oxy)ethyl]thio}phenyl)acetic acid
TLC:Rf 0.41 (chloroform : methanol = 9 : 1);
NMR(CDCl₃): δ 1.15 (t, 3H), 3.60 (s, 2H).

### Example 14(126):

{3-[2-({[(1E)-1-biphenyl-4-ylpropylidene]amino}oxy)ethyl]phenyl}acetic acid
TLC:Rf 0.28 (chloroform : methanol = 9 : 1);
NMR(CDCl₃): δ 1.11 (t, 3H), 3.63 (s, 2H), 4.40 (t, 2H).

### Example 14(127):

sodium {3-[2-({[(1E)-1-(4-cycloheptylphenyl)propylidene]amino}oxy)ethoxy]phenyl}acetate
TLC:Rf 0.57 (methylene chloride : methanol = 9 : 1);
NMR(DMSO-d₆): δ 3.17 (s, 2H), 1.86-1.40 (m, 12H), 1.02 (t, 3H).

### Example 14(128):

1-{3-[2-({[(1E)-1-biphenyl-4-ylpropylidene]amino}oxy)ethoxy]phenyl}cyclopropanecarboxylic acid
TLC:Rf 0.77 (chloroform : methanol = 9 : 1);
NMR(CDCl₃). δ 1.25 (dd, 2H), 1.63 (dd, 2H), 4.26 (t, 2H).

### Example 14(129):

5-[2-({[(1E)-1-biphenyl-4-ylpropylidene]amino}oxy)ethoxy]-1,2,3,4-tetrahydronaphthalene-1-carboxylic acid
TLC:Rf 0.71 (chloroform : methanol = 9 : 1);
NMR(CDCl₃): δ 1.16 (t, 3H), 3.83 (t, 1H).

### Example 14(130):

{3-[2-({[(1E)-1-biphenyl-4-yl-3-methylbutylidene]amino } oxy)ethoxy]-2-methylphenyl} acetic acid
TLC:RF 0.34 (methylene chloride : methanol = 10 : 1);
NMH(CDCl₃): δ 0.92 (d, 6H), 2.22 (s, 3H), 2.72 (d, 2H).

### Example 14(131):

{3-[2-({[(1E)-1-biphenyl-4-yl-3-methylbutylidene]amino }oxy)ethoxy]phenyl} acetic acid
TLC:Rf 0.42 (methylene chloride : methanol = 10 : 1);
NMR(CDCl₃): δ 0.92 (d, 6H), 2.72 (d, 2H), 7.70 (d, 2H).

### Example 14(132):

{3-[2-({[(1E)-1-biphenyl-4-yl-3-methylbutylidene]amino }oxy)ethoxy]-2-fluorophenyl}acetic acid
TLC:Rf 0.36 (methylene chloride : methanol = 10 : 1);
NMR(CDCl₃): δ 0.91 (d, 6H), 2.71 (d, 2H), 7.70 (d, 2H).

### Example 14(133):

{3-[2-({[(1E)-1-biphenyl-4-yl-4-methylpentylidene]amino}oxy)ethoxy]phenyl}acetic acid
TLC:Rf 0.49 (methylene chloride : methanol = 10 : 1);
NMR(CDCl₃): δ 0.91 (d, 6H), 2.77 (m, 2H), 7.70 (d, 2H).

### Example 14(134):

{3-[2-({[(1E)-1-biphenyl-4-yl-4-methylpentylidene]amino }oxy)ethoxy]-2-methylphenyl} acetic acid
TLC:Rf 0.52 (methylene chloride : methanol = 10 : 1);
NMR(CDCl₃): δ 0.91 (d, 6H), 2.22 (s, 3H).

### Example 14(135):

{3-[2-({[(1E)-1-biphenyl-4-yl-4-methylpentylidene]amino}oxy)ethoxy]-2-fluorophenyl} acetic acid
TLC:Rf 0.44 (methylene chloride : methanol = 10 : 1);
NMR(CDCl₃): δ 0.91 (d, 6H), 1.61 (m, 1H), 7.70 (d, 2H).

### Example 14(136):

{3-[2-({[(1Z)-1-biphenyl-4-yl-2-piperidin-1-ylethylidene]amino }oxy)ethoxy]phenyl}acetic acid
TLC:Rf 0.2 (chloroform : methanol = 9 : 1);
NMR(CDCl₃): δ 3.90 (s, 2H), 7.78 (d, 2H), 9.97 (bs, 1H).

### Example 14(137):

{3-[2-({[(1Z)-1-biphenyl-4-yl-2-morpholin-4-ylethylidene]amino}oxy)ethoxy]phenyl}acetic acid
TLC:Rf 0.45 (chloroform : methanol = 9 : 1);
NNM(CDCl₃): δ 3.56-3.62 (m, 4H), 3.71 (s, 2H), 7.85 (d, 2H).

### Example 14(138):

{3-[2-({[(1E)-1-biphenyl-4-yl-2-pyridin-2-ylethylidene]amino}oxy)ethoxy]phenyl}acetic acid
TLC:RF 0.30 (chloroform : methanol = 9 : 1);
NMR(CDCl₃): δ 3.62 (s, 2H), 7.76 (d, 2H), 8.50 (dd, 1H).

### Example 14(139):

{3-[2-({[(1E)-1-biphenyl-4-yl-2-pyridin-4-ylethylidene]amino}oxy)ethoxy]phenyl} acetic acid
TLC:Rf 0.23 (chloroform : methanol = 9 : 1);
NMR(CDCl₃): δ 3.61 (s, 2H), 8.31 (d, 2H), 9.62 (bs, 1H).

### Example 14(140):

{3-[2-({[(1E)-1-biphenyl-4-yl-2-(2-thienyl)ethylidene]amino }oxy)ethoxy]phenyl }acetic acid
TLC:Rf 0.50 (methylene chloride : methanol = 19 : 1);
NMR(DMSO-d₆): δ 3.53 (s, 2H), 12.33 (s, 1H).

### Example 14 (141) :

{3-[2-({[(1E)-1-biphenyl-4-yl-2-(2-thienyl)ethylidene]amino}oxy)ethoxy]-2-fluorophenyl} acetic acid
TLC:Rf 0.41 (methylene chloride : methanol = 19 : 1);
NMR(DMSO-d₆): δ 4.32 (s, 2H), 7.24 (dd, 1H), 12.47 (s, 1H).

### Example 14(142):

{3-[2-({[(1E)-1-biphenyl-4-yl-2-(2-thienyl)ethylidene]amino}oxy)ethoxy]-2-methylphenyl}acetic acid
TLC:Rf 0.50 (methylene chloride : methanol = 19 : 1);
NMR(DMSO-d₆): δ 2.09 (s, 3H), 4.34 (s, 2H), 12.29 (s, 1H).

### Example 14(143):

{3-[2-({[(1Z)-1-biphenyl-4-yl-2-thiomorpholin-4-ylethylidene]amino}oxy)ethoxy]phenyl}acetic acid
TLC:Rf 0.52 (chloroform : methanol = 9 : 1);
NMR(DMSO-d₆): δ 2.38-2.46 (m, 4H), 3.67 (s, 2H), 12.30 (bs, 1H).

### Example 14(144):

{3-[2-({[(1Z)-4-biphenyl-4-yl-2-(4-methylpiperazin-1-yl)ethylidene]amino} oxy)ethoxy]phenyl}acetic acid
TLC:Rf 0.28 (chloroform : methanol : 28% ammonium water = 40 : 9 : 1)
NMR(DMSO-d₆): δ 2.12 (s, 3H), 2.32-2.47 (m, 4H), 7.83 (d, 2H).

### Example 14(145):

{3-[2-({[(1E)-6-phenyl-3,4-dihydronaphthalen-1(2H)-ylidene]amino}oxy)ethoxy]phenyl }acetic acid
TLC:Rf 0.40 (methylene chloride : methanol = 19 : 1);
NMR(DMSO-d₆): δ 2.68 (t, 2H), 2.73-2.85 (m, 2H), 12.30 (s, 1H).

### Example 14(146):

{2-fluoro-3 -[2-({[(1E)-6-phenyl-3 ,4-dihydronaphthalen-1 (2H)-ylidene]amino}oxy)ethoxy]phenyl}acetic acid
TLC:Rf 0.33 (methylene chloride : methanol = 19 : 1);
NMR(DMSO-d₆): δ 2.66 (t, 2H), 2.77 (t, 2H), 12.45 (s, 1H).

### Example 14(147):

{2-methyl-3-[2-({[(1E)-6-phenyl-3,4-dihydronaphthalen-1(2H)-ylidene]amino}oxy)ethoxy]phenyl}acetic acid
TLC:Rf 0.35 (methylene chloride : methanol = 19 : 1);
NMR(DMSO-d₆): δ 2.08 (s, 3H), 2.69 (t, 2H), 12.27 (s, 1H).

### Example 14(148):

{3-[2-({[(1Z)-1-biphenyl-4-yl-2-(1H-pyrazol-1-yl)ethylidene]amino}oxy)ethoxy]-2-methylphenyl}acetic acid
TLC:Rf 0.56 (chloroform : methanol = 9 : 1);
NMR(CDCl₃): δ 2.22 (s, 3H), 5.43 (s, 2H), 6.85 (d, 1H), 7.11 (t, 1H).

### Example 14(149):

{3-[2-({[(1Z)-1-biphenyl-4-yl-2-(1H-imidazol-1-yl)ethylidene]amino}oxy)ethoxy]-2-methylphenyl}acetic acid
TLC:Rf 0.27 (chloroform : methanol = 9 : 1);
NMR(DMSO-d₆): δ 2.11 (s, 3H), 5.36 (s, 2H), 7.08 (s, 1H).

### Example 14(150):

{3-[2-({[(1E)-1-biphenyl-4-yl-2-cyclobutylethylidene]amino}oxy)ethoxy]-2-methylphenyl}acetic acid
TLC:Rf 0.40 (methylene chloride : methanol = 10 : 1);
NMR(CDCl₃): δ 1.67-1.83 (m, 4H), 2.23 (s, 3H), 2.92 (d, 2H).

### Example 14(151):

{3-[2-({[(1E)-1-biphenyl-4-ylpent-4-en-1-ylidene]amino}oxy)ethoxy]-2-methylphenyl}acetic acid
TLC:Rf 0.40 (methylene chloride : methanol = 10 : 1);
NMR(CDCl₃): δ 2.22 (s, 3H), 3.67 (s, 2H), 7.70 (d, 2H),

### Example 14(152):

{3-[2-({[(1Z)-1-biphenyl-4-yl-2-(1H-1,2,4-triazol-1-yl)ethylidene]amino }oxy)ethoxy]-2-methylphenyl} acetic acid
TLC:Rf 0.42 (chloroform : methanol = 4 : 1);
NMR(DMSO-d₆): δ 2.09 (s, 3H), 7.88 (s, 1H), 8.54 (s, 1H).

### Example 14(153):

{3-[2-({[(1Z)-1-biphenyl-4-yl-2-(4,5-dihydro-1H-pyrazol-1-yl)ethylidene]amino}oxy)ethoxy]-2-methylphenyl}acetic acid
TLC:Rf 0.54 (chloroform : methanol = 9 : 1);
NMR(CDCl₃): δ 2.20 (s, 3H), 2.54 (td, 2H), 3.00 (t, 2H).

### Example 14(154):

{3-[2-({[(1E)-1-biphenyl-4-yl-2-cyclopentylethylidene]amino}oxy)ethoxy]-2-methylphenyl}acetic acid
TLC:Rf 0.37 (methylene chloride : methanol = 19 : 1);
NMR(DMSO-d₆): δ 2.09 (s, 3H), 2.82 (d, 2H), 7.75 (d, 2H).

### Example 14(155):

{3-[2-({[(1E)-1-biphenyl-4-yl-2-(3-thienyl)ethylidene]amino}oxy)ethoxy]-2-methylphenyl}acetic acid
TLC:RF 0.38 (methylene chloride : methanol = 19 : 1);
NMR(DMSO-d₆): δ 4.15 (s, 2H), 7.01 (d, 1H), 12.29 (s, 1H).

### Example 14(156):

{3-[2-({[(1E)-1-biphenyl-4-yl-2-cyclobutylethylidene]amino}oxy)ethoxy]phenyl}acetic acid
TLC:Rf 0.45 (methylene chloride : methanol = 10 : 1);
NMR(CDCl₃): δ 2.92 (d, 2H), 3.61 (s, 2H), 7.67 (d, 2H).

### Example 14(157):

{3-[2-({[(1E)-1-biphenyl-4-yl-2-cyclobutylethylidene]amino}oxy)ethoxy]-2-fluorophenyl acetic acid
TLC:Rf 0.43 (methylene chloride : methanol = 10 : 1);
NMR(CDCl₃): δ 2.91 (d, 2H), 3.71 (d, 2H), 7.67 (d, 2H).

### Example 14(158):

{3-[2-({[(1E)-1-biphenyl-4-yl-2-pyridin-2-ylethylidene]amino}oxy)ethoxy]-2-methylphenyl}acetic acid
TLC:Rf 0.31 (chloroform : methanol = 9 : 1);
NMR(CDCl₃): δ 2.17 (s, 3H), 4.32 (s, 2H), 7.04 (ddd, 1H).

### Example 14(159):

{3-[2-({[(1E)-1-biphenyl-4-yl-2-pyridin-4-ylethylidene]amino} oxy)ethoxy]-2-methylphenyl}acetic acid
TLC:Rf 0.25 (chloroform : methanol = 9 : 1);
NMR(DMSO-d₆). δ 2.03 (s, 3H), 4.23 (s, 2H), 8.32 (d, 2H).

### Example 14(160):

{3-[2-({[(1Z}-1-biphenyl-4-yl-2-(dimethylamino)ethylidene]amino}oxy)ethoxy]-2-methylphenyl}acetic acid
TLC:Rf 0.36 (chloroform: methanol = 9 : 1);
NMR(CDCl₃): δ 2.30 (s, 6H), 3.56 (s, 2H), 3.84 (s, 2H).

### Example 14(161):

{3-[2-({[(1Z)-1-biphenyl-4-yl-2-(1,3-thiazolidin-3-yl)ethylidene]amino}oxy)ethoxy]-2-methylphenyl}acetic acid
TLC:Rf 0.54 (methylene chloride : methanol = 9 : 1);
NMR(DMSO-d₆): δ 2.81 (t, 2H), 2.92 (t, 2H).

### Example 14(162):

(2-methyl-3-{2-[({(1E)-1-(4-pyridin-2-ylphenyl)butylidene}amino)oxy]ethoxy}phenyl)acetic acid
TLC:Rf 0.55 (methylene chloride : methanol = 9 : 1);
NMR(DMSO-d₆): δ 8.11(d, 2H), 7.35(ddd, 1H), 1.50(sixtet, 2H).

### Example 14(163).

(2-methyl-3-{2-[({(1E)-1-[4-(1H-pyrazol-1-yl)phenyl] butylidene} amino)oxy]ethoxy}phenyl)acetic acid
TLC:Rf 0.53 (methylene chloride : methanol = 9 : 1);
NMR(DMSO-d₆): δ 8.53(d, 1H), 4.47(t, 2H), 0.86(t, 3H).

### Example 14(164):

{3-[2-({[(1Z)-1-biphenyl-4-yl-2-(2,5-dihydro-1H-pyrrol-1-yl)ethylidene]amino}oxy)ethoxy]-2-methylphenyl}acetic acid
TLC:Rf 0.43 (methylene chloride : methanol = 9 : 1);
NMR(DMSO-d₆): δ 2.07 (s, 3H), 3.40 (s, 4H), 5.67 (s, 2H).

### Example 14(165):

[3-(2-{[((1E)-1-biphenyl-4-yl-4,4,4-trifluorobutylidene)amino]oxy}ethoxy)-2-methylphenyl]acetic acid
TLC:Rf 0.47 (methylene chloride : methanol = 10 : 1);
NMR(CDCl₃): δ 3.67 (s, 2H), 6.83 (d, 1H), 7.70 (d, 2H).

### Example 14(166):

{2-methyl-3-[2-({(1E)-1-(4-pyridin-2-ylphenyl)pentylidene]amino}oxy)ethoxy]phenyl}acetic acid
TLC:Rf 0.50 (chloroform : methanol = 6 : 1);
NMR(CDCl₃): δ 0.88 (t, 3H), 3.66 (s, 2H), 6.83 (dd, 2H).

### Example 14(167):

(2-methyl-3-{2-[({(1E)-3-methyl-1-[4-(1H-pyrazol-1-yl)phenyl]butylidene}amino)oxy]ethoxy}phenyl)acetic acid
TLC:Rf 0.42 (chloroform : methanol = 6 : 1);
NMR(CDCl₃): δ 0.90 (d, 6H), 2.20 (s, 3H), 7.93 (d, 1H).

### Example 14(168):

[3-(2-{[((1Z)-1-biphenyl-4-yl-2-piperidin-1-ylethylidene)amino]oxy}ethoxy)-2-methylphenyl]acetic acid
TLC:Rf 0.29 (chloroform: methanol : 28% ammonium water = 40 : 9 : 1);
NMR(DMSO-d₆): δ 1.10-1.47 (m, 6H), 3.55 (s, 2H), 7.84 (d, 2H).

### Example 14(169):

[3-(2-{[((1Z)-1-biphenyl-4-yl-2-piperidin-1-ylethylidene)amino]oxy}ethoxy)-2-fluorophenyl]acetic acid
TLC:Rf 0.21 (chloroform : methanol: 28% ammonium water = 40 : 9 : 1);
NMR(DMSO-d₆): δ 1.15-1.48 (m, 6H), 7.60-7.75 (m, 4H), 7.84 (d, 2H).

### Example 14(170):

{3-[2-({[(1Z)-1-biphenyl-4-yl-2-(3,6-dihydropyridin-1(2H)-yl)ethylidene]amino}oxy)ethoxy]-2-methylphenyl}acetic acid
TLC:Rf 0.33 (methylene chloride : methanol = 9 : 1);
NMR(DMSO-d₆): δ 2.08 (s, 3H), 5.49 (d, 1H), 5.59 (d, 1H).

### Example 14(171):

{3-[2-({[(1Z)-1-biphenyl-4-yl-2-(3,6-dihydropyridin-1(2H)-yl)ethylidene]amino}oxy)ethoxy]-2-fluorophenyl} acetic acid
TLC:Rf 0.27 (methylene chloride : methanol = 9 : 1);
NMR(DMSO-d₆): δ 5.49 (d, 1H), 5.58 (d, 1H), 7.82 (d, 2H).

### Example 14(172):

{3-[2-({[(1Z)-1-biphenyl-4-yl-2-(3,6-dihydropyridin-1(2H)-yl)ethylidene]amino}oxy)ethoxy]phenyl}acetic acid
TLC:Rf 0.25 (methylene chloride : methanol = 9 : 1);
NMR(DMSO-d₆): δ 5.56 (d, 1H), 5.65 (d, 1H), 7.88 (d, 2H).

### Example 14(173):

{2-methyl-3-[2-({(1E)-3-methyl-1-(4-pyridin-2-ylphenyl)butylidene]amino}oxy)ethoxy]phenyl}acetic acid
TLC:Rf 0.53 (chloroform : methanol = 6 : 1);
NMR(CDCl₃): δ 0.91 (d, 6H), 2.72 (d, 2H), 3.66 (s, 2H).

### Example 14(174):

{3-[2-({[(1Z)-1-biphenyl-4-yl-2-(3,6-dihydropyridin-1 (2H)-yl)ethylidene]amino}oxy)ethoxy]-2-methylphenyl}acetic acid
TLC:Rf 0.33 (methylene chloride : methanol = 9 : 1);
NMR(DMSO-d₆): δ 2.91 (s, 2H), 5.49 (d, 1H), 5.59 (d, 1H), 7.84 (d, 2H).

### Example 14(175):

{3-[2-({[(1Z)-1-biphenyl-4-yl-2-(3,6-dihydropyridin-1(2H)-yl)ethylidene]amino}oxy)ethoxy]-2-fluorophenyl} acetic acid
TLC:Rf 0.27 (methylene chloride : methanol = 9 : 1);
NNM(DMSO-d₆): δ 2.89 (s, 2H), 5.49 (d, 1H), 5.58 (d, 1H), 7.82 (d, 2H).

### Example 14(176):

{3 -[2-({[(1Z)-1-biphenyl-4-yl-2-(3,6-dihydropyridin-1(2H)-yl)ethylidene]amino}oxy)ethoxy]phenyl}acetic acid
TLC:Rf 0.25 (methylene chloride : methanol = 9 : 1);
NMR(DMSO-d₆): δ 2.96 (s, 2H), 5.56 (d, 1H), 5.65 (d, 1H), 7.88 (d, 2H).

### Example 14(177):

[2-methyl-3-(2-{[((1E)-5-phenyl-2,3-dihydro-1H-inden-1-ylidene)amino]oxy}ethoxy)phenyl]acetic acid
TLC:Rf 0.40 (chloroform : methanol = 9 : 1);
NMR(CDCl₃): δ 2.89-2.96 (m, 2H), 3.03-3.10 (m, 2H), 6.83 (d, 1H).

### Example 14(178):

[2-methyl-3-{2-{[((1E)-6-pyridin-2-yl-3,4-dihydronaphthalen-1(2H)-ylidene)amino]oxy}ethoxy)phenyl]acetic acid
TLC:Rf 0.47 (chloroform : methanol = 6 : 1);
NMR(DMSO-d₆): δ 2.69 (t, 2H), 2.80 (t, 2H), 7.35 (ddd, 1H).

### Example 14(179):

{2-methyl-3-[2-({[(1E)-3-methyl-1-(4-pyridin-2-ylphenyl)butylidene]amino}oxy)ethoxy]phenyl}acetic acid
TLC:Rf 0.53 (chloroform : methanol = 9 : 1);
NMR(CDCl₃): δ 0.91 (d, 6H), 2.72 (d, 2H), 6.83 (dd, 2H).

### Example 14(180):

{2-methyl-3-[2-({[(1E)-6-(1H-pyrazol-yl)-3,4-dihydronaphthaten-1(2H)-ylidene]amino}oxy)ethoxy]phenyl}acetic acid
TLC:Rf 0.53 (chloroform : methanol = 9 : 1);
NMR(CDCl₃) : δ 2.71-2.82 (m, 4H), 6.47 (dd, 1H), 7.93 (d, 1H), 8.04 (d, 1H).

### Example 14(181):

(3-{2-[({(1Z)-2-(dimethylamino)-1-[4-(1H-pyrazol-1-yl)phenyl]ethylidene}amino)oxy]ethoxy}-2-methylphenyl)acetic acid
TLC:Rf 0.32 (chloroform: methanol = 4 : 1);
NMR(CDCl₃): δ 2.30 (s, 6H), 3.58 (s, 2H), 3.80 (s, 2H), 6.48 (dd, 1H), 7.79 (d, 2H), 7.93 (dd,1H).

### Example 14(182):

{2-methyl-3-[2-({ [(1Z)-1-[4-(1H-pyrazol-1-yl)phenyl]-2-(1,3-thiazolidin-3-yl)ethylidene]amino}oxy)ethoxy]phenyl}acetic acid
TLC:Rf 0.52 (chloroform : methanol = 9 : 1);
NMR(CDCl₃): δ 3.66 (s, 2H), 3.73 (s, 2H), 4.08 (s, 2H), 6.48 (dd, 1H), 7.94 (dd, 1H).

### Example 14(183):

{3-[2-({[(1Z)-2-(dimethylamino)-1-(4-pyridin-2-ylphenyl)ethylidene]amino}oxy)ethoxy]-2-methylphenyl}acetic acid
TLC:Rf 0.10 (chloroform : methanol = 6 : 1);
NMR(CDCl₃): δ 3.57 (s, 2H), 3.83 (s, 2H), 7.05 (t, 1H), 8.71 (d, 1H).

### Example 14(184):

{2-methyl-3-[2-({[(1Z)-1-(4-pyridin-2-ylphenyl)-2-(1,3-thiazolidin-3-yl)ethylidene]amino}oxy)ethoxy]phenyl}acetic acid
TLC:Rf 0.46 (chloroform : methanol = 6 : 1);
NMR(DMSO-d₆): δ 3.28 (s, 2H), 3.75 (s, 2H), 4.04 (s, 2H), 6.77 (d, 2H), 8.11 (d, 2H).

### Reference Example 7:

### 2- {[(1-biphenyl-4-ylpropylidene)amino]oxy}ethyl 4-methylbenzenesulfonate

A solution of 1-biphenyl-4-ylpropan-1-one O-(2-hydroxyethyl)oxime (5.0g) and 2-(2-bromoethoxy)tetrahydro-2H-pyran (5.56g) in anhydrous DMF (50mL) was dropped by a solution of potassium-t-butoxide in 1M THF (26.6mL) at a room temperature and stirred for 10 minutes at same temperature. The reaction solution was diluted with water and extracted by ethyl acetate. The organic layer was washed with water and concentrated. The residue was dissolved with methanol (20mL), added by hydrogen chloride in 10% methanol solution (20mL) and the mixture was stirred for 20 minutes and concentrated. The residue was dissolved with pyridine (25mL), added by p-toluenesulfonylchloride (5.23g) at a room temperature and stirred for 3 hours at same temperature. The reaction solution was diluted with water, extracted by ethyl acetate and the organic layer was washed with hydrochloric acid and water successively, and concentrated. The residue was recrystrallized from ethanol/water to give the title compounds (6.37g) having the following physical data.
TLC:Rf 0.50 (n-hexane : ethyl acetate = 2 : 1).

### Example 15:

### methyl 2-{3-[2-({[(1E)-1-biphenyl-4-ylpropylidene]amino}oxy)ethoxy]phenyl}propanoic acid

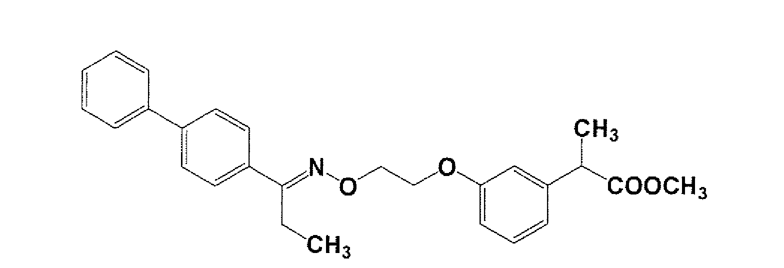

### Process 1:

A solution of sodium hydroxide (1.61g; 60% in oil) in benzene (35mL) was slowly added by dimethylcarbonate (4.8mL) and under atmosphere of argon the mixture was stirred for an hour at 90°C. The mixture was slowly added by a solution of 3-methoxymethoxyphenylacetic acid (2.1g) in benzene (6mL) and under atmosphere of argon the mixture was stirred for an hour at 90°C, and additionally for 2 hours at 100°C. The mixture was cooled down till room temperature, poured into iced water and extracted by ethyl acetate. The organic layer was washed with saturated sodium hydrogen carbonate aqueous solution and saturated brine successively, dried over and then concentrated to give the rough purification compounds of dimethyl [3-(methoxymethoxy)phenyl]malonate.

### Process 2:

Under atmosphere of argon, a solution of rough purification compounds of dimethyl [3-(methoxymethoxy)phenyl]malonate obtained in Process 1 in N,N-dimethylformamide (20mL) was added by sodium hydroxide (800mg) and methyl iodide (1.25mL) and the mixture was stirred for 18 hours at a room temperature. The mixture was poured into iced water and extracted by ethyl acetate. The organic layer was washed with water and saturated brine successively, dried over and then concentrated to give the rough purification compounds of dimethyl [3-(methoxymethoxy)phenyl](methyl)malonate,

### Process 3:

A solution of rough purification compounds of dimethyl [3-(methoxymethoxy)phenyl](methyl)malonate obtained in Process 2 in methanol (10mL)/tetrahydrofuran (10mL) was added by 5N sodium hydroxide aqueous solution (4mL) and the mixture was stirred for 2 hours at 70°C. The mixture was neutralized by 2N hydrochloric acid (10.0mL) at a room temperature and extracted by ethyl acetate. The organic layer was washed with water and saturated brine successively, dried over and then concentrated to give 2-[3-(methoxymethoxy)phenyl]propanoic acid.

### Process 4:

Under atmosphere of argon, a methanol solution (30mL) was dropped by thionylchloride (1.1mL) at -78°C, stirred for 10 minutes at -78°C and stirred for 10 minutes at 0°C. The mixture was added by a solution of 2-[3-(methoxymethoxy)phenyl]propanoic acid in methanol (20mL) at 0°C, slowly risen until room temperature and stirred for 2 hours. The mixture was toluene azetrope, added by water and extracted by ethyl acetate. The organic layer was washed with saturated brine, dried over and then concentrated. The residue was purified by column to give methyl 2-(3-hydroxyphenyl)propanoate (400mg) having the following physical data.

### Process 5:

By the same procedure as described in Example 1 using the compounds obtained in the above-mentioned Process 4 instead of the compounds prepared in Reference Example 1 and the compound prepared in Reference Example 7 instead of the compounds prepared in Reference Example 3, the compounds of the present invention having the following physical data were obtained.
TLC:Rf 0.68 (n-hexane : ethyl acetate = 1 : 1).

### Example 16:

### 2-{3-[2-({[(1E)-1-biphenyl-4-ylpropylidene]amino}oxy)ethoxy]phenyl}propanoic acid

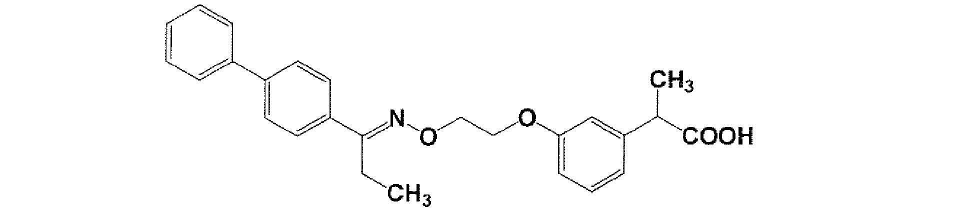

By the same procedure as described in Example 2 using the compound prepared in Example 15 instead of the compounds prepared in Example 1, the compounds of the present invention having the following physical data were obtained.
TLC:Rf 0.52 (chloroform : methanol = 9 : 1);
NMR(CDCl₃): δ 1.15 (t, 3H), 1.49 (d, 3H), 2.79 (q, 2H), 3.70 (q, 1H), 4.27 (t, 2H), 4.53 (t, 2H), 6.84-6.92 (m, 3H), 7.21-7.26 (m, 1H), 7.32-7.38 (m, 1H), 7.42-7.47 (m, 2H), 7.59-7.62 (m, 4H), 7.70-7.73 (m, 2H).

### Example 16(1)-Example 16(3)

By the same procedure as described in Process 5 in Example 15 and Example 2 using methyl 2-(3-hydroxyphenyl)propanoate or the corresponding compounds instead thereof and the compounds prepared in Reference Example 7 or the corresponding compounds instead thereof, the following compounds in the present invention were obtained. Also, NMR data of the following compounds of the present invention was described as the characteristic peak.

### Example 16(1):

{3-[2-({[(1Z)-1-biphenyl-4-ylpropylidene]amino}oxy)ethoxy]phenyl}acetic acid
TLC:Rf 0.37 (chloroform : methanol = 9 : 1);
NNM(CDCl₃): δ 1.09 (t, 3H), 3.59 (s, 2H).

### Example 16(2):

{3-[2-({[(1E)-1-(4-cyclopenthylphenyl)propylidene]amino}oxy)ethoxy]phenyl} acetic acid
TLC:Rf 0.57 (methylene chloride : methanol = 9 : 1),
NMR(DMSO-d₆): δ 3.51 (s, 2H), 1.01 (t, 3H).

### Example 16(3):

{3-[2-({[(1E)-1-(4-cyclohexylphenyl)propylidene]amino}oxy)ethoxy]phenyl}acetic acid
TLC:Rf 0.57 (methylene chloride : methanol = 9 : 1);
NMR(DMSO-d₆): δ 3.51 (s, 2H), 1.01 (t, 3H).

### Example 17:

### diethyl {3-[2-({[(1E)-1-biphenyl-4-ylpropylidene}amino}oxy)ethoxy]phenyl}malonic acid

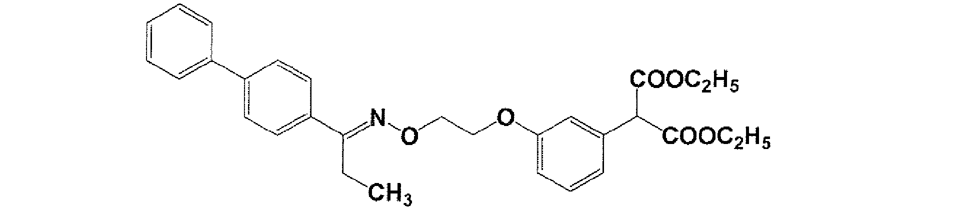

Process 1: under atmosphere of argon, a solution of the compound prepared in Example 14(17) (1.0g) in acetonitrile (10mL) was added by cesium carbonate (1.6g) and ethyl iodide (0.21mL) at a room temperature and the mixture was stirred for 3 hours at 80°C. The mixture was poured into ammonium chloride cooled aqueous solution and extracted by ethyl acetate. The organic layer was washed with water and saturated brine successively, dried over and concentrated. The residue was purified by column to give ethyl {3-[2-({[(1E)-1-biphenyl-4-ylpropylidene]amino}oxy)ethoxy]phenyl}acetic acid (982mg) having the following physical data.
   TLC:Rf 0.64 (n-hexane : ethyl acetate = 1 : 1).
Process 2: by the same procedure as described in Process 1 in Example 15 using the compounds prepared in Process 1 (200mg) instead of 3-methoxymethoxyphenylacetic acid, the compounds in the present invention (390mg) having the following physical data were obtained.
   TLC:Rf 0.59 (n-hexane : ethyl acetate = 1 : 1);
   NMR(CDCl₃): δ 1.16 (t, 3H), 1.26 (t, 6H), 2.80 (q, 2H), 4.16-426 (m, 4H), 4.29 (t, 2H) 4.54 (t, 2H), 4.57 (s, 1H), 6.91-7.02 (m, 3H), 7.24-7.30 (m, 1H), 7.32-7.39 (m, 1H), 7.42-7.48 (m, 2H), 7.59-7.62 (m, 4H), 7.70-7.73 (m, 2H).

### Example 18:

### methyl [3-(2-{[((1E)-1-{4'-[(dimethylamino)carbonyl]biphenyl-4-yl}propylidene)amino]oxy}ethoxy)phenyl]acetic acid

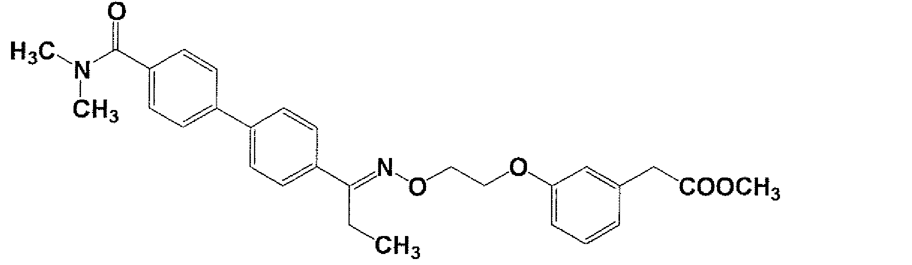

A solution of methyl {3-[2-({[(1E)-1-(4-bromophenyl)propylidene]amino}oxy)ethoxy]phenyl}acetate (635mg) in dimethoxyethane (6.0mL) was added by p-dimethylaminocarbonylbenzeneboronic acid (353mg), sodium carbonate (205mg) aqueous solution and tetrakis(triphenylphosphine)palladium(0) (89mg) successively and the mixture was stirred for an hour at 90°C. The mixture was cooled down till room temperature, added by water and filtrated. The filtrate was extracted by ethyl acetate and the organic layer was washed with water and saturated brine successively, dried over and then concentrated. The residue was purified by column to give the title compounds having the following physical data.
TLC:Rf 0.16 (n-hexane : ethyl acetate = 1 : 1).

### Example 19:

### [3-(2-{[((1E)-1-{4'-[(dimethylamino)carbonyl]biphenyl-4-yl}propylidene)amino]oxy} ethoxy)phenyl]acetic acid

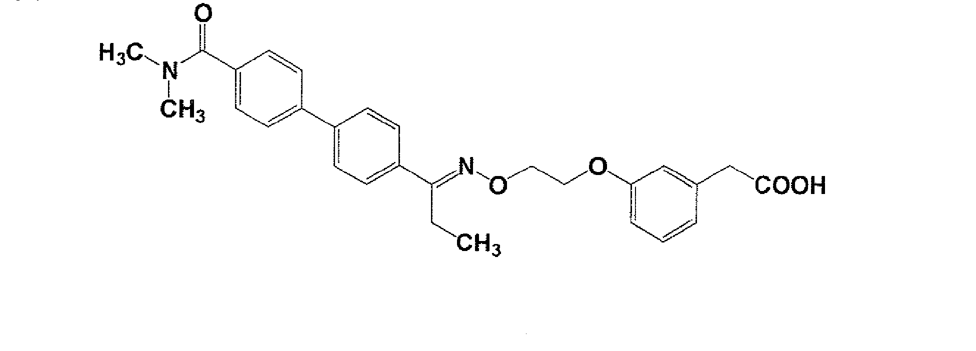

By the same procedure as described in Example 2 using the compounds prepared in Example 18 instead of the compounds prepared in Example 1, the compounds in the present invention having the following physical data were obtained.
TLC:Rf 0.46 (methanol : methylene chloride = 1 : 9);
NMR(CDCl₃): δ 7.72 (d, 2H), 7.62 (d, 2H), 7.59 (d, 2H), 7.50 (d, 2H), 7.22 (t, 1H), 6.92-6.82 (m, 3H), 4.53 (t, 2H), 4.27 (t, 2H), 3.59 (s, 2H), 3.13 (brs, 3H), 3.03 (brs, 3H).

### Example 19(1)-Example 19(2)

By the same procedure as described in Example 18 and Example 2 using the corresponding compounds instead of p-dimethylaminocarbonylbenzeneboronic acid, the following compounds in the present invention were obtained. Also, NMR data of the following compounds of the present invention was described as the characteristic peak.

### Example 19(1):

(3-{2-[({(1E)-1-[4'-(dimethylamino)biphenyl-4-yl]propylidene}amino)oxy]ethoxy}phenyl)acetic acid
TLC:Rf 0.58 (methanol : methylene chloride = 1 : 9);
NMR(DMSO-d₆): δ 4.44 (t, 2H), 3.52 (s, 2H), 1.05 (t, 3H).

### Example 19(2):

{3-[2-({[(1E)-1-(3'-methoxybiphenyl-4-yl)propylidene]amino}oxy)ethoxy]phenyl} acetic acid
TLC:Rf 0.47 (chloroform : methanol = 9 : 1);
NMR(CDCl₃): δ 1.15 (t, 3H), 3.61 (s, 2H), 3.87 (s, 3H).

### Example 20:

### (1E)-1-biphenyl-4-ylpropan-1-one O-{2-[3-(1H-tetrazol-5-ylmethyl)phenoxy]ethyl}oxime

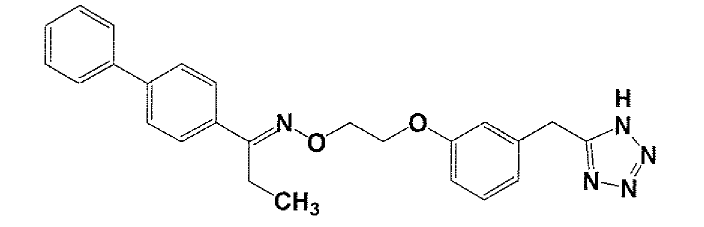

### Process 1:

A suspended solution of the compounds prepared in Example 14(17) (696mg) in toluene (7.0mL) was added by N,N-dimethylformamide (3 drops) and thionylchloride (0.2mL) and the mixture was stirred for 30 minutes at 80°C. The mixture was concentrated to give the rough acid chloride compounds. Separately, ammonia water was dropped by a solution of the above-mentioned rough acid chloride compounds in toluene (mL) at 0°C and stirred for 2 hours with rising till room temperature. The mixture was added by water and extracted by ethyl acetate. The organic layer was washed with 1M hydrochloric acid, water and saturated brine successively, dried over and then concentrated to give {3-[2-({[(1E)-1-biphenyl-4-ylpropylidene]amino}oxy)ethoxy]phenyl}acetic acid amide (504mg) having the following physical data.
TLC:Rf 0.13 (n-hexane : ethyl acetate = 1 : 2).

### Process 2:

A solution of triphenylphosphine (527mg) and N-chlorosuccinimide (278mg) in methylene chloride (8.0mL) was added by the compounds prepared in Process 1 and the mixture was stirred for an hour at room temperature. The mixture was concentrated and the residue was purified by column to give {3-[2-({[(1E)-1-biphneyl-4-ylpropylidene]amino}oxy)ethoxy]phenyl}acetanitrile (339mg) having the following physical data.
TLC:Rf 0.33 (n-hexane : ethyl acetate = 4 : 1).

### Process 3:

A solution of the compounds prepared in Process 2 in toluene (5.0mL) was added by trianethyltinazide (355mg) and the mixture was stirred overnight at 120°C. The mixture was added by methanol and 2M hydrochloric acid and extracted by ethyl acetate. The organic layer was washed with 1M hydrochloric acid, water and saturated brine successively, dried over and then concentrated. The residue was purified by column and recrystallized (n-hexane : ethyl acetate = 2 : 1) to give the compound in the present invention (170mg) having the following physical data were obtained.
TLC:Rf 0.50 (methylene chloride : methanol = 9 : 1);
NMR(DMSO-d₆); δ 7.79-7.63(m, 6H), 7.47(t, 2H), 7.37(d, 1H), 7.23(t, 1H), 6.95-6.77(m, 3H), 4.45(t, 2H), 4.25(t, 2H), 4.24(s, 2H), 2.72(q, 2H), 1.04(t, 3H).

### Biological Examples

It was confirmed that compounds of the present invention represented by formula (I) has PPAR regulatory activities by the following experiments.

### Measurement of PPAR agonistic activities:

### (1) Preparation of materials in luciferase assay using human PPAR

The whole operations were based on the basic gene engineering techniques and the conventional methods in yeast One-hybrid or Two-hybrid system were carried out. The measurement of present invention is the method which has advancement of the measurement accuracy and improvement of the measurement sensitivity in order to evaluate the compounds of the present invention as follows.

That is, as a luciferase gene expression vector under the control of thymidine kinase (TK) promoter, luciferase structural gene was excised from PicaGene Basic Vector 2 (trade name, Toyo Ink Inc., catalogue No. 309-04821), to prepare luciferase gene expression vector pTK-Luc. under the control of TK promoter (-105/+51) as a minimum essential promoter activity from pTKβ having TK promoter (Chrontech Inc., catalogue No. 6179-1). In the upper stream of TK promoter, four times repeated UAS sequence was inserted, which is the response sequence of Gal4 protein, a basic transcription factor in yeast, to construct 4 X UAS-TK-Luc. as reporter gene. The following is the enhancer sequence used (SEQ ID NO:1).
SEQ ID NO:1: Enhancer sequence repeating Gal4 protein response sequence
5'-T(CGACGGAGTACTGTCCTCCG)x4 AGCT-3'

A vector was prepared as described hereafter which expresses chimeric receptor protein wherein in carboxyl terminus of yeast Ga14 protein DNA binding domain was fused to ligand binding domain of human PPAR α, γ or δ. That is to say, PicaGene Basic Vector 2 (trade name, Toyo Ink Inc., catalogue No. 309-04821) was used as a basic expression vector, the structural gene was exchanged for that of chimeric receptor protein, while promoter and enhancer domains were kept as they were.

DNA encoding ligand binding domain of human PPAR α, γ or δ fused to DNA encoding Gal4 protein DNA binding domain, the downstream of DNA encoding the 1st to 147th amino acid sequence for fitting their frames and inserted to the downstream of promotor/enhancer in PicaGene Basic Vector 2 (trade name, Toyo Ink Inc., catalogue No. 309-04821). Here, DNA sequence was aligned as follows, the amino terminus of human PPAR α, γ or δ ligand binding domain was sequenced nuclear translocation signal originated from SV-40 T-antigen, Ala Pro Lys Lys Lys Arg Lys Val Gly (SEQ ID NO:2), to make an expressed chimeric protein localizing intranuclearly. On the other hand, the carboxyl terminus of them was sequenced influenza hemagglutinin epitope, Tyr Pro Tyr Asp Val Pro Asp Tyr Ala (SEQ ID NO:3) and stop codon for translation in this order, to detect an expressed fused protein tagged epitope sequence.

According to the comparison of human PPAR structures described in the literatures by R. Mukherjee *et al.* (See *J. Steroid Biochem. Molec. Biol.,* 51, 157 (1994)), M. E. Green *et al.,* (See *Gene Expression.,* 4, 281 (1995)), A Elbrecht *et al.* (See *Biochem Biophys. Res. Commun.,* 224, 431 (1996)) or A. Schmidt *et al.* (See Mol. Endocrinology, 6, 1634 (1992)), the portion of structural gene used as ligand binding domain of human PPAR α, γ or δ was DNA encoding the following peptide:
human PPAR α ligand binding domain: Ser¹⁶⁷-Tyr⁴⁶⁸
human PPAR γ ligand binding domain: Ser¹⁷⁶-Tyr⁴⁷⁸
human PPAR δ ligand binding domain: Ser¹³⁹-Tyr⁴⁴¹
(each human PPAR γ1 ligand binding domain and human PPAR γ2 ligand binding domain is Ser²⁰⁴-Tyr⁵⁰⁶ which is identical sequence each other). In order to measure basal level of transcription, an expression vector containing DNA binding domain of Gal4 protein lacking in PPAR ligand binding domain, which is exclusively encoding the 1st to 147th amino acid sequence in Gal4 protein was also prepared.

### (2) Luciferase assay using human PPAR α, γ or δ

CV-1 cells used as host cells were cultured by a conventional technique. That is to say, Dulbecco's modified Eagle medium (DMEM) supplemented 10% bovine fetal serum (GIBCO BRL Inc., catalogue No. 26140-061) and 50 U/ ml of penicillin G and 50 µg/ ml of streptomycin sulfate were used to culture CV-1 cells under the atmosphere of 5% carbon dioxide gas at 37°C.

In case of the transfection for introducing DNA, both reporter gene and Gal4-PPAR expression vector, into host cells, 2 x 10⁶ cells were seeded in a 10 cm dish, and once washed with the medium without serum, followed by addition of the medium (10 ml) thereto. Reporter gene (10 µg), Gal4-PPAR expression vector (0.5 µg) and 50 µl of LipofectAMINE (GIBRO BRL Inc., catalogue No. 18324-012) were well mixed and added to the culture dishes. They were cultured at 37°C for 5-6 hours, and thereto was added 10 ml of medium containing 20% of dialyzed bovine fetal serum (GIBRO BRL Inc., catalogue No. 26300-061), and then cultured at 37°C overnight. The cells were dispersed by trypsin treatment, and they were again seeded in 96-well plates in a density of 8000 cells/100 µl of DMEM-10% dialyzed serum/well. Several hours after the cultivation, when cells were attached to the plastic ware, then 100 µl of DMEM-10% dialyzed serum containing the compounds of the present invention, whose concentration is twice as high as the final concentration of them, was added thereto. The culture was settled at 37°C for 42 hours and the cells were dissolved to measure luciferase activity according to manufacturer's instruction.

In addition, the relative activity of the compounds of the present invention (10 µM) was measured under the condition that luciferase activity was defined as 1.0 in case of carbacyclin (10 µM) as a positive control compound, which could activate transcription of luciferase gene significantly to PPAR α (See *Eur*. *J. Biochem*., 233, 242 (1996); *Genes & Development.,* 10, 974 (1996)).

Also, the relative activity of the compounds of the present invention (10 µM) was measured under the condition that luciferase activity was defined as 1.0 in case of troglitazone (10 µM) as a positive control compound, which could activate transcription of luciferase gene significantly to PPAR γ (See *Cell.,* 83, 863 (1995); *Endocrinology.,* 137, 4189 (1996) and *J. Med. Chem.,* 39, 665 (1996)) and has been already launched as hypoglycemic agent.

As to PPAR δ activity, the relative activity of the compounds of the present invention was measured under the condition that luciferase activity was defined as 1.0 in case of addition of only solvent without the compounds.

As a result, the compounds of the present invention showed superior agonistic activity against, particularly, PPAR δ.

### Lowering effect of blood cholesterol and blood lipid (1):

Male, 6-weeks old SD rats (five rats per group) were brought in, fed pellet diet (CRF-1, oriental bio service) and tap water *ad libitum* in single cages for one week and habituated. Next, high cholesterol diet (CRF-1 pellet diet mixed of 5.5% peanut oil, 1.5% cholesterol and 0.5% cholic acid, oriental bio service) began to load the rats for one week.

After one week of the load, body weight of fasted rat was measured in the morning (a.m. 9:00 - a.m. 11:40) at the current day of dividing group (Day 0). Next, blood samples were collected from coccygeal vein and various parameters in plasma were measured. The measurement items were low density lipoprotein (LDL), high density lipoprotein (HDL), neutral fat (triglyceride (TG) level), non-esterified fatty acid (NFEA), and total cholesterol (TC) level. Based on HDL concentration, rats were divided into some groups (five rats per group). The body weight of rats were measured in the morning of the next day (1st day) of dividing groups and the compounds were compellingly orally administered into rats once a day for six days in a row and loads of high cholesterol diet were continued. The compounds of the present invention were dissolved by 0.5% methyl cellulose (MC) aqueous solution and then the administration solution was orally administered.

In the morning of the 1st and 4th day of onset of administration and the next day of final administration termination, food intakes were measured to calculate average food intakes. In addition, in the next day of final administration termination, blood samples were collected from coccygeal vein to measure the blood lipid (TG, HDL, LDL, NEFA, TC level) after administration of the compound of the present invention. Additionally, food intakes were not significantly different from control group (administration only 0.5% MC) and administration of the compound of the present invention group.

As a result, the compound of the present invention raised HDL depending on dose, and lowered LDL. Therefore, the compounds of the present invention are useful for therapeutic agent for hyperlipidemia.

### Hypoglycemic and hypolipidemic effects (2):

3 to 4-years old male cynomolgus monkeys (mean body weight: approximately 3kg) were bought and all animals having legal medical inspection were performed a medical inspection and habituated in the period of more than one month. The animals were housed individually in monkey cages and fed approximately 100g of pellet diet once a day. On habituation proceeding, animals came to finish feeding diet within an hour everyday. In addition, animals ingested tap water from automatic water supplying equipment *ad libitum.* Next, animals were pre-bred and 14 days and 2 weeks and 1 week before the test, the body weight of animals was measured, and then blood samples were collected from hindlimb saphenous vein to execute hematological test (measurement of the number of red blood cells, hematocrit, hemoglobin content, the number of platelets and the number of leukocytes) and blood biochemical test (measurement of GOT, GPT, alkaline phosphatase, total protein, blood urea nitrogen, creatinine, creatinine kinase, total bilirubin, blood glucose, total cholesterol, HDL, LDL and TG). Additionally, a general condition of animals is observed and individuals well grown during habituation and pre-breeding were selected to be used for test. Also, food intakes of all animals were measured everyday including the period of pre-breeding.

On the basis of body weight measured on final day of habituation period, each animals were divided into some groups (three animals pre group) using a stratified randomization method. In the morning of 1st, 3rd, 7th, 10th and 14th day of administration onset, body weight of animals was measured and administration volumes of the compounds of the present invention were calculated based on the latest body weight. The drug solution including diluted solution or the compounds of the present invention (3-100 mg/kg/day) was nasally intragastric administered into animals with nutrition catheters and syringes once a day for 14 days iteratively. On 1st, 7th, and 14th day after the administration onset, blood samples were collected before administration of the compounds of the present invention to measure the above-mentioned hematological test and blood biochemical test. It confirmed that the compounds of the present invention were not effect blood glucose. In addition, three weeks before, and 14th days after administration onset, blood sample were collected from hindlimb saphenous vein or antebrachial vein at 1, 2 and 4 hours after administration, and at 1, 2 and 3 hours after feeding a diet, to measure blood glucose, total cholesterol, HDL, LDL and TG

As a result, the compounds of the invention showed lowering effect of TG level in plasma, TC value and LDL value during fasting state. Additionally, the compounds of the present invention showed inhibitory effect of TG rising after feeding diets. Therefore, the compounds of the invention are useful for therapeutic agent for hyperlipidemia.

It is suggested that the lowering effect of plasma TG levels in fasted normal cynomolgus monkeys has possibility as the preventing and/or therapeutic agent for hyperlipidemia and atherosclerosis and so on. It is also observed in inhibitory effect on TG rising postprandial. Additionally, it can be estimated whether compounds have a toxicity change or not from other blood biochemical parameters.

### Preparation Example 1:

The following components were admixed in a conventional method, punched out to give 10000 tablets each containing 10 mg of active ingredient.

| | |
|---|---|
| {3-[2-({[bis(4-methylphenyl)methylene]amino}oxy)ethoxy]phenyl}acetic acid | 100 g |
| carboxymethylcellulosecalcium (distintegrant) | 4 g |
| magnesium stearate (lubricant) | 2 g |
| microcrystalline cellulose | 470 g |

### Preparation Example 2:

After mixing the following components by a conventional method, the resulting solution was filtrated by dust-proof filter and 5 ml portions thereof were filled in ampuls, respectively, and heat-sterilized by autoclave to obtain 10000 ampuls of injection containing each 20 mg of the active ingredient.

| | |
|---|---|
| {3-[2-({[bis(4-methylphenyl)methylene]amino}oxy)ethoxy]phenyl}acetic acid | 200 g |
| mannitol | 2 kg |
| distilled water | 50 L |

## Claims

1. A compound represented by formula (I) 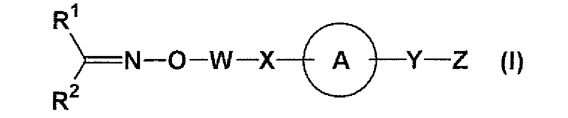 wherein R¹ and R² each independently represents (1) a hydrogen atom, (2) hydrocarbon which may have a substituent(s), (3) a cyclic group which may have a substituent(s), or (4) R¹ and R² are taken together to be a cyclic ring which may have a substituent(s),
W represents a spacer of which main chain has an atom number of 1-6,
X represents a single bond, -O-, -S-, -S(O)-, -SO₂- or -N(R³)-, in which R³ represents a hydrogen atom, alkyl which may have a substituent(s), acyl which may have a substituent(s), or alkoxycarbonyl which may have a substituent(s),
ringA is a cyclic group which may further have a substituent(s),
Y represents a single bond, or a spacer of which main chain has an atom number of 1-6,
Z represents an acidic group,
a salt thereof, a solvent thereof or an N-oxide thereof, or a prodrug thereof.

2. The compound according to claim 1, wherein either R¹ or R² is alkyl which may have a substituent(s) or a cyclic ring which may have a substituent(s), a salt thereof, a solvent thereof or an N-oxide thereof, or a prodrug thereof.

3. The compound according to claim 1, wherein R¹ and R² are each a cyclic ring which may have a substituent(s), a salt thereof, a solvent thereof or an N-oxide thereof, or a prodrug thereof.

4. The compound according to claim 1, wherein Y is unsubstituted -(CH₂)ₓ-, in which x represents an integer of 1 to 6, a salt thereof, a solvent thereof or an N-oxide thereof, or a prodrug thereof.

5. The compound according to claim 1, wherein one of R¹ and R² is alkyl which may have a substituent(s), the other of R¹ and R² is a cyclic ring which may have a substituent(s), a salt thereof, a solvent thereof or an N-oxide thereof, or a prodrug thereof.

6. The compound according to claim 1, wherein ringA is a monocyclic ring which may have a substituent(s), a salt thereof, a solvent thereof or an N-oxide thereof, or a prodrug thereof

7. The compound according to claim 1, wherein X is -O-, a salt thereof, a solvent thereof or an N-oxide thereof, or a prodrug thereof

8. The compound according to claim 1, wherein W is C1-6 alkylene which may have a substituent(s), a salt thereof, a solvent thereof or an N-oxide thereof, or a prodrug thereof.

9. The compound according to claim 1, wherein Z is carboxyl which may be esterified, a salt thereof, a solvent thereof or an N-oxide thereof, or a prodrug thereof.

10. The compound according to claim 1, which is selected from
(1) (3-{2-[{(1E)-1-[4-(trifuloroemethyl)phenyl]propylidene}amino)oxy]ethoxy)phenyl)acetic acid,
(2) {3-[2-({[(1E)-1-biphenyl-4-ylpropylidene]amino}oxy)ethoxy]phenyl}acetic acid,
(3) (3-{2-[({(1E)-1-[4-(2-thienyl)phenyl]propylidene}amino)oxy]ethoxy}phenyl}acetic acid,
(4) {3-[2-({[(1E)-1-(4-pyridin-2-ylphenyl)propylidene]amino}oxy)ethoxy]phenyl} acetic acid,
(5) (3-{2-[({(1E)-1-[4-(1H-pyrazol-1-yl)phenyl]propylidene}amino)oxy]ethoxy}phenyl)acetic acid,
(6) {3-[2-({[(1E)-1-biphenyl-4-ylpropylidene]amino}oxy)ethoxy]-2-methylphenyl}acetic acid,
(7) (2-fuloro-3-{2-[({(1E)-1-[4-(1H-pyrazol-1-yl)phenyl]propylidene}amino)oxy]ethoxy}phenyl)acetic acid,
(8) {2-fuloro-3-[2-({[(1E)-1-(4-pyridin-2-ylphenyl)propylidene]amino}oxy)ethoxy]phenyl}acetic acid,
(9) (2-methyl-3-{2-[({(1E)-[4-(1H-pyrazol-1-yl)phenyl]propylidene}amino)oxy]ethoxy}phenyl)acetic acid,
(10) {2-methyl-3-[2-({[(1E)-1-(4-pyridin-2-ylphenyl)propylidene]amino}oxy)ethoxy]phenyl}acetic acid,
(11) (2-methyl-3-{2-[({(1E)-1-[4-(trifuloromethyl)phenyl]propylidene}amino)oxy]ethoxy}phenyl)acetic acid,
(12) {3-[2-({[(1E)-1-biphenyl-4-ylpropylidene]amino}oxy)ethoxy]-5-methoxyphenyl} acetic acid,
(13) {3-[2-({[(1E)-1-biphenyl-4-ylbutylidene]amino}oxy)ethoxy]-2-methylphenyl}acetic acid,
(14) {3-[2-({[(1E)-1-(3'-fulorobiphenyl-4yl)propylidene]amino}oxy)ethoxy]phenyl}acetic acid,
(15) {3-[2-({[(1E)-1-biphenyl-4-yl-3-methylbutylidene]amino}oxy)ethoxy]-2-methylphenyl}acetic acid,
(16) {2-methyl-3-[2-({[(1E)-6-phenyl-3,4-dihydronaphthalen-1(2H)-ylidene]amino}oxy)ethoxy]phenyl}acetic acid,
(17) {3-[2-({[(1Z)-1-biphenyl-4-yl-2-(dimethylamino)ethylidene]amino}oxy)ethoxy]-2-methylphenyl}acetic acid,
(18) {3-[2-({[(1Z)-1-biphenyl-4-yl-2-(1,3-thiazolidin-3-yl)ethylidene]amino}oxy)ethoxy]-2-methyphenyl}acetic acid,
(19) {2-methyl-3-[2-({[(1E)-1-(4-pyridin-2-ylphenyl)butylidene]amino}oxy)ethoxy]phenyl}acetic acid,
(20) (2-methyl-3-{2-[({(1E)-1-[4-(1H-pyrazol-1-yl)phenyl]butylidene}amino)oxy]ethoxy}phenyl)acetic acid,
(21) {3-[2-({[(1Z)-1-biphenyl-4-yl-2-(2,5-dihydro-1H-pyrol-1-yl)ethylidene]amino}oxy)ethoxy]-2-methylphenyl}acetic acid,
(22) [3-(2-{[((1E)-1-biphenyl-4-yl-4,4,4-triflorobutylidene)amino]oxy}ethoxy)-2-methylphenyl]acetic acid,
(23) {2-methyl-3-[2-({[(1E)-1-(4-pyridin-2-ylphenyl)pentylidene] amino }oxy)ethoxy]phenyl} acetic acid,
(24) (2-methyl-3-{2-[({(1E)-3-methyl-1-[4-(1H-pyrazol-1-yl)phenyl]butylidene}amino)oxy]ethoxy} phenyl) acetic acid,
(25) [3 -(2- {[((1Z)-1-biphenyl-4-yl-2-piperidin-1-ylethylidene)amino]oxy}ethoxy)-2-methylphenyl]acetic acid,
(26) {3-[2-({[(1Z)-1-biphenyl-4-yl-2-(3,6-dihydropyridin-1(2H)-yl)ethylidene]amino }oxy)ethoxy]-2-methylphenyl}acetic acid,
(27) [2-methyl-3-(2-{[((1E)-5-phenyl-2,3-dihydro-1H-inden-1-ylidene)amino]oxy}ethoxy)phenyl acetic acid,
(28) [2-methyl-3-(2-{[((1E)-6-pyridin-2-yl-3,4-dihydronaphthalen-1(2H)-ylidene)amino]oxy}ethoxy)phenyl]acetic acid,
(29) {2-methyl-3-[2-({[(1E)-3-methyl-1-(4-pyridin-2-ylphenyl)butylidene]amino }oxy)ethoxy]phenyl} acetic acid,
(30) {2-methyl-3-[2-({[(1E)-6-(1H-pyrazol-1-yl)-3,4-dihydronaphthalen-1(2H)-ylidene]amino}oxy)ethoxy]phenyl}acetic acid,
(31) (3-{2-[({(1Z)-2-(dimethylammo)-1-[4-(1H-pyrazol-1-yl)phenyl]ethylidene}amino)oxy]ethoxy}-2-methylphenyl)acetic acid,
(32) {2-methyl-3-[2-({[(1Z)-1-[4-(1H-pyrazol-1-yl)phenyl]-2-(1,3-thiazolidin-3-yl)ethylidene]amino}oxy)ethoxy]phenyl}acetic acid,
(33) {3-[2-({[(1Z)-2-(dimethylamino)-1-(4-pyridin-2-ylphenyl)ethylidene]amino}oxy)ethoxy]-2-methylphenyl acetic acid, and
(34) {2-methyl-3-[2-({[(1Z)-1-(4-pyridin-2-ylphenyl)-2-{1,3-thiazolidin-3-yl)ethylidene]amino}oxy)ethoxy]phenyl}acetic acid,
a salt thereof, a solvent thereof or an N-oxide thereof, or a prodrug thereof

11. A pharmaceutical composition comprising the compound according to claim 1, a salt thereof, a solvent or an N-oxide or the prodrug thereof.

12. The pharmaceutical composition according to claim 11, wherein the pharmaceutical composition is a preventive and/or therapeutic agent for a disease caused by PPARδ.

13. The pharmaceutical composition according to claim 12, wherein the disease caused by PPARδ is hyperlipidemia or adiposity.

14. A method for prevention and/or treatment for a disease caused by PPARδ in a mammal, which comprises administering to a mammal an effective amount of a compound represented by formula (I): 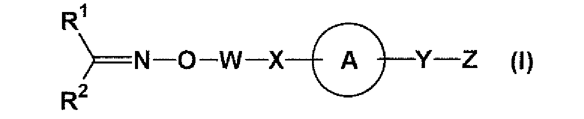 wherein all symbols have the same meanings as defined in claim 1, a salt thereof, a solvent thereof or an N-oxide thereof, or a prodrug thereof.

15. The method for prevention and/or treatment according to claim 14, wherein the disease caused by PPARδ is hyperlipidemia or adiposity.

16. Use of a compound represented by formula (I): 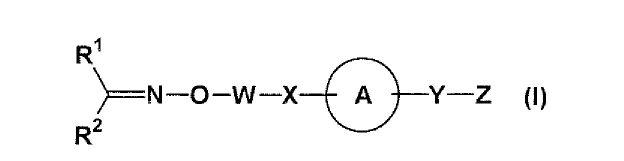 wherein all symbols have the same meanings as defined in claim 1, a salt thereof, a solvent thereof or an N-oxide thereof, or a prodrug thereof for preparing a prevention and/or treatment agent for a disease caused by PPARδ.

17. The use of the compound or a salt thereof according to claim 16, wherein the disease caused by PPARδ is hyperlipidemia or adiposity.

18. A medicament comprising the compound according to claim 1, a salt thereof, a solvent thereof or an N-oxide thereof, or a prodrug thereof and one kind or more kinds selected from an anti-adiposity drug, a therapeutic agent for diabetes and a lipid improvement drug.

19. The medicament according to claim 18, wherein the lipid improvement drug is an ACAT inhibitor, an MTP inhibitor, an HMG-CoA reductase inhibitor, a bile acid absorption inhibitor or a cholesterol absorption inhibitor.
